# EUROPÄISCHE PATENTSCHRIFT

(11) **EP 2 699 578 B1**
(45) Veröffentlichungstag und Bekanntmachung des Hinweises auf die Patenterteilung: **20.04.2016**
(21) Anmeldenummer: 12716404.4
(22) Anmeldetag: 20.04.2012
(51) Int. Cl.: C07D 487/04, A61K 31/519, A61K 31/53, A61P 9/00, A61P 11/00, A61P 13/12

(54) **FLUORALKYL-SUBSTITUIERTE PYRAZOLOPYRIDINE UND IHRE VERWENDUNG**
FLUOROALKYL-SUBSTITUTED PYRAZOLOPYRIDINES AND USE THEREOF
PYRAZOLOPYRIDINES SUBSTITUÉES PAR UN FLUORO-ALKYLE ET LEUR UTILISATION

(30) Priorität: 21.04.2011 DE 102011007890; 11.01.2012 DE 102012200357
(43) Veröffentlichungstag der Anmeldung: 26.02.2014
(73) Patentinhaber: Bayer Intellectual Property GmbH, 40789 Monheim (DE)
(72) Erfinder: FOLLMANN, Markus, 50859 Köln (DE); STASCH, Johannes-Peter, 42651 Solingen (DE); REDLICH, Gorden, 44799 Bochum (DE); GRIEBENOW, Nils, 41541 Dormagen (DE); WUNDER, Frank, 42117 Wuppertal (DE); LI, Volkhart, Min-Jian, 42553 Velbert (DE); LANG, Dieter, 42553 Velbert (DE)
(74) Vertreter: BIP Patents
(86) Internationale Anmeldenummer: PCT/EP2012/057269
(87) Internationale Veröffentlichungsnummer: WO 2012/143510

(56) Entgegenhaltungen:
- WO-A1-2010/065275

## Beschreibung

Die vorliegende Anmeldung betrifft neue Fluoralkyl-substituierte Pyrazolopyridine, Verfahren zu ihrer Herstellung, diese Verbindungen zur Verwendung allein oder in Kombinationen zur Behandlung und/oder Prophylaxe von Krankheiten sowie ihre Verwendung zur Herstellung von Arzneimitteln zur Behandlung und/oder Prophylaxe von Krankheiten, insbesondere zur Behandlung und/oder Prophylaxe von Herz-Kreislauf-Erkrankungen.

Eines der wichtigsten zellulären Übertragungssysteme in Säugerzellen ist das cyclische Guanosinmonophosphat (cGMP). Zusammen mit Stickstoffmonoxid (NO), das aus dem Endothel freigesetzt wird und hormonelle und mechanische Signale überträgt, bildet es das NO/cGMP-System. Die Guanylatcyclasen katalysieren die Biosynthese von cGMP aus Guanosintriphosphat (GTP). Die bisher bekannten Vertreter dieser Familie lassen sich sowohl nach strukturellen Merkmalen als auch nach der Art der Liganden in zwei Gruppen aufteilen: Die partikulären, durch natriuretische Peptide stimulierbaren Guanylatcyclasen und die löslichen, durch NO stimulierbaren Guanylatcyclasen. Die löslichen Guanylatcyclasen bestehen aus zwei Untereinheiten und enthalten höchstwahrscheinlich ein Häm pro Heterodimer, das ein Teil des regulatorischen Zentrums ist. Dieses hat eine zentrale Bedeutung für den Aktivierungsmechanismus. NO kann an das Eisenatom des Häms binden und so die Aktivität des Enzyms deutlich erhöhen. Hämfreie Präparationen lassen sich hingegen nicht durch NO stimulieren. Auch Kohlenmonoxid (CO) ist in der Lage, an das Eisen-Zentralatom des Häms zu binden, wobei die Stimulierung durch CO deutlich geringer ist als die durch NO.

Durch die Bildung von cGMP und der daraus resultierenden Regulation von Phosphodiesterasen, Ionenkanälen und Proteinkinasen spielt die Guanylatcyclase eine entscheidende Rolle bei unterschiedlichen physiologischen Prozessen, insbesondere bei der Relaxation und Proliferation glatter Muskelzellen, der Plättchenaggregation und -adhäsion, der neuronalen Signalübertragung sowie bei Erkrankungen, welche auf einer Störung der vorstehend genannten Vorgänge beruhen. Unter pathophysiologischen Bedingungen kann das NO/cGMP-System supprimiert sein, was zum Beispiel zu Bluthochdruck, einer Plättchenaktivierung, einer vermehrten Zellproliferation, endothelialer Dysfunktion, Arteriosklerose, Angina pectoris, Herzinsuffizienz, Myokardinfarkt, Thrombosen, Schlaganfall und sexueller Dysfunktion führen kann.

Eine auf die Beeinflussung des cGMP-Signalweges in Organismen abzielende NO-unabhängige Behandlungsmöglichkeit für derartige Erkrankungen ist aufgrund der zu erwartenden hohen Effizienz und geringen Nebenwirkungen ein vielversprechender Ansatz.

Zur therapeutischen Stimulation der löslichen Guanylatcyclase wurden bisher ausschließlich Verbindungen wie organische Nitrate verwendet, deren Wirkung auf NO beruht. Dieses wird durch Biokonversion gebildet und aktiviert die lösliche Guanylatcyclase durch Angriff am Eisen-Zentralatom des Häms. Neben den Nebenwirkungen gehört die Toleranzentwicklung zu den entscheidenden Nachteilen dieser Behandlungsweise.

In den letzten Jahren wurden einige Substanzen beschrieben, die die lösliche Guanylatcyclase direkt, d.h. ohne vorherige Freisetzung von NO stimulieren, wie beispielsweise 3-(5'-Hydroxy-methyl-2'-furyl)-l-benzylindazol [YC-1; Wu et al., Blood 84 (1994), 4226; Mülsch et al., Brit. J. Pharmacol. 120 (1997), 681], Fettsäuren [Goldberg et al., J. Biol. Chem. 252 (1977), 1279], Diphenyliodonium-hexafluorophosphat [Pettibone et al., Eur. J. Pharmacol. 116 (1985), 307], Isoliquiritigenin [Yu et al., Brit. J. Pharmacol. 114 (1995), 1587] sowie verschiedene substituierte Pyrazol-Derivate (WO 98/16223).

Als Stimulatoren der löslichen Guanylatcyclase werden in WO 00/06569 annellierte Pyrazol-Derivate und in WO 03/095451 Carbamat-substitutierte 3-Pyrimidinyl-Pyrazolopyridine offenbart. WO 2010/065275 und WO 2011/149921 offenbarrn substituierte Pyrrolo- und Dihydropyridopyrimidine als sGC Aktivatoren.

Aufgabe der vorliegenden Erfindung war die Bereitstellung neuer Substanzen, die als Stimulatoren der löslichen Guanylatcyclase wirken und ein gleiches oder verbessertes therapeutisches Profil gegenüber den aus dem Stand der Technik bekannten Verbindungen aufweisen, wie beispielsweise hinsichtlich ihrer *in-vivo* Eigenschaften, wie beispielsweise ihrem pharmakokinetischem und pharmakodynamischem Verhalten und/oder ihres Metabolismus-Profils und/oder ihrer Dosis-Wirkungsbeziehung.

Offenbarungsgegenstand sind Verbindungen der allgemeinen Formel (I) 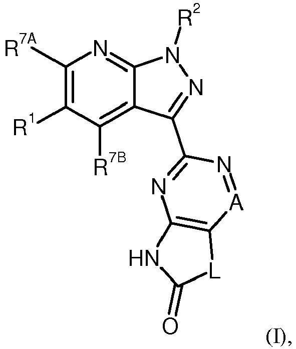 in welcher
- A: für Stickstoff oder CR³ steht,
wobei
R³ für Wasserstoff, Deuterium, Halogen, Difluormethyl, Trifluormethyl, (C₁-C₄)-Alkyl, (C₂-C₄)-Alkenyl, (C₂-C₄)-Alkinyl, Cyclopropyl, Cyclobutyl, Hydroxy, Phenyl oder 5- oder 6-gliedriges Heteroaryl steht,
worin (C₁-C₄)-Alkyl, (C₂-C₄)-Alkenyl, (C₂-C₄)-Alkinyl, Phenyl und 5- oder 6-gliedriges Heteroaryl mit 1 bis 3 Substituenten unabhängig voneinander ausgewählt aus der Gruppe mit Fluor, Difluormethyl, Trifluormethyl, (C₁-C₄)-Alkyl, Difluormethoxy, Trifluormethoxy, (C₁-C₄)-Alkoxy, (C₁-C₄)-Alkoxycarbonyl, Cyclopropyl und Cyclobutyl substituiert sein kann,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidinring bzw Triazinring steht,
p für eine Zahl 0, 1 oder 2 steht,
R^{4A} für Wasserstoff, Fluor, (C₁-C₄)-Alkyl, Hydroxy oder Amino steht,
   worin (C₁-C₄)-Alkyl mit 1 bis 3 Substituenten unabhängig voneinander ausgewählt aus der Gruppe Fluor, Trifluormethyl, Hydroxy, Hydroxycarbonyl, (C₁-C₄)-Alkoxycarbonyl und Amino substituiert sein kann,
R^{4B} für Wasserstoff, Fluor, Difluormethyl, Trifluormethyl, (C₁-C₆)-Alkyl, (C₁-C₄)-Alkoxycarbonylamino, Cyano, (C₃-C₇)-Cycloalkyl, Difluormethoxy, Trifluormethoxy, Phenyl oder eine Gruppe der Formel -M-R⁸ steht,
   worin (C₁-C₆)-Alkyl mit 1 bis 3 Substituenten unabhängig voneinander ausgewählt aus der Gruppe Fluor, Cyano, Trifluormethyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl und Amino substituiert sein kann,
   und worin
   M für eine Bindung oder (C₁-C₄)-Alkandiyl steht,
   R⁸ für -(C=O)ᵣ-OR⁹, -(C=O)ᵣ-NR⁹R¹⁰, -C(=S)-NR⁹R¹⁰, -NR⁹-(C=O)-R¹², -NR⁹-(C=O)-NR¹⁰R¹¹, -NR⁹-SO₂-NR¹⁰R¹¹, -NR⁹-SO₂-R¹², -S(O)ₛ-R¹², -SO₂-NR⁹R¹⁰, 4- bis 7-gliedriges Heterocyclyl, Phenyl oder 5- oder 6-gliedriges Heteroaryl steht,
   worin
   r die Zahl 0 oder 1 bedeutet,
   s die Zahl 0, 1 oder 2 bedeutet,
   R⁹, R¹⁰ und R¹¹ unabhängig voneinander jeweils für Wasserstoff, (C₁-C₆)-Alkyl, (C₃-C₈)-Cycloalkyl, 4- bis 7-gliedriges Heterocyclyl, Phenyl oder 5- oder 6-gliedriges Heteroaryl stehen, oder
   R⁹ und R¹⁰ bilden zusammen mit dem/den Atom/-en, an die sie jeweils gebunden sind, einen 4- bis 7-gliedrigen Heterocyclus,
   worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann, oder
   R¹⁰ und R¹¹ bilden zusammen mit dem/den Atom/-en, an die sie jeweils gebunden sind, einen 4- bis 7-gliedrigen Heterocyclus,
   worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann,
   R¹² für (C₁-C₆)-Alkyl oder (C₃-C₇)-Cycloalkyl steht, oder
   R⁹ und R¹² bilden zusammen mit dem/den Atom/-en, an die sie jeweils gebunden sind, einen 4- bis 7-gliedrigen Heterocyclus,
   worin der 4- bis 7-gliedrigen Heterocyclus seinerseits mit 1 oder 2 Substituenten unabhängig voneinander ausgewählt aus der Gruppe Cyano, Trifluormethyl, (C₁-C₆)-Alkyl, Hydroxy, Oxo, (C₁-C₆)-Alkoxy, Trifluormethoxy, (C₁-C₆)-Alkoxycarbonyl, Amino, Mono-(C₁-C₆)-alkylamino und Di-(C₁-C₆)-alkylamino substituiert sein kann, und
   worin 4- bis 7-gliedriges Heterocyclyl, Phenyl und 5- oder 6-gliedriges Heteroaryl ihrerseits mit 1 bis 3 Substituenten unabhängig voneinander ausgewählt aus der Gruppe Halogen, Cyano, Difluormethyl, Trifluormethyl, (C₁-C₆)-Alkyl, (C₃-C₇)-Cycloalkyl, Hydroxy, Oxo, Thiooxo und (C₁-C₄)-Alkoxy substituiert sein können,
   und
   worin die zuvor genannten (C₁-C₄)-Alkyl-, (C₁-C₆)-Alkyl-, (C₃-C₈)-Cycloalkyl- und 4- bis 7-gliedriges Heterocyclyl-Gruppen, sofern nicht anders angegeben, jeweils unabhängig voneinander weiterhin mit 1 bis 3 Substituenten unabhängig voneinander ausgewählt aus der Gruppe Fluor, Difluormethyl, Trifluormethyl, (C₁-C₆)-Alkyl, (C₃-C₇)-Cycloalkyl, Hydroxy, Difluormethoxy, Trifluormethoxy, (C₁-C₄)-Alkoxy, Hydroxycarbonyl, (C₁-C₄)-Alkoxycarbonyl, Amino, Phenyl, 4- bis 7-gliedriges Heterocyclyl und 5- oder 6-gliedriges Heteroaryl substituiert sein können,
oder
- R^{4A} und R^{4B}: zusammen mit dem Kohlenstoffatom, an das sie gebunden sind, eine (C₂-C₄)-Alkenyl-Gruppe, eine Oxo-Gruppe, einen 3- bis 6-gliedrigen Carbocyclus oder einen 4- bis 7-gliedrigen Heterocyclus bilden, worin der 3- bis 6-gliedrigen Carbocyclus und der 4- bis 7-gliedrigen Heterocyclus mit 1 oder 2 Substituenten unabhängig voneinander ausge wählt aus der Gruppe Fluor und (C₁-C₄)-Alkyl substituiert sein können,
R^{5A} für Wasserstoff, Fluor, (C₁-C4)-Alkyl oder Hydroxy steht,
R^{5B} für Wasserstoff, Fluor, (C₁-C₄)-Alkyl oder Trifluormethyl steht,
- R¹: für Wasserstoff, Halogen, Cyano, Difluormethyl, Trifluormethyl, (C₁-C₄)-Alkyl oder (C₃-C₇)-Cycloalkyl steht,
- R²: für eine Gruppe der Formel *-(CR^{6A}R^{6B})_{q}CHF₂, *-(CR^{6A}R^{6B})_{q}CF₃ oder *-(CR^{6A}R^{6B})_{q}-(C₃-C₇)-Cycloalkyl steht,
wobei
* für die Anknüpfstelle an das Pyrazolopyridin steht,
q für eine Zahl 1, 2 oder 3 steht,
R^{6A} für Wasserstoff oder Fluor steht,
R^{6B} für Wasserstoff oder Fluor steht,
und
wobei (C₃-C₇)-Cycloalkyl mit 1 bis 3 Substituenten unabhängig voneinander ausgewählt aus der Gruppe Fluor und (C₁-C₄)-Alkyl substituiert sein kann
- R^{7A}: für Wasserstoff, Cyano, Difluormethyl, Trifluormethyl, (C₁-C₄)-Alkyl oder (C₃-C₇)-Cycloalkyl steht,
- R^{7B}: für Wasserstoff, Cyano, Difluormethyl, Trifluormethyl, (C₁-C₄)-Alkyl oder (C₃-C₇)-Cycloalkyl steht,
sowie ihre *N*-Oxide, Salze, Solvate, Salze der *N*-Oxide und Solvate der *N*-Oxide und Salze.

Erfindungsgemäße Verbindungen sind die Verbindungen der Formel (I) und deren Salze, Solvate und Solvate der Salze, die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Erfindungsgemäße Verbindungen sind ebenso *N*-Oxide der Verbindungen der Formel (I) sowie deren Salze, Solvate und Solvate der Salze.

Als Salze sind im Rahmen der vorliegenden Erfindung physiologisch unbedenkliche Salze der erfindungsgemäßen Verbindungen bevorzugt. Umfasst sind auch Salze, die für pharmazeutische Anwendungen selbst nicht geeignet sind, jedoch beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Ameisensäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt. Als Solvate sind im Rahmen der vorliegenden Erfindung Hydrate bevorzugt.

Die erfindungsgemäßen Verbindungen können in Abhängigkeit von ihrer Struktur in unterschiedlichen stereoisomeren Formen existieren, d.h. in Gestalt von Konfigurationsisomeren oder gegebenenfalls auch als Konformationsisomere (Enantiomere und/oder Diastereomere, einschließlich solcher bei Atropisomeren). Die vorliegende Erfindung umfasst deshalb die Enantiomere und Diastereomere und ihre jeweiligen Mischungen. Aus solchen Mischungen von Enantiomeren und/ oder Diastereomeren lassen sich die stereoisomer einheitlichen Bestandteile in bekannter Weise isolieren; vorzugsweise werden hierfür chromatographische Verfahren verwendet, insbesondere die HPLC-Chromatographie an achiraler bzw. chiraler Phase.

Sofern die erfindungsgemäßen Verbindungen in tautomeren Formen vorkommen können, umfasst die vorliegende Erfindung sämtliche tautomere Formen.

Die vorliegende Erfindung umfasst auch alle geeigneten isotopischen Varianten der erfindungsgemäßen Verbindungen. Unter einer isotopischen Variante einer erfindungsgemäßen Verbindung wird hierbei eine Verbindung verstanden, in welcher mindestens ein Atom innerhalb der erfindungsgemäßen Verbindung gegen ein anderes Atom der gleichen Ordnungszahl, jedoch mit einer anderen Atommasse als der gewöhnlich oder überwiegend in der Natur vorkommenden Atommasse ausgetauscht ist. Beispiele für Isotope, die in eine erfindungsgemäße Verbindung inkorporiert werden können, sind solche von Wasserstoff, Kohlenstoff, Stickstoff, Sauerstoff, Phosphor, Schwefel, Fluor, Chlor, Brom und Iod, wie ²H (Deuterium), ³H (Tritium), ¹³C, ¹⁴C, ¹⁵N, ¹⁷O, ¹⁸O, ³²P, ³³P, ³³S, ³⁴S, ³⁵S, ³⁶S, ¹⁸F, ³⁶Cl, ⁸²Br, ¹²³I, ¹²⁴I, ¹²⁹I und ¹³¹I. Bestimmte isotopische Varianten einer erfindungsgemäßen Verbindung, wie insbesondere solche, bei denen ein oder mehrere radioaktive Isotope inkorporiert sind, können von Nutzen sein beispielsweise für die Untersuchung des Wirkmechanismus oder der Wirkstoff-Verteilung im Körper; aufgrund der vergleichsweise leichten Herstell- und Detektierbarkeit sind hierfür insbesondere mit ³H- oder ¹⁴C-Isotopen markierte Verbindungen geeignet. Darüber hinaus kann der Einbau von Isotopen, wie beispielsweise von Deuterium, zu bestimmten therapeutischen Vorteilen als Folge einer größeren metabolischen Stabilität der Verbindung führen, wie beispielsweise eine Verlängerung der Halbwertszeit im Körper oder eine Reduktion der erforderlichen Wirkdosis; solche Modifikationen der erfindungsgemäßen Verbindungen können daher gegebenenfalls auch eine bevorzugte Ausführungsform der vorliegenden Erfindung darstellen. Isotopische Varianten der erfindungsgemäßen Verbindungen können nach den dem Fachmann bekannten Verfahren hergestellt werden, so beispielsweise nach den weiter unten beschriebenen Methoden und den bei den Ausführungsbeispielen wiedergegebenen Vorschriften, indem entsprechende isotopische Modifikationen der jeweiligen Reagentien und/oder Ausgangsverbindungen eingesetzt werden.

In der Formel der Gruppe, für die L bzw. R²stehen kann, steht der Endpunkt der Linie, an dem das Zeichen #, ## bzw. * steht, nicht für ein Kohlenstoffatom beziehungsweise eine CH₂-Gruppe, sondern ist Bestandteil der Bindung zu dem jeweils bezeichneten Atom, an das L bzw. R² gebunden ist.

Im Rahmen der vorliegenden Erfindung haben die Substituenten, soweit nicht anders spezifiziert, die folgende Bedeutung:
Alkyl steht im Rahmen der Erfindung für einen linearen oder verzweigten Alkylrest mit der jeweils angegebenen Anzahl an Kohlenstoffatomen. Beispielhaft und vorzugsweise seien genannt: Methyl, Ethyl, n-Propyl, Isopropyl, n-Butyl, iso-Butyl, 1-Methylpropyl, tert.-Butyl, n-Pentyl, iso-Pentyl, 1-Ethylpropyl, 1-Methylbutyl, 2-Methylbutyl, 3-Methylbutyl, n-Hexyl, 1-Methylpentyl, 2-Methylpentyl, 3-Methylpentyl, 4-Methylpentyl, 3,3-Dimethylbutyl, 1-Ethylbutyl und 2-Ethylbutyl.

Cycloalkyl bzw. Carbocyclus steht in Rahmen der Erfindung für einen monocyclischen, gesättigten Alkylrest mit der jeweils angegebenen Anzahl an Kohlenstoffatomen. Beispielhaft und vorzugsweise seien genannt: Cyclopropyl, Cyclobutyl, Cyclopentyl, Cyclohexyl und Cycloheptyl.

Alkandiyl steht im Rahmen der Erfindung für einen linearen oder verzweigten divalenten Alkylrest mit 1 bis 4 Kohlenstoffatomen. Beispielhaft und vorzugsweise seien genannt: Methylen, Ethan-1,2-diyl, Ethan-1,1-diyl, Propan-1,3-diyl, Propan-1,1-dlyl, Propan-1,2-diyl, Propan-2,2-diyl, Butan-1,4-diyl, Butan-1,2-diyl, Butan-1,3-diyl und Butan-2,3-diyl.

Alkenyl steht im Rahmen der Erfindung für einen linearen oder verzweigten Alkenylrest mit 2 bis 4 Kohlenstoffatomen und einer Doppelbindung. Beispielhaft und vorzugsweise seien genannt: Vinyl, Allyl, Isopropenyl und n-But-2-en-1-yl.

Alkinyl steht im Rahmen der Erfindung für einen linearen oder verzweigten Alkinylrest mit 2 bis 4 Kohlenstoffatomen und einer Dreifachbindung. Beispielhaft und vorzugsweise seien genannt: Ethinyl, n-Prop-1-in-1-yl, n-Prop-2-in-1-yl, n-But-2-in-1-yl und n-But-3-in-1-yl.

Alkoxy steht im Rahmen der Erfindung für einen linearen oder verzweigten Alkoxyrest mit 1 bis 6 bzw. 1 bis 4 Kohlenstoffatomen. Beispielhaft seien genannt: Methoxy, Ethoxy, n-Propoxy, Isopropoxy, 1-Methylpropoxy, n-Butoxy, iso-Butoxy, tert.-Butoxy, n-Pentoxy, iso-Pentoxy, 1-Ethylpropoxy, 1-Methylbutoxy, 2-Methylbutoxy, 3-Methylbutoxy und n-Hexoxy. Bevorzugt ist ein linearer oder verzweigter Alkoxyrest mit 1 bis 4 Kohlenstoffatomen. Beispielhaft und vorzugsweise seien genannt: Methoxy, Ethoxy, n-Propoxy, Isopropoxy, 1-Methylpropoxy, n-Butoxy, iso-Butoxy, tert.-Butoxy,

Alkoxycarbonyl steht im Rahmen der Erfindung für einen linearen oder verzweigten Alkoxyrest mit 1 bis 4 Kohlenstoffatomen und einer am Sauerstoff angebundenen Carbonylgruppe. Beispielhaft und vorzugsweise seien genannt: Methoxycarbonyl, Ethoxycarbonyl, n-Propoxycarbonyl, Isopropoxycarbonyl und tert.-Butoxycarbonyl.

Alkoxycarbonylamino steht im Rahmen der Erfindung für eine Amino-Gruppe mit einem linearen oder verzweigten Alkoxycarbonyl-Substituenten, der 1 bis 4 Kohlenstoffatome in der Alkylkette aufweist und über die Carbonylgruppe mit dem N-Atom verknüpft ist. Beispielhaft und vorzugsweise seien genannt: Methoxycarbonylamino, Ethoxycarbonylamino, Propoxycarbonylamino, n-Butoxycarbonylamino, iso-Butoxycarbonylamino und tert.-Butoxycarbonylamino.

Mono-alkylamino steht im Rahmen der Erfindung für eine Amino-Gruppe mit einem linearen oder verzweigten Alkylsubstituenten, der 1 bis 6 Kohlenstoffatome aufweist. Beispielhaft und vorzugsweise seien genannt: Methylamino, Ethylamino, n-Propylamino, Isopropylamino und tert.-Butylamino.

Di-alkylamino steht im Rahmen der Erfindung für eine Amino-Gruppe mit zwei gleichen oder verschiedenen linearen oder verzweigten Alkylsubstituenten, die jeweils 1 bis 6 Kohlenstoffatome aufweisen. Beispielhaft und vorzugsweise seien genannt: *N,N*-Dimethylamino, *N,N*-Diethylamino, *N*-Ethyl-*N*-methylamino, *N*-Methyl-*N*-*n*-propylamino, *N*-Isopropyl-*N*-n-propylamino, N-tert.-Butyl-*N*-methylamino, *N*-Ethyl-*N*-n-pentylamino und *N*-n-Hexyl-*N*-methylamino.

Heterocyclyl bzw. Heterocyclus steht im Rahmen der Erfindung für einen gesättigten Heterocyclus mit insgesamt 4 bis 7 Ringatomen, der ein oder zwei Ring-Heteroatome aus der Reihe N, O, S, SO und/oder SO₂ enthält. Beispielhaft seien genannt: Azetidinyl, Oxetanyl, Pyrrolidinyl, Pyrazolidinyl, Imidazolinyl, Tetrahydrofuranyl, Piperidinyl, Piperazinyl, Tetrahydropyranyl, Morpholinyl, Thiomorpholinyl und Dioxidothiomorpholinyl. Bevorzugt sind Azetidinyl, Oxetanyl, Pyrrolidinyl, Tetrahydrofuranyl, Piperidinyl, Tetrahydropyranyl und Morpholinyl.

5- oder 6-gliedriges Heteroaryl steht im Rahmen der Erfindung für einen monocyclischen aromatischen Heterocyclus (Heteroaromaten) mit insgesamt 5 oder 6 Ringatomen, der bis zu drei gleiche oder verschiedene Ring-Heteroatome aus der Reihe N, O und/oder S enthält und über ein Ring-Kohlenstoffatom oder gegebenenfalls über ein Ring-Stickstoffatom verknüpft ist. Beispielhaft und vorzugsweise seien genannt: Furyl, Pyrrolyl, Thienyl, Pyrazolyl, Imidazolyl, Thiazolyl, Oxazolyl, Isoxazolyl, Isothiazolyl, Triazolyl, Oxadiazolyl, Thiadiazolyl, Pyridyl, Pyrimidinyl, Pyridazinyl, Pyrazinyl und Triazinyl. Bevorzugt sind: Pyrazolyl, Oxazolyl, Thiazolyl, Triazolyl, Oxadiazolyl, Thiadiazolyl, Pyridyl und Pyrimidinyl.

Halogen steht im Rahmen der Erfindung für Fluor, Chlor, Brom und Iod. Bevorzugt sind Brom und Iod.

Eine Oxo-Gruppe steht im Rahmen der Erfindung für ein Sauerstoffatom, das über eine Doppelbindung an ein Kohlenstoffatom gebunden ist.

Eine Thiooxo-Gruppe steht im Rahmen der Erfindung für ein Schwefelatom, das über eine Doppelbindung an ein Kohlenstoffatom gebunden ist.

Wenn Reste in den erfindungsgemäßen Verbindungen substituiert sind, können die Reste, soweit nicht anders spezifiziert, ein- oder mehrfach substituiert sein. Im Rahmen der vorliegenden Erfindung gilt, dass für alle Reste, die mehrfach auftreten, deren Bedeutung unabhängig voneinander ist. Eine Substitution mit ein, zwei oder drei gleichen oder verschiedenen Substituenten ist bevorzugt.

Im Sinne der vorliegenden Erfindung umfasst der Begriff "Behandlung" oder "behandeln" ein Hemmen, Verzögern, Aufhalten, Lindern, Abschwächen, Einschränken, Verringern, Unterdrücken, Zurückdrängen oder Heilen einer Krankheit, eines Leidens, einer Erkrankung, einer Verletzung oder einer gesundheitlichen Störung, der Entfaltung, des Verlaufs oder des Fortschreitens solcher Zustände und/oder der Symptome solcher Zustände. Der Begriff "Therapie" wird hierbei als synonym mit dem Begriff "Behandlung" verstanden.

Die Begriffe "Prävention", "Prophylaxe" oder "Vorbeugung" werden im Rahmen der vorliegenden Erfindung synonym verwendet und bezeichnen das Vermeiden oder Vermindern des Risikos, eine Krankheit, ein Leiden, eine Erkrankung, eine Verletzung oder eine gesundheitliche Störung, eine Entfaltung oder ein Fortschreiten solcher Zustände und/oder die Symptome solcher Zustände zu bekommen, zu erfahren, zu erleiden oder zu haben.

Die Behandlung oder die Prävention einer Krankheit, eines Leidens, einer Erkrankung, einer Verletzung oder einer gesundheitlichen Störung können teilweise oder vollständig erfolgen.

Die Verbindungen der Formel (I-1) bilden eine Untergruppe der erfindungsgemäßen Verbindungen der Formel (I), in welcher R^{7A} und R^{7B} für Wasserstoff stehen.

Offenbarungsgegenstand sind Verbindungen der Formel (I), in welcher
- A: für Stickstoff oder CR³ steht,
wobei
R³ für Wasserstoff, Deuterium, Fluor, Iod, Difluormethyl, Trifluormethyl, (C₁-C₄)-Alkyl, Vinyl, Allyl, Ethinyl, Cyclopropyl, Cyclobutyl, Hydroxy, Pyrazolyl oder Pyridyl steht,
worin (C₁-C₄)-Alkyl, Vinyl, Allyl, Ethinyl und Pyridyl mit 1 oder 2 Substituenten unabhängig voneinander ausgewählt aus der Gruppe mit Methyl, Cyclopropyl und Cyclobutyl substituiert sein können,
- L: für eine Gruppe #-CR^{4A}R^{4B}-(CR^{5A}R^{5B})ₚ-## steht,
wobei
# für die Anlcnüpfstelle an die Carbonylgruppe steht,
## für die Anlcnüpfstelle an den Pyrimidinring bzw Triazinring steht,
p für eine Zahl 0 oder 1 steht,
R^{4A} für Wasserstoff, Fluor, Methyl, Ethyl, Hydroxy oder Amino steht,
R^{4B} für Wasserstoff, Fluor, Difluormethyl, Trifluormethyl, (C₁-C₄)-Alkyl, Methoxycarbonylamino, Cyano, Cyclopropyl, Cyclobutyl, Cyclopentyl, Phenyl oder eine Gruppe der Formel -M-R⁸ steht,
worin (C₁-C₄)-Alkyl mit 1 bis 3 Substituenten unabhängig voneinander ausgewählt aus der Gruppe Fluor, Cyano, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein kann,
und worin
M für eine Bindung oder Methylen steht,
R⁸ für -(C=O)ᵣ-NR⁹R¹⁰, -C(=S)-NR⁹R¹⁰, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 oder 1 bedeutet,
R⁹ und R¹⁰ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl ihrerseits mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können, oder
R^{4A} und R^{4B} zusammen mit dem Kohlenstoffatom, an das sie gebunden sind, einen Cyclopropyl-, Cyclobutyl-, Cyclopentyl-, Azetidinyl-, Tetrahydrofuranyl-, Pyrrolidinyl- oder Tetrahydropyranyl-Ring bilden,
worin der Cyclopropyl-, Cyclobutyl-, Cyclopentyl-, Azetidinyl-, Tetrahydrofuranyl-, Pyrrolidinyl- und Tetrahydropyranyl-Ring mit 1 oder 2 Substituenten unabhängig voneinander ausgewählt aus der Gruppe Fluor und Methyl substituiert sein können,
R^{5A} für Wasserstoff, Fluor, Methyl, Ethyl oder Hydroxy steht,
R^{5B} für Wasserstoff, Fluor, Methyl, Ethyl oder Trifluormethyl steht,
- R¹: für Wasserstoff oder Fluor steht,
- R²: für eine Gruppe der Formel *-(CR^{6A}R^{6B})_{q}CHF₂, *-(CR^{6A}R^{6B})_{q}CF₃, Cyclobutylmethyl oder Cyclopentylmethyl steht,
wobei
* für die Anknüpfstelle an das Pyrazolopyridin steht,
q für eine Zahl 2 oder 3 steht,
R^{6A} für Wasserstoff oder Fluor steht,
R^{6B} für Wasserstoff oder Fluor steht,
und
wobei Cyclobutylmethyl und Cyclopentylmethyl mit 1 oder 2 Substituenten Fluor substituiert sein können,
- R^{7A}: für Wasserstoff oder Methyl steht,
- R^{7B}: für Wasserstoff steht,
sowie ihre Salze, Solvate und Solvate der Salze.

Offenbarungsgegenstand sind auch Verbindungen der Formel (I), in welcher
- A: für Stickstoff oder CR³ steht,
wobei
R³ für Wasserstoff steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidinring bzw Triazinring steht,
p für eine Zahl 0 oder 1 steht,
R^{4A} für Wasserstoff, Fluor, Methyl, Ethyl, Hydroxy oder Amino steht,
R^{4B} für Wasserstoff, Fluor, Difluormethyl, Trifluormethyl, Methyl, Ethyl, Methoxycarbonylamino, Cyclopropyl, Cyclobutyl, Cyclopentyl oder eine Gruppe der Formel -M-R⁸ steht,
worin Methyl und Ethyl mit 1 bis 3 Substituenten unabhängig voneinander ausgewählt aus der Gruppe Fluor, Cyano, Trifluormethyl, Cyclopropyl, Cyclobutyl, Hydroxy, Difluormethoxy, Trifluormethoxy, Methoxy, Ethoxy, Hydroxycarbonyl, Methoxycarbonyl, Ethoxycarbonyl und Amino substituiert sein kann,
und worin
M für eine Bindung steht,
R⁸ für -(C=O)ᵣ-NR⁹R¹⁰, Phenyl, Thiazolyl, Triazolyl, Oxadiazolyl, Thiadiazolyl oder Pyrimidinyl steht,
worin
r die Zahl 1 bedeutet,
R⁹ und R¹⁰ unabhängig voneinander jeweils für Wasserstoff, oder Cyclopropyl stehen,
und
worin Phenyl, Thiazolyl, Triazolyl, Oxadiazolyl, Thiadiazolyl und Pyrimidinyl ihrerseits mit 1 oder 2 Substituenten unabhängig voneinander ausgewählt aus der Gruppe Fluor, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl substituiert sein können, oder
R^{4A} und R^{4B} zusammen mit dem Kohlenstoffatom, an das sie gebunden sind, einen Cyclopropyl-, Cyclobutyl-, Cyclopentyl-, Azetidinyl-, Tetrahydrofuranyl-, Pyrrolidinyl- oder Tetrahydropyranyl-Ring bilden,
worin der Cyclopropyl-, Cyclobutyl-, Cyclopentyl-, Azetidinyl-, Tetrahydrofuranyl-, Pyrrolidinyl- und Tetrahydropyranyl-Ring mit 1 oder 2 Substituenten unabhängig voneinander ausgewählt aus der Gruppe Fluor und Methyl substituiert sein können,
R^{5A} für Wasserstoff, Fluor, Methyl oder Hydroxy steht,
R^{5B} für Wasserstoff, Fluor, Methyl oder Trifluormethyl steht,
- R¹: für Wasserstoff oder Fluor steht,
- R²: für 3,3,3-Trifluorprop-1-yl, 4,4,4-Trifluorbut-lyl, 3,3,4,4-Tetrafluorbut-lyl, 3,3,4,4,4-Pentafluorbut-1yl, Cyclobutylmethyl oder Cyclopentylmethyl steht,
wobei Cyclobutylmethyl und Cyclopentylmethyl mit 1 oder 2 Substituenten Fluor substituiert sein können,
- R^{7A}: für Wasserstoff oder Methyl steht,
- R^{7B}: für Wasserstoff steht,
sowie ihre Salze, Solvate und Solvate der Salze.

Besonders bevorzugt im Rahmen der vorliegenden Erfindung sind Verbindungen der Formel (I), in welcher
- A: für Stickstoff oder CR³ steht,
wobei
R³ für Wasserstoff steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidinring bzw Triazinring steht,
p für eine Zahl 0 steht,
R^{4A} für Wasserstoff, Fluor, Methyl oder Hydroxy steht,
R^{4B} für Wasserstoff, Fluor, Trifluormethyl, 2,2,2-Trifluorethyl, Methyl oder eine Gruppe der Formel -M-R⁸ steht,
worin
M für eine Bindung steht,
R⁸ für -(C=O)ᵣ-NR⁹R¹⁰ steht,
worin
r die Zahl 1 bedeutet,
R⁹ und R¹⁰ unabhängig voneinander jeweils für Wasserstoff, oder Cyclopropyl stehen,
- R¹: für Wasserstoff oder Fluor steht,
- R²: für 4,4,4-Trifluorbut-1yl, 3,3,4,4-Tetrafluorbut-1yl, 3,3,4,4,4-Pentafluorbut-1yl, Cyclobutylmethyl oder Cyclopentylmethyl steht,
wobei Cyclobutylmethyl und Cyclopentylmethyl mit 1 oder 2 Substituenten Fluor substituiert sein können,
- R^{7A}: für Wasserstoff oder Methyl steht,
- R^{7B}: für Wasserstoff steht,
sowie ihre Salze, Solvate und Solvate der Salze.

### Weiter offenbart sind Verbindungen der Formel (I-1)

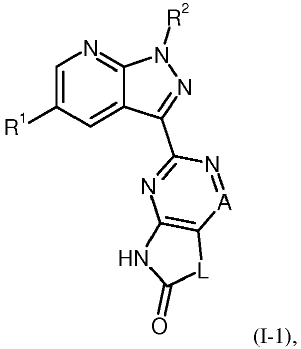

in welcher
- A: für Stickstoff oder CR³ steht,
wobei
R³ für Wasserstoff, Deuterium, Fluor, Difluormethyl, Trifluormethyl, (C₁-C₄)-Alkyl, Cyclopropyl oder Cyclobutyl steht,
- L: für eine Gruppe #-CR^{4A}R^{4B}-(CR^{5A}R^{5B})p-## steht,
wobei
# für die Anknüpfstelle an die Carbonylgruppe steht,
## für die Anknüpfstelle an den Pyrimidin- bzw. Triazinring steht,
p für eine Zahl 0, 1 oder 2 steht,
R^{4A} für Wasserstoff, Fluor, (C₁-C₄)-Alkyl, Hydroxy oder Amino steht,
R^{4B} für Wasserstoff, Fluor, (C₁-C₄)-Alkyl, Trifluormethyl, (C₁-C₄)-Alkoxycarbonylamino oder Phenyl steht,
worin (C₁-C₄)-Alkyl mit 1 bis 3 Substituenten unabhängig voneinander ausgewählt aus der Gruppe Fluor, Trifluormethyl, Hydroxy, Hydroxycarbonyl und (C₁-C₄)-Alkoxycarbonyl substituiert sein kann,
oder
R^{4A} und R^{4B} zusammen mit dem Kohlenstoffatom, an das sie gebunden sind, eine Oxo-Gruppe, einen 3- bis 6-gliedrigen Carbocyclus oder einen 4- bis 6-gliedrigen Heterocyclus bilden,
worin der 3- bis 6-gliedrigen Carbocyclus und der 4- bis 6-gliedrigen Heterocyclus mit 1 oder 2 Substituenten unabhängig voneinander ausgewählt aus der Gruppe Fluor und (C₁-C₄)-Alkyl substituiert sein können,
oder
R^{4A} und R^{4B} zusammen mit dem Kohlenstoffatom, an das sie gebunden sind, eine (C₂-C₄)-Alkenyl-Gruppe bilden,
R^{5A} für Wasserstoff, Fluor, (C₁-C₄)-Alkyl oder Hydroxy steht,
R^{5B} für Wasserstoff, Fluor, (C₁-C₄)-Alkyl oder Trifluormethyl steht,
- R¹: für Wasserstoff oder Fluor steht,
- R²: für eine Gruppe der Formel *-(CR^{6A}R^{6B})_{q}CF₃ steht,
wobei
* für die Anknüpfstelle an das Pyrazolopyridin steht,
q für eine Zahl 1, 2 oder 3 steht,
R^{6A} für Wasserstoff oder Fluor steht,
R^{6B} für Wasserstoff oder Fluor steht,
sowie ihre Salze, Solvate und Solvate der Salze.

Weiter offenbart sind Verbindungen der Formel (I-1), in welcher
- A: für Stickstoff oder CR³ steht,
wobei
R³ für Wasserstoff, Fluor, Difluormethyl, Trifluormethyl, Methyl, Ethyl oder Cyclopropyl steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidin- bzw. Triazinring steht,
p für eine Zahl 0 oder 1 steht,
R^{4A} für Wasserstoff, Fluor, Methyl, Ethyl oder Hydroxy steht,
R^{4B} für Wasserstoff, Fluor, Methyl, Ethyl, Trifluormethyl, Methoxycarbonylamino oder Phenyl steht,
worin Methyl und Ethyl mit 1 bis 3 Substituenten unabhängig voneinander ausgewählt aus der Gruppe Fluor, Trifluormethyl und Hydroxy substituiert sein können,
oder
R^{4A} und R^{4B} zusammen mit dem Kohlenstoffatom, an das sie gebunden sind, einen Cyclopropyl-, Cyclobutyl-, Cyclopentyl-, Azetidinyl-, Pyrrolidinyl-, Tetrahydrofuranyl-, Piperidinyl- oder Tetrahydropyranyl-Ring bilden,
worin der Cyclopropyl-, Cyclobutyl-, Cyclopentyl-, Azetidinyl-, Pyrrolidinyl-, Tetrahydrofuranyl-, Piperidinyl- und Tetrahydropyranyl-Ring mit 1 oder 2 Substituenten unabhängig voneinander ausgewählt aus der Gruppe Fluor und Methyl substituiert sein können,
R^{5A} für Wasserstoff, Fluor, Methyl, Ethyl oder Hydroxy steht,
R^{5B} für Wasserstoff, Fluor, Methyl, Ethyl oder Trifluormethyl steht,
- R¹: für Wasserstoff oder Fluor steht,
- R²: für 2,2,2-Trifluorethyl, 3,3,3-Trifluorprop-1-yl, 4,4,4-Trifluorbut-1yl oder 3,3,4,4,4-Pentafluorbut-1yl steht,
sowie ihre Salze, Solvate und Solvate der Salze.

Weiter offenbart sind Verbindungen der Formel (I-1), in welcher
- A: für CR³ steht,
wobei
R³ für Wasserstoff steht,
- L: für eine Gruppe #-CR^{4A}R^{4B}-(CR^{5A}R^{5B})ₚ-## steht,
wobei
# für die Anlcnüpfstelle an die Carbonylgruppe steht,
## für die Anknüpfstelle an den Pyrimidinring steht,
p für eine Zahl 0 steht,
R^{4A} für Wasserstoff, Fluor, Methyl oder Hydroxy steht,
R^{4B} für Wasserstoff, Fluor, Methyl oder Trifluormethyl steht,
oder
R^{4A} und R^{4B} zusammen mit dem Kohlenstoffatom, an das sie gebunden sind, einen Cyclopropyl- oder Cyclobutyl-Ring bilden,
worin der Cyclopropyl- und der Cyclobutyl-Ring mit 1 oder 2 Substituenten unabhängig voneinander ausgewählt aus der Gruppe Fluor und Methyl substituiert sein können,
- R¹: für Wasserstoff oder Fluor steht,
- R²: für 3,3,4,4,4-Pentafluorbut-lyl steht,
sowie ihre Salze, Solvate und Solvate der Salze.

Weiter offenbart sind auch Verbindungen der Formel (I), in welcher
- A: für Stickstoff steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Triazinring steht,
p für eine Zahl 0 steht,
R^{4A} für Wasserstoff, Fluor, Methyl oder Hydroxy steht,
R^{4B} für Wasserstoff, Fluor, Methyl oder Trifluormethyl steht,
oder
R^{4A} und R^{4B} zusammen mit dem Kohlenstoffatom, an das sie gebunden sind, einen Cyclopropyl- oder Cyclobutyl-Ring bilden,
worin der Cyclopropyl- und der Cyclobutyl-Ring mit 1 oder 2 Substituenten unabhängig voneinander ausgewählt aus der Gruppe Fluor und Methyl substituiert sein können,
- R¹: für Wasserstoff oder Fluor steht,
- R²: für 3,3,4,4,4-Pentafluorbut-lyl steht,
sowie ihre Salze, Solvate und Solvate der Salze.

Weiter offenbart sind auch Verbindungen der Formel (I) und (I-1), in welcher R¹ für H steht, sowie ihre Salze, Solvate und Solvate der Salze.

Weiter offenbart sind auch Verbindungen der Formel (I) und (I-1), in welcher R¹ für Fluor steht, sowie ihre Salze, Solvate und Solvate der Salze.

Weiter offenbart sind auch Verbindungen der Formel (I) und (I-1), in welcher A für N oder CH steht, sowie ihre Salze, Solvate und Solvate der Salze.

Weiter offenbart sind auch Verbindungen der Formel (I) und (I-1), in welcher A für CH steht, sowie ihre Salze, Solvate und Solvate der Salze.

Weiter offenbart sind auch Verbindungen der Formel (I) und (I-1), in welcher A für N steht, sowie ihre Salze, Solvate und Solvate der Salze.

Weiter offenbart sind Verbindungen der Formel (I) und (I-1), in welcher
- L: für eine Gruppe #-CR^{4A}R^{4B}-(CR^{5A}R^{5B})ₚ-## steht
wobei
# für die Anknüpfstelle an die Carbonylgruppe steht,
## für die Anknüpfstelle an den Pyrimidin- bzw. Triazinring steht,
p für eine Zahl 0 steht,
R^{4A} und R^{4B} zusammen mit dem Kohlenstoffatom, an das sie gebunden sind, einen Cyclopropyl-, Cyclobutyl-, Cyclopentyl-, Azetidinyl-, Pyrrolidinyl-, Tetrahydrofuranyl-, Piperidinyl- oder Tetrahydropyranyl-Ring bilden,
sowie ihre Salze, Solvate und Solvate der Salze.

Weiter offenbart sind auch Verbindungen der Formel (I) und (I-1), in welcher
- L: für eine Gruppe #-CR^{4A}R^{4B}-(CR^{5A}R^{5B})ₚ-## steht
wobei
# für die Anlcnüpfstelle an die Carbonylgruppe steht,
## für die Anknüpfstelle an den Pyrimidin- bzw. Triazinring steht,
p für eine Zahl 0 steht,
R^{4A} und R^{4B} zusammen mit dem Kohlenstoffatom, an das sie gebunden sind, einen Azetidinyl-, Pyrrolidinyl-, Tetrahydrofuranyl-, Piperidinyl- oder Tetrahydropyranyl-Ring bilden, sowie ihre Salze, Solvate und Solvate der Salze.

Weiter offenbart sind auch Verbindungen der Formel (I) und (I-1), in welcher
- L: für eine Gruppe #-CR^{4A}R^{4B}-(CR^{5A}R^{5B})ₚ-## steht
wobei
# für die Anlcnüpfstelle an die Carbonylgruppe steht,
## für die Anknüpfstelle an den Pyrimidin- bzw. Triazinring steht,
p für eine Zahl 0 steht,
R^{4A} für Wasserstoff, Fluor, Methyl, oder Hydroxy steht,
R^{4B} für Wasserstoff, Fluor, Methyl oder Trifluormethyl steht,
sowie ihre Salze, Solvate und Solvate der Salze.

Weiter offenbart sind auch Verbindungen der Formel (I) und (I-1), in welcher
- L: für eine Gruppe #-CR^{4A}R^{4B}-(CR^{5A}R^{5B})ₚ-## steht
wobei
# für die Anknüpfstelle an die Carbonylgruppe steht,
## für die Anknüpfstelle an den Pyrimidin- bzw. Triazinring steht,
p für eine Zahl 0 steht,
R^{4A} für Wasserstoff, Fluor, Methyl, Hydroxy oder Amino steht,
R^{4B} für Wasserstoff, Fluor, Methyl, Trifluormethyl, 2,2,2-Trifluorethyl oder 1,1,2,2,2-Pentafluorethyl steht,
sowie ihre Salze, Solvate und Solvate der Salze.

Weiter offenbart sind auch Verbindungen der Formel (I) und (I-1), in welcher
- L: für eine Gruppe #-CR^{4A}R^{4B}-(CR^{5A}R^{5B})ₚ-## steht
wobei
# für die Anknüpfstelle an die Carbonylgruppe steht,
## für die Anknüpfstelle an den Pyrimidin- bzw. Triazinring steht,
p für eine Zahl 0 steht,
R^{4A} für Hydroxy oder Amino steht,
R^{4B} für Trifluormethyl, 2,2,2-Trifluorethyl oder 1,1,2,2,2-Pentafluorethyl steht,
sowie ihre Salze, Solvate und Solvate der Salze.

Weiter offenbart sind auch Verbindungen der Formel (I) und (I-1), in welcher
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidin- bzw. Triazinring steht,
p für eine Zahl 0 steht,
R^{4A} für Methyl steht,
R^{4B} für Methyl steht,
sowie ihre Salze, Solvate und Solvate der Salze.

Weiter offenbart sind auch Verbindungen der Formel (I) und (I-1), in welcher
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidin- bzw. Triazinring steht,
p für eine Zahl 0 steht,
R^{4A} und R^{4B} zusammen mit dem Kohlenstoffatom, an das sie gebunden sind, einen Cyclopropyl- oder Cyclobutyl-Ring bilden,
worin der Cyclopropyl- und der Cyclobutyl-Ring mit 1 oder 2 Substituenten unabhängig voneinander ausgewählt aus der Gruppe Fluor und Methyl substituiert sein können,
sowie ihre Salze, Solvate und Solvate der Salze.

Weiter offenbart sind auch Verbindungen der Formel (I) und (I-1), in welcher
- R²: für 3,3,4,4,4-Pentafluorbut-lyl steht,
sowie ihre Salze, Solvate und Solvate der Salze.

Weiter offenbart sind auch Verbindungen der Formel (I), in welcher
- A: für Stickstoff steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Triazinring steht,
p für eine Zahl 0 steht,
R^{4A} für Wasserstoff, Fluor, Methyl oder Hydroxy steht,
R^{4B} für Wasserstoff, Fluor, Trifluormethyl, 2,2,2-Trifluorethyl oder Methyl steht,
sowie ihre Salze, Solvate und Solvate der Salze.

Weiter offenbart sind auch Verbindungen der Formel (I), in welcher
- A: für CR³ steht,
wobei
R³ für Wasserstoff steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidinring steht,
p für eine Zahl 0 steht,
R^{4A} für Wasserstoff, Fluor, Methyl oder Hydroxy steht,
R^{4B} für Wasserstoff, Fluor, Trifluormethyl, 2,2,2-Trifluorethyl oder Methyl steht,
sowie ihre Salze, Solvate und Solvate der Salze.

Weiter offenbart sind auch Verbindungen der Formel (I), in welcher
- A: für CR³ steht,
wobei
R³ für Wasserstoff steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidinring steht,
p für eine Zahl 0 steht,
R^{4A} für Wasserstoff, Fluor, Methyl, Ethyl, Hydroxy oder Amino steht,
R^{4B} für eine Gruppe der Formel -M-R⁸ steht,
worin
M für eine Bindung steht,
R⁸ für -(C=O)ᵣ-NR⁹R¹⁰, -C(=S)-NR⁹R¹⁰, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 bedeutet,
R⁹ und R¹⁰ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl ihrerseits mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können,
sowie ihre Salze, Solvate und Solvate der Salze.

Weiter offenbart sind auch Verbindungen der Formel (I), in welcher
- A: für N steht,
- L: für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Triazinring steht,
p für eine Zahl 0 steht,
R^{4A} für Wasserstoff, Fluor, Methyl, Ethyl, Hydroxy oder Amino steht,
R^{4B} für eine Gruppe der Formel -M-R⁸ steht,
worin
M für eine Bindung steht,
R⁸ für -(C=O)ᵣ-NR⁹R¹⁰, -C(=S)-NR⁹R¹⁰, Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl oder Pyrazinyl steht,
worin
r die Zahl 0 bedeutet,
R⁹ und R¹⁰ unabhängig voneinander jeweils für Wasserstoff, Methyl, Ethyl, iso-Propyl, Cyclopropyl, Cyclobutyl, Cyclopentyl, Oxetanyl, Azetidinyl, Tetrahydrofuranyl, Pyrrolidinyl, Tetrahydropyranyl, Piperidinyl, Piperazinyl, Morpholinyl, Phenyl, Pyrazolyl oder Pyridyl stehen,
worin Methyl, Ethyl und iso-Propyl ihrerseits mit 1 oder 2 Substituenten unabhängig voneinander ausgewählt aus der Gruppe Fluor, Difluormethyl, Trifluormethyl, Cyclopropyl, Cyclobutyl, Cyclopentyl, Hydroxy, Difluormethoxy, Trifluormethoxy, Methoxy, Ethoxy, Hydroxycarbonyl, Methoxycarbonyl, Ethoxycarbonyl und Amino substituiert sein können, und
worin Oxadiazolonyl, Oxadiazolthionyl, Phenyl, Oxazolyl, Thiazolyl, Pyrazolyl, Triazolyl, Oxadiazolyl, Thiadiazolyl, Pyridyl, Pyrimidinyl und Pyrazinyl ihrerseits mit 1 oder 2 Substituenten unabhängig voneinander ausgewählt aus der Gruppe Fluor, Chlor, Cyano, Difluormethyl, Trifluormethyl, Methyl, Ethyl, iso-Propyl, 2,2,2-Trifluorethyl, 1,1,2,2,2-Pentafluorethyl, Cyclopropyl, Cyclobutyl, Cyclopropylmetyl, Cyclobutylmethyl, Hydroxy, Methoxy und Ethoxy substituiert sein können,
sowie ihre Salze, Solvate und Solvate der Salze.

Die in den jeweiligen Kombinationen bzw. bevorzugten Kombinationen von Resten im einzelnen angegebenen Reste-Definitionen werden unabhängig von den jeweiligen angegebenen Kombinationen der Reste beliebig auch durch Reste-Definitionen anderer Kombinationen ersetzt.

Weiter offenbart sind Kombinationen von zwei oder mehreren der oben genannten Vorzugsbereiche.

Weiterer Gegenstand der Erfindung ist ein Verfahren zur Herstellung der erfindungsgemäßen Verbindungen der Formel (I) dadurch gekennzeichnet, dass man eine Verbindung der Formel (II) 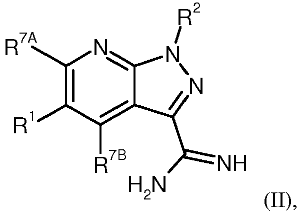 in welcher R¹, R², R^{7A} und R^{7B} jeweils die oben genannten Bedeutungen haben,
[A] in einem inerten Lösungsmittel in Gegenwart einer geeigneten Base mit einer Verbindung der Formel (III) 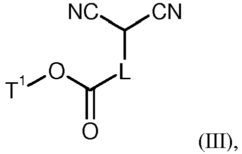
   in welcher L die oben genannte Bedeutung hat und
   T¹ für (C₁-C₄)-Alkyl steht,
   zu einer Verbindung der Formel (IV) 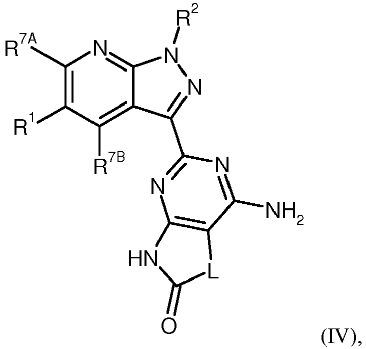
   in welcher L, R¹, R², R^{7A} und R^{7B} jeweils die oben angegebenen Bedeutungen haben,
   umsetzt, diese dann mit iso-Pentylnitrit und einem Halogen-Äquivalent in eine Verbindung der Formel (V) 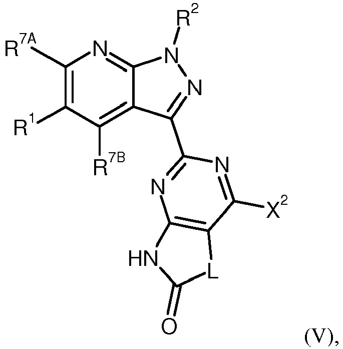
   in welcher L, R¹, R², R^{7A} und R^{7B} jeweils die oben angegebenen Bedeutungen haben und
   X² für Brom oder Iod steht,
   überführt, und diese im Anschluss in einen inerten Lösungsmittel in Gegenwart eines geeigneten Übergangsmetallkatalysators zu einer Verbindung der Formel (I-A) 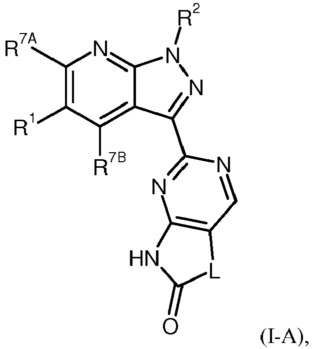
   in welcher L, R¹, R², R^{7A} und R^{7B} jeweils die oben angegebenen Bedeutungen haben,
      umsetzt,
   oder
[B] in einem inerten Lösungsmittel in Gegenwart einer geeigneten Base mit Hydrazinhydrat zu einer Verbindung der Formel (VI) 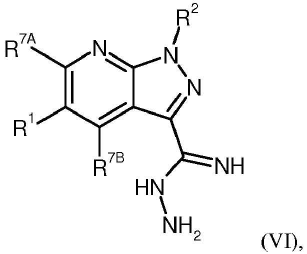
   in welcher R¹, R², R^{7A} und R^{7B} jeweils die oben genannten Bedeutungen haben,
   umsetzt, diese dann in einem inerten Lösungsmittel mit einer Verbindung der Formel (VII) 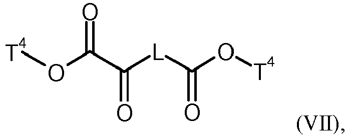
   in welcher L die oben angegebene Bedeutung hat und
   T⁴ für (C₁-C₄)-Alkyl steht,
   zu einer Verbindung der Formel (VIII) 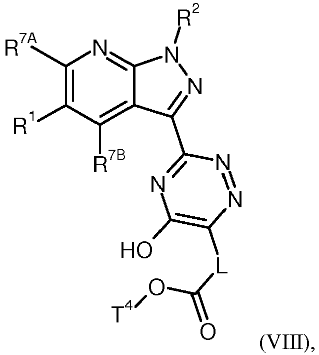
   in welcher L, R¹, R², R^{7A}, R^{7B} und T⁴ jeweils die oben angegebenen Bedeutungen haben,
   reagiert, diese anschliessend mit Phosphorylchlorid in eine Verbindung der Formel (IX) 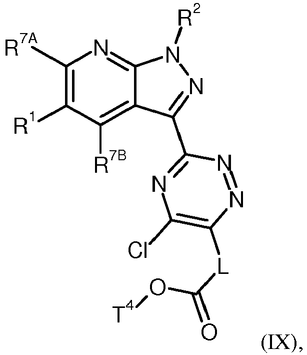
   in welcher L, R¹, R², R^{7A}, R^{7B} und T⁴ jeweils die oben angegebenen Bedeutungen haben,
   überführt, und diese direkt mit Ammoniak zu einer Verbindung der Formel (X) 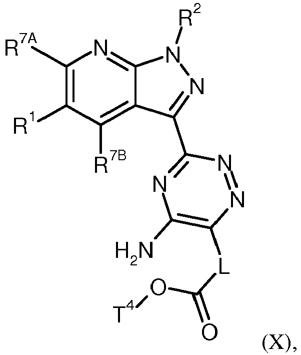
   in welcher L, R¹, R², R^{7A}, R^{7B} und T⁴ jeweils die oben angegebenen Bedeutungen haben,
   umsetzt, und schliesslich in einem inerten Lösungsmittel gegebenenfalls in Gegenwart einer geeigneten Base zu einer Verbindung der Formel (I-B) 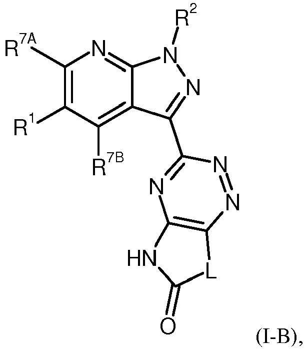
   in welcher L, R¹, R², R^{7A} und R^{7B} jeweils die oben angegebenen Bedeutungen haben,
   cyclisiert,
und gegebenenfalls die resultierenden Verbindungen der Formeln (I-A) und (I-B) gegebenenfalls mit den entsprechenden (i) Lösungsmitteln und/oder (ii) Säuren oder Basen in ihre Solvate, Salze und/oder Solvate der Salze überführt.

Die Verbindungen der Formeln (I-A) und (I-B) bilden zusammen die Gruppe der erfindungsgemäßen Verbindungen der Formel (I).

Inerte Lösungsmittel für den Verfahrensschritt (II) + (III) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N.N'-*Dimethylpropylenharnstoff (DMPU), *N*-Methylpyrrolidon (NMP), Pyridin, Acetonitril, Sulfolan oder auch Wasser. Ebenso ist es möglich, Gemische der genannten Lösungsmittel einzusetzen. Bevorzugt ist tert.-Butanol oder Methanol.

Geeignete Basen für den Verfahrensschritt (II) + (III) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kaliumtert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Kalium-tert.-butylat oder Natriummethanolat.

Die Reaktion (II) + (III) wird im Allgemeinen in einem Temperaturbereich von +20°C bis +150°C, bevorzugt bei +75°C bis +100°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Der Verfahrensschritt (IV) → (V) erfolgt mit oder ohne Lösungsmittel. Als Lösungsmittel eignen sich alle organischen Lösungsmittel, die unter den Reaktionsbedingungen inert sind. Bevorzugtes Lösungsmittel ist Dimethoxyethan.

Die Reaktion (IV) → (V) erfolgt im Allgemeinen in einem Temperaturbereich von +20°C bis +100°C, bevorzugt im Bereich von +50°C bis +100°C, gegebenenfalls in einer Mikrowelle. Die Umsetzung kann bei normalem, erhöhtem oder erniedrigtem Druck durchgeführt werden (z.B. im Bereich von 0.5 bis 5 bar). Im Allgemeinen arbeitet man bei Normaldruck.

Als Halogen-Quelle bei der Umsetzung (IV) → (V) eignen sich beispielsweise Düodmethan, eine Mischung aus Cäsiumiodid, Iod und Kupfer-(I)-iodid oder Kupfer -(II)-bromid.

Der Verfahrensschritt (IV) → (V) erfolgt im Fall von Diiodmethan als Halogenquelle mit einem Molverhältnis von 10 bis 30 Mol Isopentylnitrit und 10 bis 30 Mol des Iod-Äquivalents bezogen auf 1 Mol der Verbindung der Formel (IV).

Inerte Lösungsmittel für den Verfahrensschritt (V) → (I-A) sind Alkohole wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, oder andere Lösungsmittel wie Dimethylformamid (DMF), *N,N*'-Dimethylpropylenharnstoff (DMPU), N-Methylpyrrolidon (NMP), Pyridin oder auch Wasser. Ebenso ist es möglich, Gemische der genannten Lösungsmittel einzusetzen. Bevorzugt ist DMF.

Die Reduktion (V) → (I-A) erfolgt mit Wasserstoff in Verbindung mit Übergangsmetallkatalysatoren wie beispielsweise Palladium (10% auf Aktivkohle), Raney-Nickel oder Palladiumhydroxid.

Die Reaktion (V) → (I-A) erfolgt im Allgemeinen in einem Temperaturbereich von +20°C bis +50°C. Die Umsetzung kann bei normalem oder erhöhtem Druck durchgeführt werden (z.B. im Bereich von 0.5 bis 5 bar). Im Allgemeinen arbeitet man bei Normaldruck.

Die Umsetzung (VIII) → (IX) kann in einem unter den Reaktionsbedingungen inerten Lösungsmittel oder ohne Lösungsmittel erfolgen. Bevorzugtes Lösungsmittel ist Sulfolan.

Die Reaktion (VIII) → (IX) erfolgt im Allgemeinen in einem Temperaturbereich von +70°C bis +150°C, bevorzugt von +80°C bis +130°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Insbesondere bevorzugt erfolgt die Umsetzung (VIII) → (IX) ohne Lösungsmittel in einem Temperaturbereich von 0°C bis +50°C bei Normaldruck.

Der Verfahrensschritt (IX) → (X) erfolgt in einem unter den Reaktionsbedingungen inerten Lösungmittel. Geeignete Lösungsmittel sind beispielsweise Ether wie Diethylether, Dioxan, Tetrahydrofuran, Glykoldimethylether oder Diethylenglykoldimethylether, oder anderen Lösungsmitteln wie Dimethylformamid (DMF), Dimethylsulfoxid (DMSO), *N,N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist Acetonitril.

Die Reaktion (IX) → (X) erfolgt im Allgemeinen in einem Temperaturbereich von +20°C bis +100°C, bevorzugt von +40°C bis +70°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Die Cyclisierung (X) → (I-B) erfolgt in einem unter den Reaktionsbedingungen inerten Lösungsmittel wie beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran (THF), Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N*'-Dimethylpropylenharnstoff (DMPU), N-Methylpyrrolidon (NMP), Pyridin, Acetonitril oder Sulfolan. Ebenso ist es möglich, Gemische der genannten Lösungsmittel einzusetzen. Bevorzugt ist THF.

Geeignete Basen für den Verfahrensschritt (X) → (I-B) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Kalium-tert.-butylat.

Die Reaktion (X) → (I-B) erfolgt im Allgemeinen in einem Temperaturbereich von 0°C bis +50°C, bevorzugt von +10°C bis +30°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Bevorzugt erfolgt die Cyclisierung zu (I-B) direkt bei der Umsetzung (IX) → (X) ohne Zugabe weiterer Reagenzien.

In einer alternativen Durchführung des Verfahrens [B] wird die Umwandlung (VI) + (VII) → (VIII) → (IX) → (X) → (I-B) ohne Isolierung der Zwischenstufen durchgeführt.

Bevorzugt erfolgen die Umsetzungen (VIII) → (IX) → (X) → (I-B) ohne Isolierung der Zwischenstufen.

Inerte Lösungsmittel für den Verfahrensschritt (VI) + (VII) → (VIII) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N*'-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist Methanol oder Ethanol.

Die Reaktion (VI) + (VII) → (VIII) erfolgt im Allgemeinen in einem Temperaturbereich von +50°C bis +120°C, bevorzugt von +50°C bis +100°C, gegebenenfalls in einer Mikrowelle. Die Umsetzung kann bei normalem oder erhöhtem Druck durchgeführt werden (z.B. im Bereich von 0.5 bis 5 bar). Im Allgemeinen arbeitet man bei Normaldruck.

Inerte Lösungsmittel für den Verfahrensschritt (II) → (VI) sind beispielsweise Alkohole wie Methanol, Ethanol, n-Propanol, Isopropanol, n-Butanol oder tert.-Butanol, Ether wie Diethylether, Dioxan, Dimethoxyethan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N.N'-*Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist Ethanol.

Geeignete Basen für den Verfahrensschritt (II) → (VI) sind Alkalihydroxide wie beispielsweise Lithium-, Natrium- oder Kaliumhydroxid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt Triethylamin.

Die Reaktion (II) → (VI) erfolgt im Allgemeinen in einem Temperaturbereich von 0°C bis +60°C, bevorzugt von +10°C bis +30°C. Die Umsetzung kann bei normalem oder erhöhtem Druck durchgeführt werden (z.B. im Bereich von 0.5 bis 5 bar). Im Allgemeinen arbeitet man bei Normaldruck.

Die beschriebenen Herstellverfahren können durch die folgenden Syntheseschemata (Schema 1 und 2) beispielhaft verdeutlicht werden: 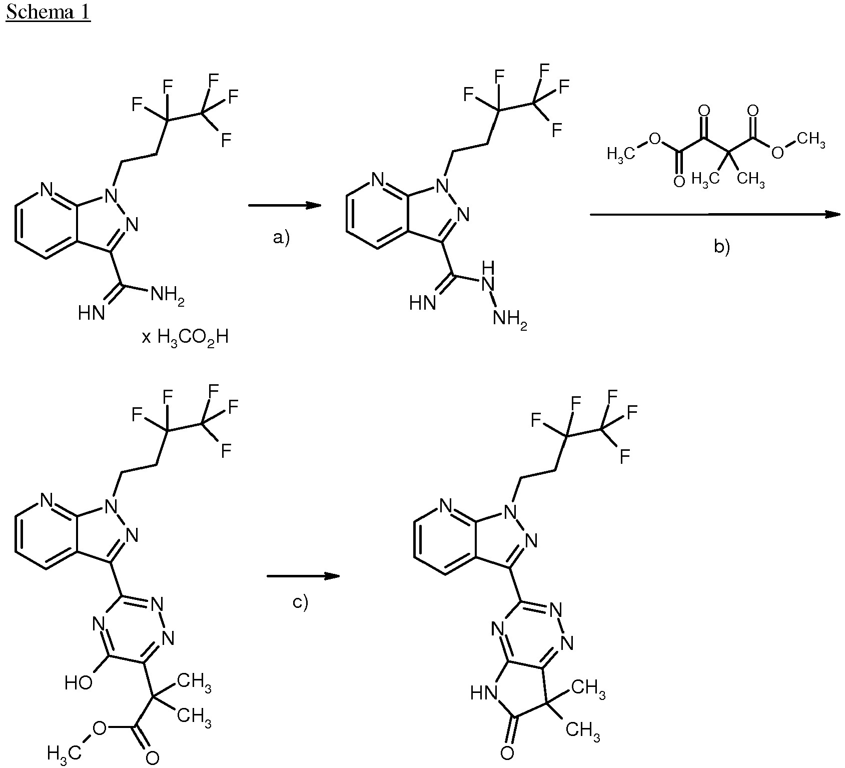 [a): Hydrazinhydrat, NEt₃, EtOH b): EtOH c): 1. POCl₃; 2. konz. NH₃, Acetonitril]. 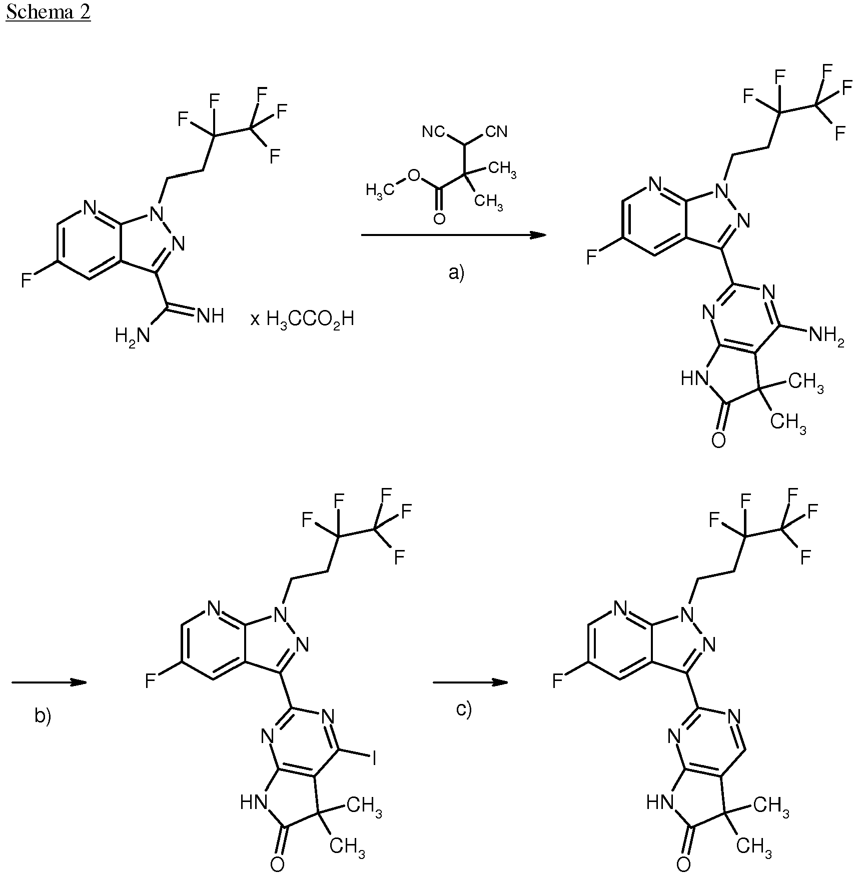 [a): KOt-Bu, tert.-Butanol; b): Diiodmethan, iso-Pentylnitrit; c): Pd/C, Wasserstoff, DMF].

In einem alternativen Verfahren kann die Herstellung der erfindungsgemäßen Verbindungen der Formel (I) durch Umkehrung der Reaktitionsschritte unter Verwendung von Schutzgruppenchemie erfolgen, wie im nachfolgenden Syntheseschema (Schema 6) beispielhaft gezeigt: 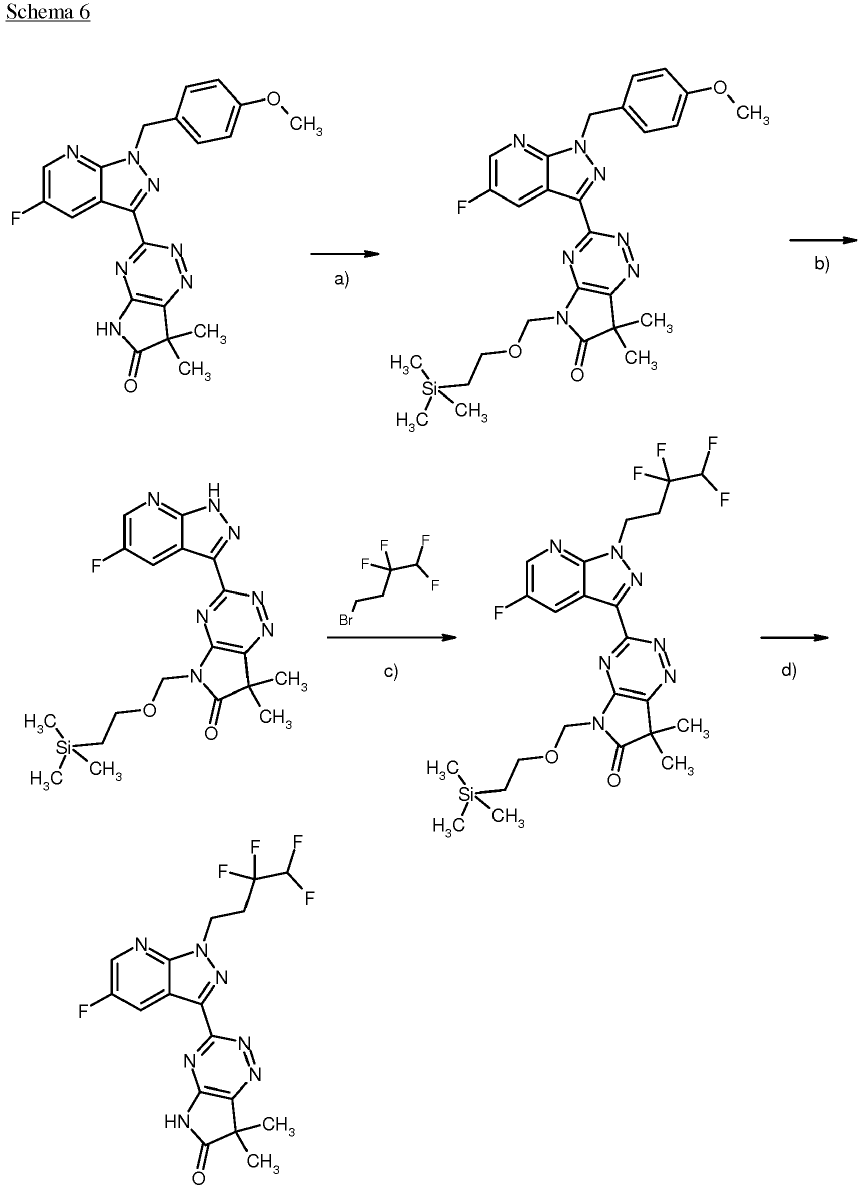 [a): 2-(Trimethylsilyl)ethoxymethylchlorid, Cs₂CO₃, DMF; b): Ammoniumcer(IV)-nitrat, Acetonitril, Wasser; c): Cs₂CO₃,DMF; d): 1) TFA, Dichlormethan, 2) HCl, Ethanol].

Weitere erfindungsgemäße Verbindungen können gegebenenfalls auch hergestellt werden durch Umwandlungen von funktionellen Gruppen einzelner Substituenten, insbesondere den unter L und R³ aufgeführten, ausgehend von den nach obigen Verfahren erhaltenen Verbindungen der Formel (I). Diese Umwandlungen werden nach üblichen, dem Fachmann bekannten Methoden durchgeführt und umfassen beispielsweise Reaktionen wie nukleophile und elektrophile Substitutionen, Oxidationen, Reduktionen, Hydrierungen, Übergangsmetall-katalysierte Kupplungsreaktionen, Eliminierungen, Alkylierung, Aminierung, Veresterung, Esterspaltung, Veretherung, Etherspaltung, Bildung von Carbonamide, sowie Einführung und Entfernung temporärer Schutzgruppen.

Die Verbindungen der Formel (II) können hergestellt werden, indem man eine Verbindung der Formel (XI) 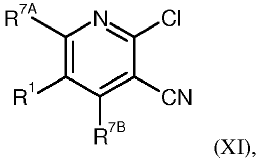 in welcher R¹, R^{7A} und R^{7B} jeweils die oben angegebenen Bedeutungen haben,
in einem inerten Lösungsmittel mit Hydrazinhydrat zur Verbindung der Formel (XII) 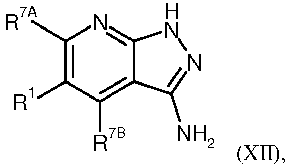 in welcher R¹, R^{7A} und R^{7B} jeweils die oben angegebenen Bedeutungen haben,
zyklisiert, diese anschliessend in einem inerten Lösungsmittel in Gegenwart einer geeigneten Lewis-Säure zunächst mit Isopentylnitrit zum entsprechenden Diazoniumsalz umsetzt, und dieses dann direkt mit Natriumiodid in die Verbindung der Formel (XIII) 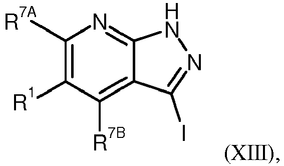 in welcher R¹, R^{7A} und R^{7B} jeweils die oben angegebenen Bedeutungen haben,
überführt, diese im Folgenden in einem inerten Lösungsmittel in Gegenwart einer geeigneten Base mit der Verbindung der Formel (XIV)

R²-X¹ (XIV),

in welcher R² die oben angegebene Bedeutung hat und
- X¹: für eine geeignete Abgangsgruppe, wie beispielsweise Halogen, Tosylat oder Mesylat, steht,
in eine Verbindung der Formel (XV) 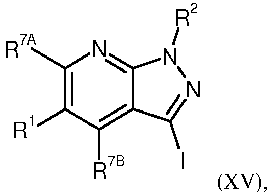 in welcher R¹, R², R^{7A} und R^{7B} jeweils die oben angegebenen Bedeutungen haben,
überführt, diese anschließend in einem inerten Lösungsmittel mit Kupfercyanid zu einer Verbindung der Formel (XVI) 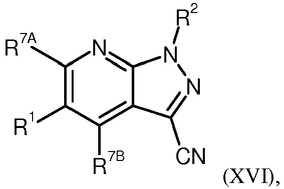 in welcher R¹, R², R^{7A} und R^{7B} jeweils die oben angegebenen Bedeutungen haben,
reagiert, und diese schließlich unter sauren Bedingungen mit einem Ammoniak-Äquivalent umsetzt.

Inerte Lösungsmittel für den Verfahrensschritt (XI) → (XII) sind Alkohole wie Methanol, Ethanol, n-Propanol, Isopropanol, n-Butanol, tert.-Butanol oder 1,2-Ethandiol, Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N*'-Dimethylpropylenharnstoff (DMPU), *N*-Methylpyrrolidon (NMP), Pyridin, Acetonitril oder auch Wasser. Ebenso ist es möglich, Gemische der genannten Lösungsmittel einzusetzen. Bevorzugt ist 1,2-Ethandiol.

Die Reaktion (XI) → (XII) wird im Allgemeinen in einem Temperaturbereich von +60°C bis +200°C, bevorzugt bei +120°C bis +180°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für die Umsetzung (XII) → (XIII) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N,N*'-Dimethylpropylenharnstoff (DMPU), *N*-Methylpyrrolidon (NMP), Pyridin oder Acetonitril. Bevorzugt ist DMF.

Als Lewis-Säuren für den Verfahrensschritt (XII) → (XIII) eignen sich Bortrifluorid-Diethylether-Komplex, Cer(IV)ammoniumnitrat (CAN), Zinn(II)chlorid, Lithiumperchlorat, Zink(II)chlorid, Indium(III)chlorid oder Indium(III)bromid. Bevorzugt ist Bortrifluorid-Diethylether-Komplex.

Die Reaktion (XII) → (XIII) wird im Allgemeinen in einem Temperaturbereich von -78°C bis +40°C, bevorzugt bei 0°C bis +20°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für die Umsetzung (XIII) + (XIV) → (XV) sind beispielsweise Halogenkohlenwasserstoffe wie Dichlormethan, Trichlormethan, Tetrachlormethan, Trichlorethylen oder Chlorbenzol, Ether wie Diethylether, Dioxan, Tetrahydrofuran, Glykoldimethylether oder Diethylenglykoldimethylether, oder andere Lösungsmittel wie Dimethylformamid (DMF), Dimethylsulfoxid (DMSO), *N,N*'-Dimethylpropylenharnstoff (DMPU), *N*-Methylpyrrolidon (NMP), Pyridin, Acetonitril. Bevorzugt ist DMF.

Geeignete Basen für den Verfahrensschritt (XIII) + (XIV) → (XV) sind Alkalihydride wie Kaliumhydrid oder Natriumhydrid, Alkalicarbonate wie Lithium-, Natrium-, Kalium- oder Cäsiumcarbonat, Alkalihydrogencarbonate wie Natrium- oder Kaliumhydrogencarbonat, Alkalialkoholate wie Natrium- oder Kaliummethanolat, Natrium- oder Kaliumethanolat oder Kalium-tert.-butylat, Amide wie Natriumamid, Lithium-, Natrium- oder Kalium-bis-(trimethylsilyl)amid oder Lithiumdiisopropylamid, metallorganische Verbindungen wie Butyllithium oder Phenyllithium, oder organische Amine wie Triethylamin, Diisopropylethylamin, Pyridin, 1,8-Diazabicyclo[5.4.0]undec-7-en (DBU) oder 1,5-Diazabicyclo[4.3.0]non-5-en (DBN). Bevorzugt ist Cäsiumcarbonat.

Die Reaktion (XIII) + (XIV) → (XV) wird im Allgemeinen in einem Temperaturbereich von 0°C bis +60°C, bevorzugt bei +10°C bis +25°C, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Inerte Lösungsmittel für den Verfahrensschritt (XV) → (XVI) sind beispielsweise Ether wie Diethylether, Dioxan, Tetrahydrofuran, Glykoldimethylether oder Diethylenglykoldimethylether, Kohlenwasserstoffe wie Benzol, Xylol, Toluol, Hexan, Cyclohexan oder Erdölfraktionen, oder andere Lösungsmittel wie Dimethylformamid (DMF), Dimethylsulfoxid (DMSO), *N,N*'-Dimethylpropylenharnstoff (DMPU), *N*-Methylpyrrolidon (NMP), Pyridin oder Acetonitril. Ebenso ist es möglich, Gemische der genannten Lösungsmittel einzusetzen. Bevorzugt ist DMSO.

Die Reaktion (XV) → (XVI) wird im Allgemeinen in einem Temperaturbereich von +20°C bis +180°C, bevorzugt bei +100°C bis +160°C, gegebenenfalls in einer Mikrowelle, durchgeführt. Die Umsetzung kann bei normalem, erhöhtem oder bei erniedrigtem Druck erfolgen (z.B. von 0.5 bis 5 bar). Im Allgemeinen arbeitet man bei Normaldruck.

Die Umsetzung (XVI) → (II) erfolgt nach den dem Fachmann bekannten Methoden in einem zweistufigen Prozess zunächst unter Bildung des Iminoesters mit Natriummethanolat in Methanol bei 0°C bis +40°C und anschliessender nucleophiler Addition eines Ammoniak-Äquivalents wie beispielsweise Ammoniak oder Ammoniumchlorid in einer geeigneten Säure unter Bildung des Amidins (III) bei +50 bis +150°C.

Geeignete Säure für die Bildung des Amidins (II) sind anorganische Säuren wie beispielsweise Chlorwasserstoff/ Salzsäure, Schwefelsäure, Polyphosphorsäure oder Phosphorsäure oder organische Säuren wie beispielsweise Essigsäure, Trifluoressigsäure oder Ameisensäure. Bervorzugt werden Salzsäure oder Essigsäure verwendet.

Das beschriebene Herstellverfahren kann durch das folgende Syntheseschema (Schema 3) beispielhaft verdeutlicht werden: 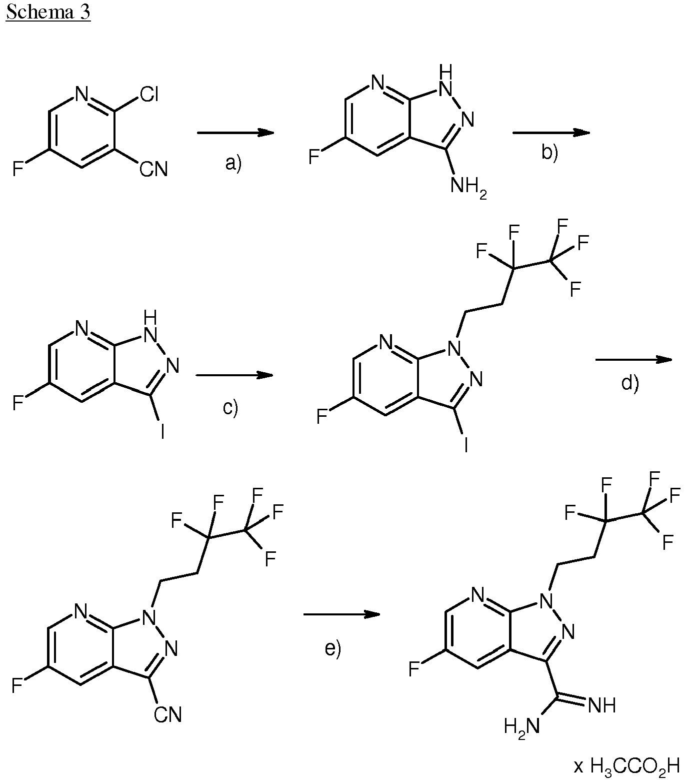 [a): Hydrazinhydrat, 1,2-Ethandiol; b): iso-Pentylnitrit, NaI, THF; b): 1,1,1,2,2-Pentafluor-4-iodbutan, Cs₂CO₃, DMF; d): CuCN, DMSO, e): 1. NaOMe, MeOH, 2. NH₄Cl, Esssigsäure].

Alternativ erfolgt die Herstellung der Verbindungen der Formel (II) wie im nachfolgenden Syntheseschema (Schema 4) gezeigt: 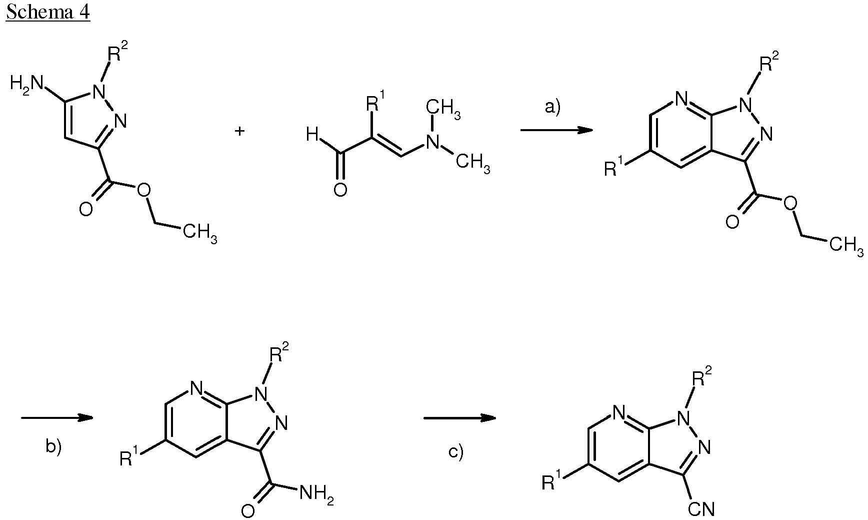 [a): TFA, Dioxan; b) NH₃; c) Trifluoressigsäureanhydrid].

Die Verbindung der Formel (XI) ist literaturbekannt [vgl. z.B. Winn M., J. Med. Chem. 1993, 36, 2676-7688; EP 634 413-A1; CN 1613849-A; EP 1626045-A1; WO 2009/018415], kann in Analogie zu literaturbekannten Verfahren oder wie im nachstehenden Syntheseschema gezeigt (Schema 5) hergestellt werden: 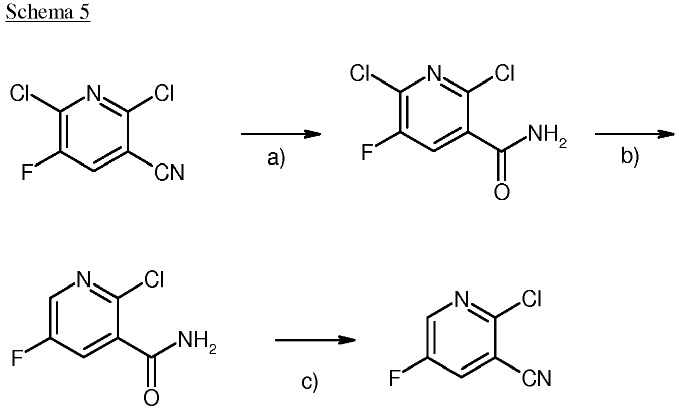 [a): Schwefelsäure; b): Zink, Methanol, Eisessig; c): Trifluoressigsäureanhydrid, Dichlormethan].

Die Verbindungen der Formeln (III) und (VII) sind kommerziell erhältlich, literaturbekannt oder können in Analogie zu literaturbekannten Verfahren hergestellt werden.

Die erfindungsgemäßen Verbindungen wirken als potente Stimulatoren der löslichen Guanylatcyclase und weisen ein gleiches oder verbessertes therapeutisches Profil gegenüber den aus dem Stand der Technik bekannten Verbindungen auf, wie beispielsweise hinsichtlich ihrer *in-vivo* Eigenschaften, wie beispielsweise ihrem pharmakokinetischem und pharmakodynamischem Verhalten und/oder ihrer Dosis-Wirkungsbeziehung und/oder ihres Sicherheitsprofils. Sie eignen sich daher zur Behandlung und/ oder Prophylaxe von Erkrankungen bei Menschen und Tieren.

Die erfindungsgemäßen Verbindungen bewirken eine Gefäßrelaxation und eine Hemmung der Thrombozytenaggregation und führen zu einer Blutdrucksenkung sowie zu einer Steigerung des koronaren Blutflusses. Diese Wirkungen sind über eine direkte Stimulation der löslichen Guanylatcyclase und einen intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (endothelium-derived relaxing factor), NO-Donatoren, Protoporphyrin IX, Arachidonsäure oder Phenylhydrazin-Derivate.

Die erfindungsgemäßen Verbindungen eignen sich zur Behandlung und/oder Prophylaxe von kardiovaskulären, pulmonalen, thromboembolischen und fibrotischen Erkrankungen.

Die erfindungsgemäßen Verbindungen können daher in Arzneimitteln zur Behandlung und/oder Prophylaxe von kardiovaskulären Erkrankungen wie beispielsweise Bluthochdruck, akute und chronische Herzinsuffizienz, koronare Herzerkrankung, stabile und instabile Angina pectoris, periphere und kardiale Gefäßerkrankungen, Arrhythmien, Rhythmusstörungen der Vorhöfe und der Kammern sowie Überleitungsstörungen wie beispielsweise atrio-ventrikuläre Blockaden Grad I-III (AB-Block I-III), supraventrikuläre Tachyarrhythmie, Vorhofflimmern, Vorhoffflattern, Kammerflimmern, Kammerflattern, ventrikuläre Tachyarrhytmie, Torsade de pointes-Tachykardie, Extrasystolen des Vorhoffs und des Ventrikels, AV-junktionale Extrasystolen, Sick-Sinus Syndrom, Synkopen, AV-Knoten-Reentrytachykardie, Wolff-Parkinson-White-Syndrom, von akutem Koronarsyndrom (ACS), autoimmune Herzerkrankungen (Perikarditis, Endokarditis, Valvolitis, Aortitis, Kardiomyopathien), Schock wie kardiogenem Schock, septischem Schock und anaphylaktischem Schock, Aneurysmen, Boxerkardiomyopathie (premature ventricular contraction (PVC)), zur Behandlung und/oder Prophylaxe von thromboembolischen Erkrankungen und Ischämien wie myokardiale Ischämie, Myokardinfarkt, Hirnschlag, Herzhypertrophie, transistorischen und ischämischen Attacken, Präeklampsie, entzündliche kardiovaskuläre Erkrankungen, Spasmen der Koronararterien und peripherer Arterien, Ödembildung wie beispielsweise pulmonales Ödem, Hirnödem, renales Ödem oder Herzinsuffizienz-bedingtes Ödem, peripheren Durchblutungsstörungen, Reperfusionsschäden, arterielle und venöse Thrombosen, Mikroalbuminurie, Herzmuskelschwäche, endotheliale Dysfunktion, zur Verhinderung von Restenosen wie nach Thrombolysetherapien, percutan-transluminalen Angioplastien (PTA), transluminalen Koronarangioplastien (PTCA), Herztransplantationen und Bypass-Operationen, sowie mikro- und makrovaskuläre Schädigungen (Vasculitis), erhöhte Spiegel von Fibrinogen und von LDL geringer Dichte sowie erhöhte Konzentrationen von Plasminogenaktivator-Inhibitor 1 (PAI-1), sowie zur Behandlung und/oder Prophylaxe von erektiler Dysfunktion und weiblicher sexueller Dysfunktion eingesetzt werden.

Im Sinne der vorliegenden Erfindung umfasst der Begriff Herzinsuffizienz sowohl akute als auch chronische Erscheinungsformen der Herzinsuffizienz, wie auch spezifischere oder verwandte Krankheitsformen wie akut dekompensierte Herzinsuffizienz, Rechtsherzinsuffizienz, Linksherzinsuffizienz, Globalinsuffizienz, ischämische Kardiomyopathie, dilatative Kardiomyopathie, hypertrophe Kardiomyopathie, idiopathische Kardiomyopathie, angeborene Herzfehler, Herzinsuffizienz bei Herzklappenfehlern, Mitralklappenstenose, Mitralklappeninsuffizienz, Aortenklappenstenose, Aortenklappeninsuffizienz, Trikuspidalstenose, Trikuspidalinsuffizienz, Pulmonalklappenstenose, Pulmonalklappeninsuffizienz, kombinierte Herzklappenfehler, Herzmuskelentzündung (Myokarditis), chronische Myokarditis, akute Myokarditis, virale Myokarditis, diabetische Herzinsuffizienz, alkoholtoxische Kardiomyopathie, kardiale Speichererkrankungen, diastolische Herzinsuffizienz sowie systolische Herzinsuffizienz und akute Phasen der Verschlechterung einer bestehenden chronischen Herzinsuffizienz (worsening heart failure).

Darüber hinaus können die erfindungsgemäßen Verbindungen auch zur Behandlung und/oder Prophylaxe von Arteriosklerose, Lipidstoffwechselstörungen, Hypolipoproteinämien, Dyslipidämien, Hypertriglyceridämien, Hyperlipidämien, Hypercholesterolämien, Abetelipoproteinämie, Sitosterolämie, Xanthomatose, Tangier Krankheit, Fettsucht (Adipositas), Fettleibigkeit (Obesitas) und von kombinierten Hyperlipidämien sowie des Metabolischen Syndroms eingesetzt werden.

Außerdem können die erfindungsgemäßen Verbindungen zur Behandlung und/oder Prophylaxe von primärem und sekundärem Raynaud-Phänomen, von Mikrozirkulationsstörungen, Claudicatio, peripheren und autonomen Neuropathien, diabetischen Mikroangiopathien, diabetischer Retinopathie, diabetischen Geschwüren an den Extremitäten, Gangren, CREST-Syndrom, Erythematose, Onychomykose, rheumatischen Erkrankungen sowie zur Förderung der Wundheilung verwendet werden.

Weiterhin eignen sich die erfindungsgemäßen Verbindungen zur Behandlung urologischer Erkrankungen wie beispielsweise benignes Prostata-Syndrom (BPS), benigne Prostata-Hyperplasie (BPH), benigne Prostata Vergrösserung (BPE), Blasenentleerungsstörung (BOO), untere Harnwegssyndrome (LUTS, einschließlich Felines Urologisches Syndrom (FUS)), Erkrankungen des Urogenital-Systems einschliesslich neurogene überaktive Blase (OAB) und (IC), Inkontinenz (UI) wie beispielsweise Misch-, Drang-, Stress-, oder Überlauf-Inkontinenz (MUI, UUI, SUI, OUI), Beckenschmerzen, benigne und maligne Erkrankungen der Organe des männlichen und weiblichen Urogenital-Systems.

Weiterhin eignen sich die erfindungsgemäßen Verbindungen zur Behandlung und/oder Prophylaxe von Nierenerkrankungen, insbesondere von aktuer und chronischer Niereninsuffizienz, sowie von akutem und chronischem Nierenversagen. Im Sinne der vorliegenden Erfindung umfasst der Begriff Niereninsuffizienz sowohl akute als auch chronische Erscheinungsformen der Niereninsuffizienz, wie auch zugrundeliegende oder verwandte Nierenerkrankungen wie renale Hypoperfusion, intradialytische Hypotonie, obstruktive Uropathie, Glomerulopathien, Glomerulonephritis, akute Glomerulonephritis, Glomerulosklerose, tubulointerstitielle Erkrankungen, nephropathische Erkrankungen wie primäre und angeborene Nierenerkrankung, Nierenentzündung, immunologische Nierenerkrankungen wie Nierentransplantatabstoßung, Immunkomplex-induzierte Nierenerkrankungen, durch toxische Substanzen induzierte Nephropathie, Kontrastmittelinduzierte Nephropathie, diabetische und nicht-diabetische Nephropathie, Pyelonephritis, Nierenzysten, Nephrosklerose, hypertensive Nephrosklerose und nephrotisches Syndrom, welche diagnostisch beispielsweise durch abnorm verminderte Kreatinin- und/oder Wasser-Ausscheidung, abnorm erhöhte Blutkonzentrationen von Harnstoff, Stickstoff, Kalium und/oder Kreatinin, veränderte Aktivität von Nierenenzymen wie z.B. Glutamylsynthetase, veränderte Urinosmolarität oder Urinmenge, erhöhte Mikroalbuminurie, Makroalbuminurie, Läsionen an Glomerula und Arteriolen, tubuläre Dilatation, Hyperphosphatämie und/oder die Notwendigkeit zur Dialyse charakterisiert werden können. Die vorliegende Erfindung umfasst auch die Verwendung der erfindungsgemäßen Verbindungen zur Behandlung und/oder Prophylaxe von Folgeerscheinungen einer Niereninsuffizienz, wie beispielsweise Lungenödem, Herzinsuffizienz, Urämie, Anämie, Elektrolytstörungen (z.B. Hyperkalämie, Hyponaträmie) und Störungen im Knochen- und Kohlenhydrat-Metabolismus.

Weiterhin eignen sich die erfindungsgemäßen Verbindungen auch zur Behandlung und/oder Prophylaxe von asthmatischen Erkrankungen, pulmonaler arterieller Hypertonie (PAH) und anderen Formen der pulmonalen Hypertonie (PH), umfassend mit Linksherzerkrankung, HIV, Sichelzellanämie, Thromboembolien (CTEPH), Sarkoidose, COPD oder Lungenfibrose assoziierte pulmonale Hypertonie, der chronisch-obstruktive Lungenerkrankung (COPD), des akuten Atemwegssyndrom (ARDS), der akuten Lungenschädigung (ALI), der alpha-1-Antitrypsin-Defizienz (AATD), der Lungenfibrose, des Lungenemphysem (z.B. durch Zigarettenrauch induziertes Lungenemphysem) und der zystischen Fibrose (CF).

Die in der vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Verbesserung der Wahrnehmung, Konzentrationsleistung, Lernleistung oder Gedächtnisleistung nach kognitiven Störungen, wie sie insbesondere bei Situationen/Krankheiten/Syndromen auftreten wie "Mild cognitive impairment", altersassoziierten Lern- und Gedächtnisstörungen, altersassoziierten Gedächtnisverlusten, vaskulärer Demenz, Schädel-Hirn-Trauma, Schlaganfall, Demenz, die nach Schlaganfällen auftritt ("post stroke dementia"), post-traumatischem Schädel-Hirn-Trauma, allgemeinen Konzentrationsstörungen, Konzentrationsstörungen bei Kindern mit Lern- und Gedächtnisproblemen, Alzheimer'scher Krankheit, Demenz mit Lewy-Körperchen, Demenz mit Degeneration der Frontallappen einschliesslich des Pick's-Syndroms, Parkinson'scher Krankheit, progressiver nuclear palsy, Demenz mit corticobasaler Degeneration, Amyolateralsklerose (ALS), Huntington'scher Krankheit, Demyelinisation, Multipler Sklerose, Thalamischer Degeneration, Creutzfeld-Jacob-Demenz, HIV-Demenz, Schizophrenie mit Demenz oder Korsakoff-Psychose. Sie eignen sich auch zur Behandlung und/oder Prophylaxe von Erkrankungen des Zentralnervensystems wie Angst-, Spannungs- und Depressionszuständen, zentral-nervös bedingten Sexualdysfunktionen und Schlafstörungen sowie zur Regulierung krankhafter Störungen der Nahrungs-, Genuss- und Suchtmittelaufnahme.

Weiterhin eignen sich die erfindungsgemäßen Verbindungen auch zur Regulation der cerebralen Durchblutung und stellen wirkungsvolle Mittel zur Bekämpfung von Migräne dar. Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen und Tinnitus eingesetzt werden.

Zudem besitzen die erfindungsgemäßen Verbindungen antiinflammatorische Wirkung und können daher als entzündungshemmende Mittel zur Behandlung und/oder Prophylaxe von Sepsis (SIRS), multiplem Organversagen (MODS, MOF), entzündlichen Erkrankungen der Niere, chronischen Darmentzündungen (IBD, Crohn's Disease, UC), Pankreatitis, Peritonitis, rheumatoiden Erkrankungen, entzündlichen Hauterkrankungen sowie entzündlichen Augenerkrankungen eingesetzt werden.

Des weiteren können die erfindungsgemäßen Verbindungen ebenfalls zur Behandlung und/ oder Prophylaxe von Autoimmunerkrankungen eingesetzt werden.

Weiterhin sind die erfindungsgemäßen Verbindungen zur Behandlung und/oder Prophylaxe fibrotischer Erkrankungen der inneren Organe, wie beispielsweise der Lunge, des Herzens, der Niere, des Knochenmarks und insbesondere der Leber, sowie dermatologischer Fibrosen und fibrotischer Erkrankungen des Auges, geeignet. Im Sinne der vorliegenden Erfindungen umfasst der Begriff fibrotischer Erkrankungen insbesondere die folgenden Begriffe Leberfibrose, Leberzirrhose, Lungenfibrose, Endomyocardfibrose, Nephropathie, Glomerulonephritis, interstitielle Nierenfibrose, fibrotische Schäden in Folge von Diabetes, Knochenmarksfibrose und ähnliche fibrotische Erkrankungen, Sklerodermie, Morphaea, Keloide, hypertrophe Narbenbildung (auch nach chirurgischen Eingriffen), Naevi, diabetische Retinopathie, proliferative Vitroretinopathie und Erkrankungen des Bindegewebes (z.B. Sarkoidose).

Weiterhin eignen sich die erfindungsgemäßen Verbindungen zur Bekämpfung postoperativer Narbenbildung, z.B. in Folge von Glaukom-Operationen.

Die erfindungsgemäßen Verbindungen können ebenfalls kosmetisch bei alternder und verhornender Haut eingesetzt werden.

Außerdem sind die erfindungsgemäßen Verbindungen zur Behandlung und/ oder Prophylaxe von Hepatitis, Neoplasma, Osteoporose, Glaukom und Gastroparese geeignet.

Weiter offenbart ist die Verwendung der erfindungsgemäßen Verbindungen zur Behandlung und/oder Prophylaxe von Erkrankungen, insbesondere der zuvor genannten Erkrankungen.

Weiter offenbart ist die Verwendung der erfindungsgemäßen Verbindungen zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung sind die erfindungsgemäßen Verbindungen zur Verwendung in einem Verfahren zur Behandlung und/ oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

Die erfindungsgemäßen Verbindungen können allein oder bei Bedarf in Kombination mit anderen Wirkstoffen eingesetzt werden. Weiterer Gegenstand der vorliegenden Erfindung sind Arzneimittel, enthaltend mindestens eine der erfindungsgemäßen Verbindungen und einen oder mehrere weitere Wirkstoffe, insbesondere zur Behandlung und/oder Prophylaxe der zuvor genannten Erkrankungen. Als geeignete Kombinationswirkstoffe seien beispielhaft und vorzugsweise genannt:
- organische Nitrate und NO-Donatoren, wie beispielsweise Natriumnitroprussid, Nitroglycerin, Isosorbidmononitrat, Isosorbiddinitrat, Molsidomin oder SIN-1, sowie inhalatives NO;
- Verbindungen, die den Abbau von cyclischem Guanosinmonophosphat (cGMP) inhibieren, wie beispielsweise Inhibitoren der Phosphodiesterasen (PDE) 1, 2 und/oder 5, insbesondere PDE 5-Inhibitoren wie Sildenafil, Vardenafil und Tadalafil;
- antithrombotisch wirkende Mittel, beispielhaft und vorzugsweise aus der Gruppe der Thrombozytenaggregationshemmer, der Antikoagulantien oder der profibrinolytischen Substanzen;
- den Blutdruck senkende Wirkstoffe, beispielhaft und vorzugsweise aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika; und/oder
- den Fettstoffwechsel verändernde Wirkstoffe, beispielhaft und vorzugsweise aus der Gruppe der Thyroidrezeptor-Agonisten, Cholesterinsynthese-Inhibitoren wie beispielhaft und vorzugsweise HMG-CoA-Reduktase- oder Squalensynthese-Inhibitoren, der ACAT-Inhibitoren, CETP-Inhibitoren, MTP-Inhibitoren, PPAR-alpha-, PPAR-gamma- und/oder PPAR-delta-Agonisten, Cholesterin-Absorptionshemmer, Lipase-Inhibitoren, polymeren Gallensäureadsorber, Gallensäure-Reabsorptionshemmer und Lipoprotein(a)-Antagonisten.

Unter antithrombotisch wirkenden Mittel werden vorzugsweise Verbindungen aus der Gruppe der Thrombozytenaggregationshemmer, der Antikoagulantien oder der profibrinolytischen Substanzen verstanden.

Bei einer bevorzugten Ausführungsform der Erfindung werden die erfindungsgemäßen Verbindungen in Kombination mit einem Thrombozytenaggregationshemmer, wie beispielhaft und vorzugsweise Aspirin, Clopidogrel, Ticlopidin oder Dipyridamol, verabreicht.

Bei einer bevorzugten Ausführungsform der Erfindung werden die erfindungsgemäßen Verbindungen in Kombination mit einem Thrombin-Inhibitor, wie beispielhaft und vorzugsweise Ximelagatran, Dabigatran, Melagatran, Bivalirudin oder Clexane, verabreicht.

Bei einer bevorzugten Ausführungsform der Erfindung werden die erfindungsgemäßen Verbindungen in Kombination mit einem GPIIb/IIIa-Antagonisten, wie beispielhaft und vorzugsweise Tirofiban oder Abciximab, verabreicht.

Bei einer bevorzugten Ausführungsform der Erfindung werden die erfindungsgemäßen Verbindungen in Kombination mit einem Faktor Xa-Inhibitor, wie beispielhaft und vorzugsweise Rivaroxaban (BAY 59-7939), DU-176b, Apixaban, Otamixaban, Fidexaban, Razaxaban, Fondaparinux, Idraparinux, PMD-3112, YM-150, KFA-1982, EMD-503982, MCM-17, MLN-1021, DX 9065a, DPC 906, JTV 803, SSR-126512 oder SSR-128428, verabreicht.

Bei einer bevorzugten Ausführungsform der Erfindung werden die erfindungsgemäßen Verbindungen in Kombination mit Heparin oder einem low molecular weight (LMW)-Heparin-Derivat verabreicht.

Bei einer bevorzugten Ausführungsform der Erfindung werden die erfindungsgemäßen Verbindungen in Kombination mit einem Vitamin K-Antagonisten, wie beispielhaft und vorzugsweise Coumarin, verabreicht.

Unter den Blutdruck senkenden Mitteln werden vorzugsweise Verbindungen aus der Gruppe der Calcium-Antagonisten, Angiotensin AII-Antagonisten, ACE-Hemmer, Endothelin-Antagonisten, Renin-Inhibitoren, alpha-Rezeptoren-Blocker, beta-Rezeptoren-Blocker, Mineralocorticoid-Rezeptor-Antagonisten sowie der Diuretika verstanden.

Bei einer bevorzugten Ausführungsform der Erfindung werden die erfindungsgemäßen Verbindungen in Kombination mit einem Calcium-Antagonisten, wie beispielhaft und vorzugsweise Nifedipin, Amlodipin, Verapamil oder Diltiazem, verabreicht.

Bei einer bevorzugten Ausführungsform der Erfindung werden die erfindungsgemäßen Verbindungen in Kombination mit einem alpha-1-Rezeptoren-Blocker, wie beispielhaft und vorzugsweise Prazosin, verabreicht.

Bei einer bevorzugten Ausführungsform der Erfindung werden die erfindungsgemäßen Verbindungen in Kombination mit einem beta-Rezeptoren-Blocker, wie beispielhaft und vorzugsweise Propranolol, Atenolol, Timolol, Pindolol, Alprenolol, Oxprenolol, Penbutolol, Bupranolol, Metipranolol, Nadolol, Mepindolol, Carazalol, Sotalol, Metoprolol, Betaxolol, Celiprolol, Bisoprolol, Carteolol, Esmolol, Labetalol, Carvedilol, Adaprolol, Landiolol, Nebivolol, Epanolol oder Bucindolol, verabreicht.

Bei einer bevorzugten Ausführungsform der Erfindung werden die erfindungsgemäßen Verbindungen in Kombination mit einem Angiotensin AII-Antagonisten, wie beispielhaft und vorzugsweise Losartan, Candesartan, Valsartan, Telmisartan oder Embursatan, verabreicht.

Bei einer bevorzugten Ausführungsform der Erfindung werden die erfindungsgemäßen Verbindungen in Kombination mit einem ACE-Hemmer, wie beispielhaft und vorzugsweise Enalapril, Captopril, Lisinopril, Ramipril, Delapril, Fosinopril, Quinopril, Perindopril oder Trandopril, verabreicht.

Bei einer bevorzugten Ausführungsform der Erfindung werden die erfindungsgemäßen Verbindungen in Kombination mit einem Endothelin-Antagonisten, wie beispielhaft und vorzugsweise Bosentan, Darusentan, Ambrisentan oder Sitaxsentan, verabreicht.

Bei einer bevorzugten Ausführungsform der Erfindung werden die erfindungsgemäßen Verbindungen in Kombination mit einem Renin-Inhibitor, wie beispielhaft und vorzugsweise Aliskiren, SPP-600 oder SPP-800, verabreicht.

Bei einer bevorzugten Ausführungsform der Erfindung werden die erfindungsgemäßen Verbindungen in Kombination mit einem Mineralocorticoid-Rezeptor-Antagonisten, wie beispielhaft und vorzugsweise Spironolacton oder Eplerenon, verabreicht.

Bei einer bevorzugten Ausführungsform der Erfindung werden die erfindungsgemäßen Verbindungen in Kombination mit einem Schleifendiuretikum, wie beispielsweise Furosemid, Torasemid, Bumetanid und Piretanid, mit kaliumsparenden Diuretika wie beispielsweise Amilorid und Triamteren, mit Aldosteronantagonisten, wie beispielsweise Spironolacton, Kaliumcanrenoat und Eplerenon sowie Thiaziddiuretika, wie beispielsweise Hydrochlorothiazid, Chlorthalidon, Xipamid, und Indapamid, verabreicht.

Unter den Fettstoffwechsel verändernden Mitteln werden vorzugsweise Verbindungen aus der Gruppe der CETP-Inhibitoren, Thyroidrezeptor-Agonisten, Cholesterinsynthese-Inhibitoren wie HMG-CoA-Reduktase- oder Squalensynthese-Inhibitoren, der ACAT-hihibitoren, MTP-Inhibitoren, PPAR-alpha-, PPAR-gamma- und/oder PPAR-delta-Agonisten, Cholesterin-Absorptionshemmer, polymeren Gallensäureadsorber, Gallensäure-Reabsorptionshemmer, Lipase-Inhibitoren sowie der Lipoprotein(a)-Antagonisten verstanden.

Bei einer bevorzugten Ausführungsform der Erfindung werden die erfindungsgemäßen Verbindungen in Kombination mit einem CETP-Inhibitor, wie beispielhaft und vorzugsweise Dalcetrapib, BAY 60-5521, Anacetrapib oder CETP-vaccine (CETi-1), verabreicht.

Bei einer bevorzugten Ausführungsform der Erfindung werden die erfindungsgemäßen Verbindungen in Kombination mit einem Thyroidrezeptor-Agonisten, wie beispielhaft und vorzugsweise D-Thyroxin, 3,5,3'-Triiodothyronin (T3), CGS 23425 oder Axitirome (CGS 26214), verabreicht.

Bei einer bevorzugten Ausführungsform der Erfindung werden die erfindungsgemäßen Verbindungen in Kombination mit einem HMG-CoA-Reduktase-Inhibitor aus der Klasse der Statine, wie beispielhaft und vorzugsweise Lovastatin, Simvastatin, Pravastatin, Fluvastatin, Atorvastatin, Rosuvastatin oder Pitavastatin, verabreicht.

Bei einer bevorzugten Ausführungsform der Erfindung werden die erfindungsgemäßen Verbindungen in Kombination mit einem Squalensynthese-Inhibitor, wie beispielhaft und vorzugsweise BMS-188494 oder TAK-475, verabreicht.

Bei einer bevorzugten Ausführungsform der Erfindung werden die erfindungsgemäßen Verbindungen in Kombination mit einem ACAT-hihibitor, wie beispielhaft und vorzugsweise Avasimibe, Melinamide, Pactimibe, Eflucimibe oder SMP-797, verabreicht.

Bei einer bevorzugten Ausführungsform der Erfindung werden die erfindungsgemäßen Verbindungen in Kombination mit einem MTP-Inhibitor, wie beispielhaft und vorzugsweise Implitapide, BMS-201038, R-103757 oder JTT-130, verabreicht.

Bei einer bevorzugten Ausführungsform der Erfindung werden die erfindungsgemäßen Verbindungen in Kombination mit einem PPAR-gamma-Agonisten, wie beispielhaft und vorzugsweise Pioglitazone oder Rosiglitazone, verabreicht.

Bei einer bevorzugten Ausführungsform der Erfindung werden die erfindungsgemäßen Verbindungen in Kombination mit einem PPAR-delta-Agonisten, wie beispielhaft und vorzugsweise GW 501516 oder BAY 68-5042, verabreicht.

Bei einer bevorzugten Ausführungsform der Erfindung werden die erfindungsgemäßen Verbindungen in Kombination mit einem Cholesterin-Absorptionshemmer, wie beispielhaft und vorzugsweise Ezetimibe, Tiqueside oder Pamaqueside, verabreicht.

Bei einer bevorzugten Ausführungsform der Erfindung werden die erfindungsgemäßen Verbindungen in Kombination mit einem Lipase-Inhibitor, wie beispielhaft und vorzugsweise Orlistat, verabreicht.

Bei einer bevorzugten Ausführungsform der Erfindung werden die erfindungsgemäßen Verbindungen in Kombination mit einem polymeren Gallensäureadsorber, wie beispielhaft und vorzugsweise Cholestyramin, Colestipol, Colesolvam, CholestaGel oder Colestimid, verabreicht.

Bei einer bevorzugten Ausführungsform der Erfindung werden die erfindungsgemäßen Verbindungen in Kombination mit einem Gallensäure-Reabsorptionshemmer, wie beispielhaft und vorzugsweise ASBT (= IBAT)-Inhibitoren wie z.B. AZD-7806, S-8921, AK-105, BARI-1741, SC-435 oder SC-635, verabreicht.

Bei einer bevorzugten Ausführungsform der Erfindung werden die erfindungsgemäßen Verbindungen in Kombination mit einem Lipoprotein(a)-Antagonisten, wie beispielhaft und vorzugsweise Gemcabene calcium (CI-1027) oder Nicotinsäure, verabreicht.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende, die erfindungsgemäßen Verbindungen schnell und/oder modifiziert abgebende Applikationsformen, die die erfindungsgemäßen Verbindungen in kristalliner und/oder amorphisierter und/oder gelöster Form enthalten, wie z.B. Tabletten (nicht-überzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen oder -sprays, lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme (z.B. Pflaster), Milch, Pasten, Schäume, Streupuder, Implantate oder Stents.

Bevorzugt sind die orale oder parenterale Applikation, insbesondere die orale Applikation.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Lactose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmacks- und/oder Geruchskorrigentien.

Im Allgemeinen hat es sich als vorteilhaft erwiesen, bei parenteraler Applikation Mengen von etwa 0.001 bis 1 mg/kg, vorzugsweise etwa 0.01 bis 0.5 mg/kg Körpergewicht zur Erzielung wirksamer Ergebnisse zu verabreichen. Bei oraler Applikation beträgt die Dosierung etwa 0.001 bis 2 mg/kg, vorzugsweise etwa 0.001 bis 1 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die nachfolgenden Ausführungsbeispiele erläutern die Erfindung. Die Erfindung ist nicht auf die Beispiele beschränkt.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen und Akronyme:

- aq.: wässrige Lösung
- ber.: berechnet
- DCI: direkte chemische Ionisation (bei MS)
- DMF: Dimethylformamid
- DMSO: Dimethylsulfoxid
- d. Th.: der Theorie (bei Ausbeute)
- eq.: Äquivalent(e)
- ESI: Elektrospray-Ionisation (bei MS)
- Et: Ethyl
- gef.: Gefunden
- h: Stunde(n)
- HPLC: Hochdruck-, Hochleistungsflüssigchromatographie
- HRMS: hochaufgelöste Massenspektrometrie
- konz.: konzentriert
- LC/MS: Flüssigchromatographie-gekoppelte Massenspektrometrie
- LiHMDS: Lithiumhexamethyldisilazid
- Me: Methyl
- min: Minute(n)
- MS: Massenspektrometrie
- NMR: Kernresonanzspektrometrie
- Pd₂dba₃: Tris-(dibenzylidenaceton)-dipalladium
- Ph: Phenyl
- RT: Raumtemperatur
- Rₜ: Retentionszeit (bei HPLC)
- THF: Tetrahydrofuran
- UV: Ultraviolett-Spektrometrie
- v/v: Volumen zu Volumen-Verhältnis (einer Lösung)

### LC/MS-Methoden:

### Methode 1 (LC-MS):

Instrument: Waters ACQUITY SQD UPLC System; Säule: Waters Acquity UPLC HSS T3 1.8 µ 30 x 2 mm; Eluent A: 11 Wasser + 0.25 ml 99%ige Ameisensäure , Eluent B: 11 Acetonitril + 0.25 ml 99%ige Ameisensäure; Gradient: 0.0 min 90% A → 1.2 min 5% A → 2.0 min 5% A Ofen: 50°C; Fluss: 0.60 ml/min; UV-Detektion: 208 - 400 nm.

### Methode 2 (LC-MS):

Instrument: Waters ACQUITY SQD UPLC System; Säule: Waters Acquity UPLC HSS T3 1.8 µ 50 x 1 mm; Eluent A: 11 Wasser + 0.25 ml 99%ige Ameisensäure , Eluent B: 11 Acetonitril + 0.25 ml 99%ige Ameisensäure; Gradient: 0.0 min 90% A → 1.2 min 5% A → 2.0 min 5% A Ofen: 50 °C; Fluss: 0.40 ml/min; UV-Detektion: 210 - 400 nm.

### Methode 3 (LC-MS):

Instrument: Micromass Quattro Premier mit Waters UPLC Acquity; Säule: Thermo Hypersil GOLD 1.9 µ 50 x 1 mm; Eluent A: 11 Wasser + 0.5 ml 50%ige Ameisensäure, Eluent B: 11 Acetonitril + 0.5 ml 50%ige Ameisensäure; Gradient: 0.0 min 97% A → 0.5 min 97% A → 3.2 min 5% A → 4.0 min 5% A Ofen: 50°C; Fluss: 0.3 ml/min; UV-Detektion: 210 nm.

### Ausgangsverbindungen und Intermediate:

### Beispiel 1A

### 5-Fluor-3-iod-1H-pyrazolo[3,4-b]pyridin

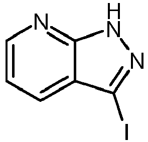

Die Synthese ist beschrieben in in WO 2006/130673, Beispiel 4.

### Beispiel 2A

### 2,6-Dichlor-5-fluornicotinamid

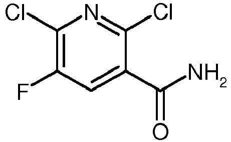

Eine Suspension aus 25 g (130.90 mmol) 2,6-Dichlor-5-fluor-3-cyanopyridin in konz. Schwefelsäure (125 ml) wurde 1 h bei 60-65°C gerührt. Nach Abkühlen auf RT wurde der Kolbeninhalt auf Eiswasser gegossen und dreimal mit Essigsäureethylester (je 100 ml) extrahiert. Die vereinigten organischen Phasen wurden mit Wasser (100 ml) und anschliessend mit gesättigter wässriger Natriumhydrogencarbonat-Lösung (100 ml) gewaschen, getrocknet und am Rotationsverdampfer eingeengt. Das erhaltene Material wurde am Hochvakuum getrocknet.

Ausbeute: 24.5 g (90% d. Theorie)

¹H-NMR (400 MHz, DMSO-d₆): δ = 7.95 (br s, 1H), 8.11 (br s, 1H), 8.24 (d, 1H).

### Beispiel 3A

### 2-Chlor-5-fluornicotinamid

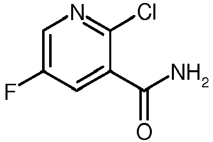

Zu einer Suspension von 21.9 g (335.35 mmol) Zink in Methanol (207 ml) wurden bei RT 44 g (210.58 mmol) 2,6-Dichlor-5-fluornicotinamid gegeben. Danach wurde mit Essigsäure (18.5 ml) versetzt und unter Rühren für 24 h zum Rückfluss erhitzt. Danach wurde der Kolbeninhalt vom Zink dekantiert und Essigsäureethylester (414 ml) sowie gesättigte wässrige Natriumhydrogencarbonat-Lösung (414 ml) zugegeben und intensiv ausgerührt. Anschliessend wurde über Kieselgur abgesaugt und dreimal mit Essigsäureethylester (je 517 ml) nachgewaschen. Die organische Phase wurde abgetrennt und die wässrige Phase mit Essigsäureethylester (258 ml) gewaschen. Die vereinigten organischen Phasen wurden einmal mit gesättigter wässriger Natriumhydrogencarbonat-Lösung (414 ml) gewaschen, getrocknet und im Vakuum eingeengt. Die so erhaltene Feststoff wurde mit Dichlormethan (388 ml) versetzt und 20 min ausgerührt. Es wurde erneut abgesaugt und mit Diethylether nachgewaschen und trockengesaugt.

Ausbeute: 20.2 g (53% d. Theorie)

¹H-NMR (400 MHz, DMSO-d₆): δ = 7.87 (br s, 1H), 7.99 (dd, 1H), 8.10 (br s, 1H), 8.52 (d, 1H).

### Beispiel 4A

### 2-Chlor-5-fluornicotinonitril

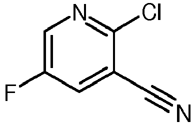

Eine Suspension von 46.2 g (264.66 mmol) 2-Chlor-5-fluornicotinamid in Dichlormethan (783 ml) wurde mit 81.2 ml (582.25 mmol) Triethylamin versetzt und auf 0°C gekühlt. Unter Rühren wurden dann 41.12 ml (291.13 mmol) Trifluoressigsäureanhydrid langsam zugetropft und 1.5 h bei 0°C nachgerührt. Die Reaktionslösung wurde danach zweimal mit gesättigter wässriger Natriumhydrogencarbonat-Lösung (je 391 ml) gewaschen, getrocknet und im Vakuum eingeengt.

Ausbeute: 42.1 g (90% d. Theorie).

¹H-NMR (400 MHz, DMSO-d₆): δ= 8.66 (dd, 1H), 8.82 (d, 1H).

### Beispiel 5A

### 5-Fluor-1H-pyrazolo[3,4-b]pyridin-3-amin

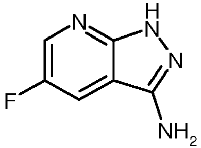

Eine Suspension aus 38.5 g (245.93 mmol) 2-Chlor-5-fluornicotinonitril wurde in 1,2-Ethandiol (380 ml) vorgelegt und danach mit Hydrazinhydrat (119.6 ml) versetzt. Es wurde unter Rühren für 4 h zum Rückfluss erhitzt. Beim Abkühlen fiel ein Feststoff aus, der mit Wasser (380 ml) versetzt und 10 min bei RT ausgerührt wurde. Anschliessend wurde die Suspension über eine Fritte abgesaugt, mit Wasser (200 ml) und mit -10°C-kaltem THF (200 ml) nachgewaschen. Der Feststoff wurde im Hochvakuum über Phosphorpentoxid getrocknet.

Ausbeute: 22.8 g (61 % d. Theorie)

¹H-NMR (400 MHz, DMSO-d₆): δ = 5.54 (s, 2H), 7.96 (dd, 1H), 8.38 (m, 1H), 12.07(m, 1H).

### Beispiel 6A

### 5-Fluor-3-iod-1H-pyrazolo[3,4-b]pyridin

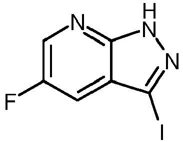

In THF (329 ml) wurden 10 g (65.75 mmol) 5-Fluor-1H-pyrazolo[3,4-b]pyridin-3-amin vorgelegt und auf 0°C abgekühlt. Anschliessend wurden 16.65 ml (131.46 mmol) Bortrifluorid-Diethylether-Komplex langsam zugesetzt. Die Reaktionsmischung wurde weiter auf -10°C abgekühlt. Danach wurde eine Lösung von 10.01 g (85.45 mmol) Isopentylnitrit in THF (24.39 ml) langsam zugefügt und weitere 30 min nachgerührt. Die Mischung wurde mit kaltem Diethylether (329 ml) verdünnt und der entstandene Feststoff abfiltriert. Das so hergestellte Diazoniumsalz wurde portionsweise in eine 0°C kalte Lösung von 12.81 g (85.45 mmol) Natriumiodid in Aceton (329 ml) gegeben und die Mischung 30 min bei RT nachgerührt. Die Reaktionsmischung wurde auf Eiswasser (1.8 1) gegeben und zweimal mit Essigsäureethylester (je 487 ml) extrahiert. Die gesammelten organischen Phasen wurden mit gesättigter wässriger Natriumchlorid-Lösung (244 ml) gewaschen, getrocknet, filtriert und eingeengt. Man erhielt 12.1 g (86%-ige Reinheit, 60 % d. Th.) der Titelverbindung als Feststoff.

LC-MS (Methode 2): Rₜ = 1.68 min

MS (ESIpos): m/z = 264 (M+H)⁺

### Beispiel 7A

### 5-Fluor-3-iod-1-(3,3,4,4,4-pentafluorbutyl)-1H-pyrazolo[3,4-b]pyridin

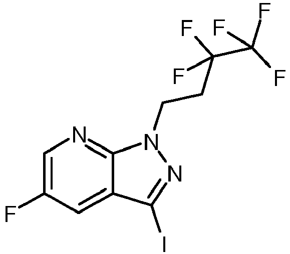

In DMF (100 ml) wurden 5.0 g (19.010 mmol) 5-Fluor-3-iod-1H-pyrazolo[3,4-b]pyridin vorgelegt und anschließend 20.83 g (76.042 mmol) 1,1,1,2,2-Pentafluor-4-iodbutan sowie 14.86 g (45.65 mmol) Cäsiumcarbonat und 0.63 g (3.802 mmol) Kaliumiodid zugefügt. Die Mischung wurde über Nacht bei 140°C gerührt. Anschließend wurde abgekühlt und mit einem Vorversuch vereinigt, der analog ausgehend von 200 mg 5-Fluor-3-iod-1H-pyrazolo[3,4-b]pyridin durchgeführt wurde. Es wurde von Feststoffen abgesaugt, mit DMF nachgewaschen, und das Filtrat anschliessend im Hochvakuum eingeengt. Der Rückstand wurde mittels präparativer HPLC gereinigt (Methanol:Wasser - Gradient). Man erhielt 4.34 g (52 % d. Th.) der Titelverbindung.

LC-MS (Methode 1): Rₜ = 1.30 min

MS (ESIpos): m/z = 410 (M+H)⁺

¹H-NMR (400 MHz, DMSO-d₆): δ = 2.84-3.00 (m, 2H), 4.79 (t, 2H), 7.93 (dd, 1H), 8.71 (dd, 1H).

### Beispiel 8A

### 5-Fluor-1-(3,3,4,4,4-pentafluorbutyl)-1H-pyrazolo[3,4-b]pyridin-3-carbonitril

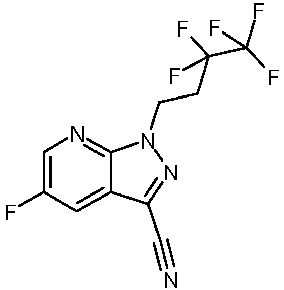

Eine Suspension aus 4.34 g (10.609 mmol) Beispiel 7A und 1.045 g (11.670 mmol) Kupfer-(I)-cyanid wurden in DMSO (30 ml) vorgelegt und 2 h bei 150°C gerührt. Nach Abkühlen wurde der Ansatz über Celite filtriert, mit Essigsäureethylester und THF nachgewaschen und anschliessend viermal mit einer Lösung aus gesättigter wässriger Ammoniumchloridlösung und konz. Ammoniakwasser (3:1 v/v) extrahiert. Die vereinigten organischen Phasen wurden mit ges. wässriger Natriumchlorid-Lösung gewaschen, über Natriumsulfat getrocknet, filtriert und im Vakuum eingeengt.

Ausbeute: 3.19 g (97 % d. Theorie)

¹H-NMR (400 MHz, DMSO-d₆): δ = 2.94-3.09 (m, 2H), 4.93 (t, 2H), 8.54 (dd, 1H), 8.88 (dd, 1H).

### Beispiel 9A

### 5-Fluor-1-(3,3,4,4,4-pentafluorbutyl)-1H-pyrazolo[3,4-b]pyridin-3-carboximidamid-Acetat

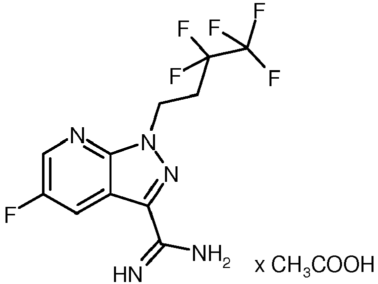

Zu 0.559 g (10.351 mmol) Natriummethanolat in Methanol (25 ml) wurden 3.19 g (10.351 mmol) Beispiel 8A gegeben und 2 h bei RT gerührt. Danach wurden 0.664 g (12.421 mmol) Ammoniumchlorid und Essigsäure (2.31 ml) zugegeben und über Nacht zum Rückfluss erhitzt. Danach wurde bis zur Trockene eingeengt und der Rückstand mit Essigsäureethylester und mit 1N Natronlauge versetzt. Die Phasen wurden getrennt. Die wässrige Phase wurde nochmals mit Essigsäureethylester extrahiert. Die vereinigten organischen Phasen wurden vereinigt und eingeengt. Das Rohprodukt wurde ohne weitere Reinigung weiter umgesetzt.

Ausbeute: 2.67 g (37 % d. Theorie, Reinheit ca. 56 %)

LC-MS (Methode 2): Rₜ = 0.68 min

MS (ESIpos): m/z = 326 (M+H)⁺

### Beispiel 10A

### Methyl-3,3-dicyan-2,2-dimethylpropanoat

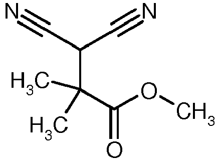

In THF (91 ml) wurden 1.816 g (45.411 mmol) Natriumhydrid (60% in Mineralöl) langsam mit 3 g (45.411 mmol) Malonsäuredinitril versetzt. Anschliessend wurden 5.876 ml (45.411 mmol) Methyl-2-brom-2-methylpropanoat zugegeben, und das Gemisch wurde über Nacht bei RT gerührt. Danach wurden nochmals 5.876 ml (45.411 mmol) Methyl-2-brom-2-methylpropanoat zugegeben und es wurde über Nacht auf 50°C erhitzt. Dann wurden abermals 1.762 ml (13.623) mmol) Methyl-2-brom-2-methylpropanoat zugegeben und es wurde weitere 4h auf 50°C erhitzt. Der Ansatz wurde dann mit gesättigter wässriger Natriumhydrogencarbonat-Lösung versetzt und dreimal mit Essigsäureethylester extrahiert. Die vereinigten organischen Phasen wurden mit gesättigter wässriger Natriumchlorid-Lösung gewaschen, über Natriumsulfat getrocknet, filtriert und bis zur Trockene eingeengt. Man erhielt 8.9 g Rohprodukt, welches per Chromatographie an Kieselgel (Cyclohexan-Essigsäureethylester 4:1) gereinigt wurde.

Ausbeute: 6.47 g (85% d. Th.)

¹H-NMR (400 MHz, DMSO-d₆): δ [ppm] = 1.40 (s, 6H), 3.74 (s, 3H), 5.27 (s, 1H).

### Beispiel 11A

### 3-Iod-1-(3,3,4,4,4-pentafluorbutyl)-1H-pyrazolo[3,4-b]pyridin

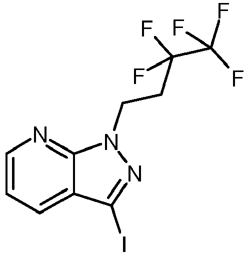

In DMF (170 ml) wurden 10.00 g (40.813 mmol) Beispiel 1A vorgelegt und anschließend 12.30 g (44.894 mmol) 1,1,1,2,2-Pentafluor-4-iodbutan in DMF (30 ml) sowie 14.628 g (44.894 mmol) Cäsiumcarbonat zugefügt. Die Mischung wurde für 2 Tage bei RT gerührt. Anschließend wurden erneut 12.30 g (44.894 mmol) 1,1,1,2,2-Pentafluor-4-iodbutan sowie 14.628 g (44.894 mmol) Cäsiumcarbonat zugegeben und für 2 Tage bei RT gerührt. Danach wurden 3.485 g (12.720 mmol) 1,1,1,2,2-Pentafluor-4-iodbutan und 4.145 g (12.720 mmol) Cäsiumcarbonat zugegeben und über Nacht bei RT gerührt. Nach dieser Zeit wurden wiederum 5.00 g (18.250 mmol) 1,1,1,2,2-Pentafluor-4-iodbutan und 5.946 g (18.250 mmol) Cäsiumcarbonat zugegeben und es wurde 6 Tage bei Raumtemperatur gerührt. Anschließend wurde für 2 Tage bei 70°C gerührt. Es wurde von Feststoffen abgesaugt, mit DMF nachgewaschen und anschliessend im Hochvakuum eingeengt. Der Rückstand wurde mittels präparativer HPLC gereinigt (Methanol:Wasser (mit 0.1% Ameisensäure) - Gradient). Man erhielt 5.48 g (34 % d. Th.) der Titelverbindung.

LC-MS (Methode 1): Rₜ = 1.23 min

MS (ESIpos): m/z = 392 (M+H)⁺

¹H-NMR (400 MHz, DMSO-d₆): δ = 2.87-3.00 (m, 2H), 4.81 (t, 2H), 7.33 (dd, 1H), 7.97 (dd, 1H), 8.65 (dd, 1H).

### Beispiel 12A

### 1-(3,3,4,4,4-Pentafluorbutyl)-1H-pyrazolo[3,4-b]pyridin-3-carbonitril

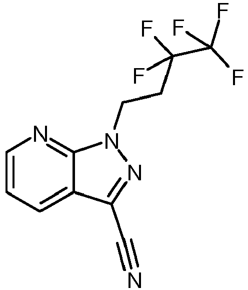

Eine Suspension aus 5.480 g (14.012 mmol) Beispiel 11A und 1.380 g (15.414 mmol) Kupfer-(I)-cyanid wurden in DMSO (50 ml) vorgelegt und 3 h bei 150°C gerührt. Nach Abkühlen wurde über Celite filtriert und mit Essigsäureethylester und THF nachgewaschen. Danach wurde viermal mit einer Lösung aus gesättigter wässriger Ammoniumchloridlösung und konz. Ammoniakwasser (3:1, v/v) und anschliessend mit gesättigter wässriger Natriumchloridlösung gewaschen. Die organische Phase wurde über Natriumsulfat getrocknet, filtriert und eingeengt und anschliessend am Hochvakuum getrocknet.

Ausbeute: 3.59 g (88 % d. Theorie)

LC-MS (Methode 2): Rₜ = 1.04 min

MS (ESIpos): m/z = 291 (M+H)⁺

¹H-NMR (400 MHz, DMSO-d₆): δ = 2.97-3.10 (m, 2H), 4.94 (t, 2H), 7.55 (dd, 1H), 8.51 (dd, 1H), 8.81 (dd, 1H).

### Beispiel 13A

### 1-(3,3,4,4,4-Pentafluorbutyl)-1H-pyrazolo[3,4-b]pyridin-3-carboximidamid-Acetat

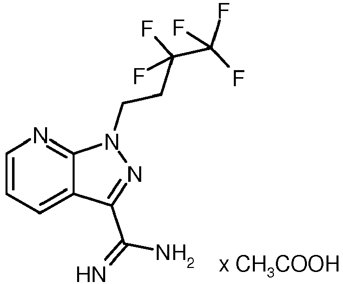

Zu 0.668 g (12.371 mmol) Natriummethanolat in Methanol (40 ml) wurden 3.59 g (12.371 mmol) Beispiel 12A in Methanol (20 ml) gegeben und 2 h bei RT gerührt. Danach wurden 0.794 g (14.845 mmol) Ammoniumchlorid und Essigsäure (2.762 ml) zugegeben und über Nacht zum Rückfluss erhitzt. Danach wurde bis zur Trockene eingeengt und der Rückstand mit Essigsäureethylester und 1N Natronlauge versetzt. Es wurde für ca. 1 h bei RT intensiv gerührt. Der erhaltene Feststoff wurde abgesaugt und mit Essigsäureethylester und Wasser nachgewaschen. Der Rückstand im Hochvakuum getrocknet. Man erhielt 0.507 g (11 % d. Th.). Die vereinigten Filtrate wurden in die Phasen getrennt, und die wässrige Phase wurde zweimal mit Essigsäureethylester extrahiert. Die vereinigten organischen Phasen wurden mit Wasser und gesättigter wässriger Natriumchloridlösung gewaschen, über Natriumsulfat getrocknet, filtriert und eingeengt und anschliessend am Hochvakuum getrocknet. Man erhielt so weitere 2.76 g (43 % d. Th., Reinheit ca. 70 %).

LC-MS (Methode 2): Rₜ = 0.58 min

MS (ESIpos): m/z = 308 (M+H)⁺

¹H-NMR (400 MHz, DMSO-d₆): δ = 1.84 (s, 3H), 2.95-3.08 (m, 2H), 4.85 (t, 2H), 7.39 (dd, 1H), 8.63-8.67 (m, 2H).

### Beispiel 14A

### 1-(3,3,4,4,4-Pentafluorbutyl)-1H-pyrazolo[3,4-b]pyridin-3-carboximidohydrazid

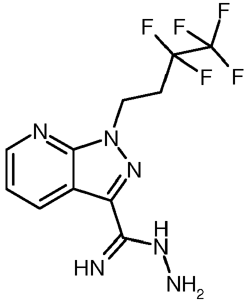

0.6 g (ca. 1.144 mmol) der Verbindung aus Beispiel 13A wurden in 10 ml Ethanol gelöst und bei 0°C mit 462 mg (4.574 mmol) Triethylamin sowie 71 mg (1.144 mmol) Hydrazinhydrat (80%-ige Lösung in Wasser) versetzt. Das Gemisch wurde über Nacht bei RT gerührt und anschließend am Rotationsverdampfer eingeengt. Es wurden 689 mg (ca. 60%-ige Reinheit) der Titelverbindung erhalten.

LC-MS (Methode 2): Rₜ = 0.57 min; MS (ESIpos): m/z = 323 (M+H)⁺

### Beispiel 15A

### 5-Fluor-1-(3,3,4,4,4-pentafluorbutyl)-1H-pyrazolo[3,4-b]pyridin-3-carboximidohydrazid

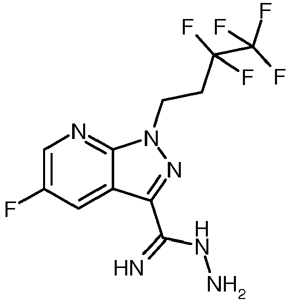

0.62 g (0.901 mmol) der Verbindung aus Beispiel 9A (Reinheit ca. 56 %) wurden in 10.3 ml Ethanol gelöst und bei 0°C mit 456 mg (4.506 mmol) Triethylamin sowie 70 mg (1.126 mmol) Hydrazinhydrat (80%-ige Lösung in Wasser) versetzt. Das Gemisch wurde über Nacht bei RT gerührt und anschließend am Rotationsverdampfer eingeengt. Es wurde eine Rohverbindung erhalten, die direkt weiter umgesetzt wurde.

LC-MS (Methode 1): Rₜ = 0.61 min; MS (ESIpos): m/z = 341 (M+H)⁺

### Beispiel 16A

### Methyl-2-{5-hydroxy-3-[1-(3,3,4,4,4-pentafluorbutyl)-1H-pyrazolo[3,4-b]pyridin-3-yl]-1,2,4-triazin-6-yl}-2-methylpropanoat

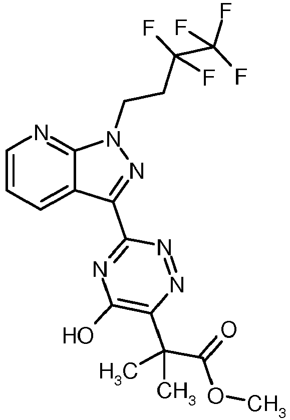

Die Rohsubstanz aus Beispiel 14A (ca 1.144 mmol) wurde in 15 ml Ethanol gelöst und mit 430 mg (2.288 mmol) Dimethyl-2,2-dimethyl-3-oxobutandioat (beschrieben in J. Am. Chem. Soc. 124(14), 3680-3691; 2002) versetzt. Das Gemisch wurde über Nacht bei RT gerührt und anschliessend für 1h zum Rückfluß erhitzt. Nach dem Abkühlen wurde der Feststoff abgesaugt, mit Ethanol gewaschen und das Filtrat eingeengt. Der Rückstand wurde mit Diethylether versetzt und von einem sich bildenden Niederschlag filtriert und mit Diethylether nachgewaschen. Das Filtrat wurde eingeengt und der Rückstand mittels präparativer HPLC gereinigt (Acetonitril:Wasser (+0.05 % Ameisensäure) - Gradient). Es wurden 264 mg der Titelverbindung erhalten (50 % d. Th.).

LC-MS (Methode 2): Rₜ = 1.03 min; MS (ESIpos): m/z = 461 (M+H)⁺

¹H-NMR (400 MHz, DMSO-d₆): δ [ppm] = 1.46 (s, 6H), 3.06-3.17 (m, 2H), 3.57 (s, 3H), 4.95 (t, 2H), 7.51 (dd, 1H), 8.70 (dd, 1H), 8.75 (dd, 1H), 14.52 (s, 1H).

### Beispiel 17A

### Methyl-2-{3-[5-fluor-1-(3,3,4,4,4-pentafluorbutyl)-1H-pyrazolo[3,4-b]pyridin-3-yl]-5-hydroxy-1,2,4-triazin-6-yl}-2-methylpropanoat

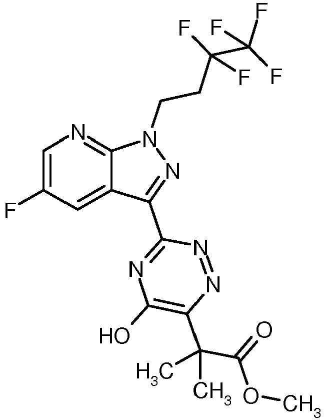

Die Rohsubstanz aus Beispiel 15A (ca. 0.845 mmol) wurde in 10 ml Ethanol gelöst und mit 318 mg (1.689 mmol) Dimethyl-2,2-dimethyl-3-oxobutandioat (beschrieben in J. Am. Chem. Soc. 124(14), 3680-3691; 2002) versetzt. Das Gemisch wurde über Nacht bei RT gerührt und anschliessend 6h zum Rückfluß erhitzt. Nach dem Abkühlen wurde eingeengt und der Rückstand mittels präparativer HPLC gereinigt (Acetonitril:Wasser (+0.05 % Ameisensäure) - Gradient). Es wurden 291 mg der Titelverbindung erhalten (72 % d. Th.).

LC-MS (Methode 2): Rₜ = 1.04 min; MS (ESIpos): m/z = 479 (M+H)⁺

¹H-NMR (400 MHz, DMSO-d₆): δ [ppm] = 1.46 (s, 6H), 3.07-3.14 (m, 2H), 3.56 (s, 3H), 4.94 (t, 2H), 8.40 (d, 1H), 8.85 (br s, 1H), 14.56 (s, 1H).

### Beispiel 18A

### 4-Amino-5,5-dimethyl-2-[1-(3,3,4,4,4-pentafluorbutyl)-1H-pyrazolo[3,4-b]pyridin-3-yl]-5,7-dihydro-6H-pyrrolo[2,3-d]pyrimidin-6-on

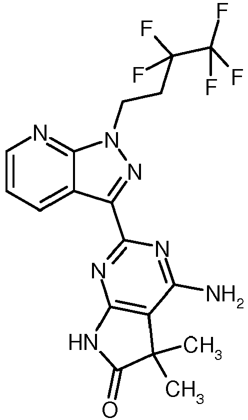

500 mg (1.361 mmol) Beispiel 13A wurden in tert.-Butanol (7.5 ml) vorgelegt und mit 183 mg (1.361 mmol) Kalium-tert.-butylat versetzt. Anschliessend wurden 226 mg (1.361 mmol) Beispiel 10A in tert.-Butanol (2.5 ml) zugetropft und die Mischung über Nacht zum Rückfluss erhitzt. Nach Abkühlen wurde mit Essigsäureethylester und Wasser versetzt, die Phasen getrennt und die wässrige Phase wurde zweimal mit Essigsäureethylester extrahiert. Die vereinigten organischen Phasen wurden mit gesättigter wässriger Natriumchloridlösung gewaschen, über Natriumsulfat getrocknet, filtriert und eingeengt. Der Rückstand wurde mit Methanol verrührt und von einem Feststoff abgesaugt. Dieser wurde kräftig mit Methanol nachgewaschen und die vereinigten Filtrate wurden eingeengt und anschliessend mittels präparativer HPLC gereinigt (Acetonitril:Wasser (+0.05 % Ameisensäure) - Gradient). Es wurden 127 mg der Titelverbindung erhalten (21 % d. Th.).

LC-MS (Methode 2): Rₜ = 0.93 min; MS (ESIpos): m/z = 442 (M+H)⁺

¹H-NMR (400 MHz, DMSO-d₆): δ [ppm] = 1.36 (s, 6H), 2.91-3.04 (m, 2H), 4.88 (t, 2H), 6.83 (br s, 2H), 7.38 (dd, 1H), 8.63 (dd, 1H), 9.02 (dd, 1H), 11.01 (br s, 1H).

### Beispiel 19A

### 4-Amino-2-[5-fluor-1-(3,3,4,4,4-pentafluorbutyl)-1H-pyrazolo[3,4-b]pyridin-3-yl]-5,5-dimethyl-5,7-dihydro-6H-pyrrolo[2,3-d]pyrimidin-6-on

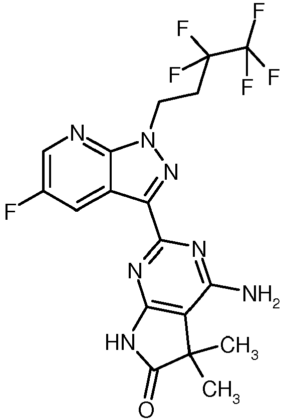

520 mg (1.350 mmol) Beispiel 9A wurden in tert.-Butanol (10 ml) vorgelegt und mit 181 mg (1.620 mmol) Kalium-tert.-butylat versetzt. Anschliessend wurden 224 mg (1.350 mmol) Beispiel 10A in tert.-Butanol (2.5 ml) zugegeben und die Mischung über Nacht zum Rückfluss erhitzt. Anschliessend wurden erneut 112 mg (0.675 mmol) Beispiel 10A zugegeben und es wurde für weitere 7.5 h zum Rückfluss erhitzt. Nach Abkühlen wurde das Reaktionsgemisch mit Wasser und Ethanol versetzt, und für 1 h im Ultraschallbad behandelt. Der entstandene Niederschlag wurde abgesaugt und mit Wasser nachgewaschen. Der Filterkuchen wurde mit wenig Ethanol (2-3 ml) verrührt und erneut abgesaugt. Der Feststoff wurde am Hochvakuum getrocknet. Es wurden 212 mg der Titelverbindung erhalten (34 % d. Th.).

LC-MS (Methode 1): Rₜ = 1.01 min; MS (ESIpos): m/z = 460 (M+H)⁺

¹H-NMR (400 MHz, DMSO-d₆): δ [ppm] = 1.36 (s, 6H), 2.92-3.04 (m, 2H), 4.87 (t, 2H), 6.88 (br s, 2H), 8.71 (br s, 1H), 8.85 (dd, 1H), 11.01 (br s, 1H).

### Beispiel 20A

### 4-Iod-5,5-dimethyl-2-[1-(3,3,4,4,4-pentafluorbutyl)-1H-pyrazolo[3,4-b]pyridin-3-yl]-5,7-dihydro-6H-pyrrolo[2,3-d]pyrimidin-6-on

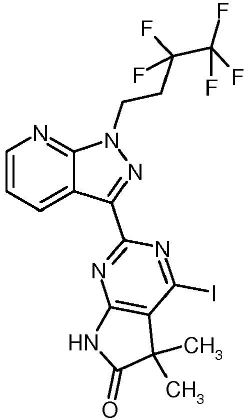

155 mg (0.351 mmol) von Beispiel 18A wurden in iso-Pentylnitrit (1.017 ml) und Diiodmethan (2.655 ml) vorgelegt und für 2d auf 85°C erhitzt. Nach Abkühlen wurde von einem Feststoff filtriert, mit Cyclohexan gewaschen und anschliessend der Feststoff mittels präparativer HPLC gereinigt (Acetonitril:Wasser (+0.05 % Ameisensäure) - Gradient). Es wurden 39 mg der Titelverbindung erhalten (20 % d. Th.).

LC-MS (Methode 2): Rₜ = 1.21 min; MS (ESIpos): m/z = 553 (M+H)⁺

¹H-NMR (400 MHz, DMSO-d₆): δ [ppm] = 1.44 (s, 6H), 2.96-3.06 (m, 2H), 4.94 (t, 2H), 7.48 (dd, 1H), 8.69 (dd, 1H), 8.79 (dd, 1H), 11.80 (br s, 1H).

### Beispiel 21A

### 2-[5-Fluor-1-(3,3,4,4,4-pentafluorbutyl)-1H-pyrazolo[3,4-b]pyridin-3-yl]-4-iod-5,5-dimethyl-5,7-dihydro-6H-pyrrolo[2,3-d]pyrimidin-6-on

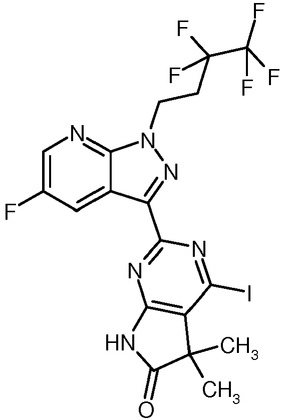

142 mg (0.310 mmol) von Beispiel 19A wurden in iso-Pentylnitrit (0.90 ml) und Diiodmethan (2.4 ml) vorgelegt und über Nacht auf 85°C erhitzt. Anschliessend wurde nochmals mit iso-Pentylnitrit (1 ml) versetzt und für weitere 22 h auf 85°C erhitzt. Danach wurde nochmals mit iso-Pentylnitrit (1 ml) versetzt und für weitere 12 h auf 85°C erhitzt. Nach Abkühlen wurde mittels präparativer HPLC gereinigt (Acetonitril:Wasser (+0.05 % Ameisensäure) - Gradient). Es wurden 71 mg der Titelverbindung erhalten (40 % d. Th.).

LC-MS (Methode 2): Rₜ = 1.28 min; MS (ESIpos): m/z = 571 (M+H)⁺

### Beispiel 22A

### 1-(Cyclopentylmethyl)-5-fluor-3-iod-1H-pyrazolo[3,4-b]pyridin

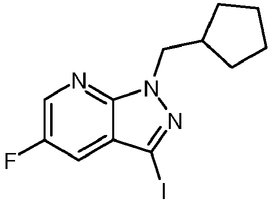

Es wurden 10.000 g (38.021 mmol) 5-Fluor-3-iod-1H-pyrazolo[3,4-b]pyridin und 13.627 g (41.823 mmol) Cäsiumcarbonat in 270 ml DMF vorgelegt und mit 8.785 g (41.823 mmol) (Iodmethyl)cyclopentan versetzt. Das Reaktionsgemisch wurde über Nacht bei RT gerührt, mit Wasser verdünnt und mit Essigsäureethylester extrahiert. Die organische Phase wurde über Natriumsulfat getrocknet und am Rotationsverdampfer eingeengt. Es wurden 6.370 g der Zielverbindung erhalten (Reinheit 83%, 49% d. Th.), welche als Rohprodukt weiter umgesetzt wurde.

LC-MS (Methode 2): Rₜ = 1.37 min; MS (ESIpos): m/z = 346 (M+H)⁺

### Beispiel 23A

### 1-(Cyclopentylmethyl)-5-fluor-1H-pyrazolo[3,4-b]pyridin-3-carbonitril

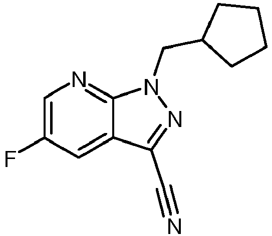

Unter einer Argonatmosphäre wurden 6.370 g (Reinheit 83%, 15.447 mmol) 1-(Cyclopentylmethyl)-5-fluor-3-iod-1H-pyrazolo[3,4-b]pyridin und 1.522 g (16.992 mmol) Kupfer(I)cyanid in 45 ml absolutem DMSO vorgelegt und 1.5 h bei 150°C erhitzt. Nach dem Abkühlen wurde der Ansatz mit Methanol verdünnt und über Celite filtriert. Die organische Phase wurde zweimal mit einer Mischung aus 25%-iger wässriger Ammoniak-Lösung und gesättigter wässriger Ammoniumchlorid-Lösung (v/v = 1:3) gewaschen, anschließend mit einer gesättigten wässrigen Natriumchlorid-Lösung gewaschen, über Natriumsulfat getrocknet und am Rotationsverdampfer eingeengt. Es wurden 3.670 g (Reinheit 86%, 97 % d. Th.) der Zielverbindung erhalten.

LC-MS (Methode 1): Rₜ = 2.56 min; MS (ESIpos): m/z = 245 (M+H)⁺

### Beispiel 24A

### 1-(Cyclopentylmethyl)-5-fluor-1H-pyrazolo[3,4-b]pyridin-3-carboximidamid-Acetat

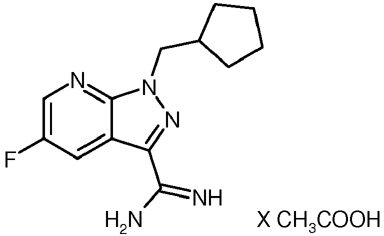

Unter einer Argonatmosphäre wurden portionsweise 296 mg (12.861 mmol) Natrium in Methanol eingerührt. Nach Abreaktion des Natriums wurden portionsweise 3.670 g (Reinheit 86%, ca. 12.861 mmol) 1-(Cyclopentylmethyl)-5-fluor-1H-pyrazolo[3,4-b]pyridin-3-carbonitril zugegeben und 2 h bei RT gerührt. Anschließend wurden 3.004 g (50.021 mmol) Essigsäure sowie 825 mg (15.433 mmol) Ammoniumchlorid addiert und die Mischung über Nacht unter Rückfluss gekocht. Das Reaktionsgemisch wurde eingeengt, der Rückstand mit 1N Natronlauge verrührt, der Niederschlag abgesaugt und im Hochvakuum getrocknet. Es wurden 2.840 g (Reinheit 98%, 83% d. Th.) der Zielverbindung erhalten.

LC-MS (Methode 2): Rₜ = 0.67 min; MS (ESIpos): m/z = 262 (M+H)⁺

### Beispiel 25A

### 4-Amino-2-[1-(cyclopentylmethyl)-5-fluor-1H-pyrazolo[3,4-b]pyridin-3-yl]-5,5-dimethyl-5,7-dihydro-6H-pyrrolo[2,3-d]pyrimidin-6-on

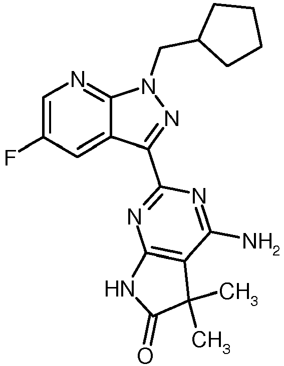

1.400 g (5.358 mmol) 1-(Cyclopentylmethyl)-5-fluor-1H-pyrazolo[3,4-b]pyridin-3-carboximid-amid wurden mit 30 ml *tert*.-Butanol, 1.068 g (6.429 mmol) Methyl-3,3-dicyan-2,2-dimethylpropanoat gelöst in 15 ml *tert*.-Butanol sowie 901 mg (8.037 mmol) Kalium-*tert*.-butylat versetzt und 24 h unter Rückfluss erhitzt. Das Reaktionsgemisch wurde am Rotationsverdampfer eingeengt, mit 60 ml Wasser/Ethanol (v/v = 5:1) verrührt, abgesaugt und im Hochvakuum getrocknet. Es wurden 1.296 g (57% d. Th.) der Zielverbindung erhalten.

LC-MS (Methode 1) Rₜ = 1.04 min; MS (ESIpos): m/z = 396 (M+H)⁺

### Beispiel 26A

### 2-[1-(Cyclopentylmethyl)-5-fluor-1H-pyrazolo[3,4-b]pyridin-3-yl]-4-iod-5,5-dimethyl-5,7-dihydro-6H-pyrrolo[2,3-d]pyrimidin-6-on

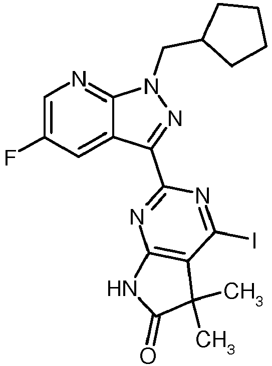

711 mg (1.799 mmol) 4-Amino-2-[1-(cyclopentylmethyl)-5-fluor-1H-pyrazolo[3,4-b]pyridin-3-yl]-5,5-dimethyl-5,7-dihydro-6H-pyrrolo[2,3-d]pyrimidin-6-on wurden mit 40.549 g (151.398 mmol) Diiodmethan sowie 4.420 g (37.738 mmol) Isopentylnitrit versetzt. Das Gemisch wurde 8 h bei 85 °C gerührt. Nach dem Abkühlen wurde filtriert, das Filtrat mit Cyclohexan verdünnt und über Kieselgel gesaugt. Das Kieselgel wurde mit Cyclohexan gewaschen und das Produkt mit Dichlormethan/Methanol (v/v = 50:2) euliert. Die gesammelten Fraktionen wurden am Rotationsverdampfer eingeengt, in 20 ml Essigsäureethylester und 5 ml Isopropanol aufgenommen, filtriert und erneut eingeengt. Der Rückstand wurde mit 5 ml Isopropanol verrührt. Der Feststoff wurde abgesaugt und im Hochvakuum getrocknet. Es wurden 230 mg (Reinheit 92%, 23% d. Th.) der Zielverbindung erhalten.

LC-MS (Methode 1) Rₜ = 1.41 min; MS (ESIpos): m/z = 507 (M+H)⁺

### Beispiel 27A

### 1-(Cyclopentylmethyl)-5-fluor-1H-pyrazolo[3,4-b]pyridin-3-carboximidohydrazid

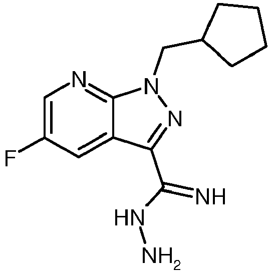

1.400 g (5.358 mmol) 1-(Cyclopentylmethyl)-5-fluor-1H-pyrazolo[3,4-b]pyridin-3-carboximid-amid wurden in 26 ml Ethanol vorgelegt und auf 0°C gekühlt. Es wurden 2.169 g (21.431 mmol) Triethylamin sowie 335 mg (5.358 mmol) 80%-iges Hydrazinhydrat zugegeben und 18 h bei Raumtemperatur gerührt. Das Gemisch wurde am Rotationsverdampfer eingeengt, in Essigsäureethylester aufgenommen und dreimal mit gesättigter wässriger Natriumchlorid-Lösung gewaschen. Die organische Phase wurde über Natriumsulfat getrocknet, am Rotationsverdampfer eingeengt und im Hochvakuum getrocknet. Es wurden 1.070 g (Reinheit 77%, 56 % d. Th.) der Zielverbindung erhalten.

LC-MS (Methode 2): Rₜ = 0.67 min; MS (ESIpos): m/z = 277 (M+H)⁺

### Beispiel 28A

### Methyl-2-{3-[1-(cyclopentylmethyl)-5-fluor-1H-pyrazolo[3,4-b]pyridin-3-yl]-5-hydroxy-1,2,4-triazin-6-yl}-2-methylpropanoat

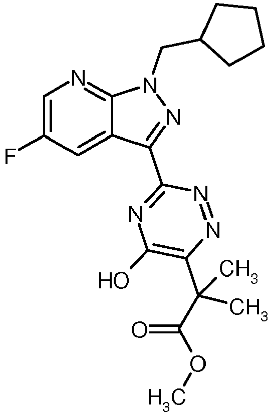

Es wurden 841 mg (4.473 mmol) Dimethyl-2,2-dimethyl-3-oxobutandioat in 20 ml Ethanol vorgelegt und zum Rückfluss erhitzt. Anschließend wurden 1.070 g (2.982 mmol) 1-(Cyclopentylmethyl)-5-fluor-1H-pyrazolo[3,4-b]pyridin-3-carboximidohydrazid suspendiert in 26 ml Ethanol addiert und über Nacht unter Rückfluss gekocht. Nach dem Abkühlen wurde das Gemisch eingeengt, in Acetonitril aufgenommen und filtriert. Das Filtrat wurde mittels präparativer HPLC (Eluent: Acetonitril/Wasser, Gradient 30:70 → 100:0) gereinigt. Es wurden 396 mg der Zielverbindung erhalten (32% d. Th.).

LC-MS (Methode 2): Rₜ = 1.15 min; MS (ESIpos): m/z = 415 (M+H)⁺

### Beispiel 29A

### 5-Fluor-3-iod-1-(4-methoxybenzyl)-1H-pyrazolo[3,4-b]pyridin

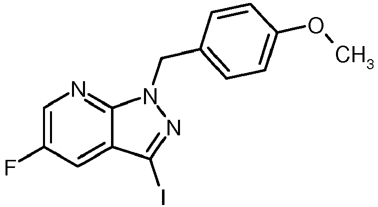

10.00 g (38.021 mmol) Beispiel 6A wurden in Analogie zur Vorschrift unter Beispiel 22A mit 4-Methoxybenzylchlorid umgesetzt. Man erhielt nach Chromatographie an Kieselgel (Eluent: Cyclohexan-Essigsäurethylester-Gemisch) 8.94 g (61 % d. Th.) der Titelverbindung.

LC-MS (Methode 1): Rₜ = 1.25 min

MS (ESIpos): m/z = 384 (M+H)⁺

### Beispiel 30A

### 5-Fluor-1-(4-methoxybenzyl)-1H-pyrazolo[3,4-b]pyridin-3-carbonitril

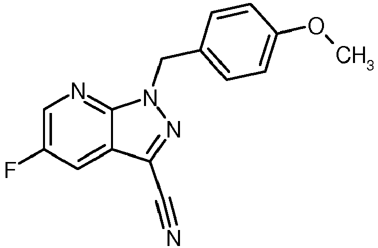

8.94 g (23.332 mmol) Beispiel 29A wurden in Analogie zur Vorschrift unter Beispiel 23A umgesetzt. Das so erhaltene Rohprodukt wurde ohne weitere Reinigung umgesetzt.

Ausbeute: 6.52 g (99 % d. Theorie)

LC-MS (Methode 2): Rₜ = 1.11 min

MS (ESIpos): m/z = 283 (M+H)⁺

### Beispiel 31A

### 5-Fluor-1-(4-methoxybenzyl)-1H-pyrazolo[3,4-b]pyridin-3-carboximidamid-Acetat

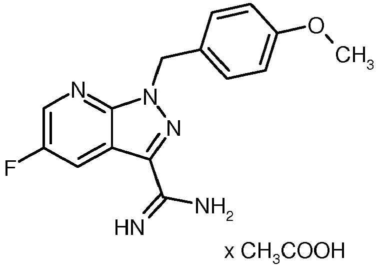

6.52 g (23.098 mmol) Beispiel 30A wurden in Analogie zur Vorschrift unter Beispiel 13A umgesetzt. Ausbeute: 6.16 g (74 % d. Theorie)

LC-MS (Methode 1): Rₜ = 0.55 min

MS (ESIpos): m/z = 300 (M+H)⁺

### Beispiel 32A

### 5-Fluor-1-(4-methoxybenzyl)-1H-pyrazolo[3,4-b]pyridin-3-carboximidohydrazid

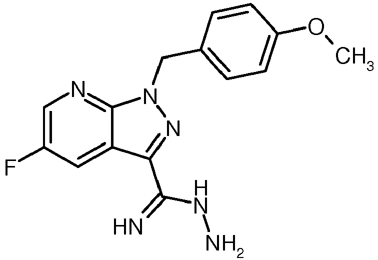

6.16 g (17.141 mmol) Beispiel 31A wurden in Analogie zur Vorschrift unter Beispiel 27A umgesetzt. Es wurden 4.90 g (90 % d. Th.) der Titelverbindung erhalten.

LC-MS (Methode 1): Rₜ = 0.57 min; MS (ESIpos): m/z = 315 (M+H)⁺

### Beispiel 33A

### Methyl-2-{3-[5-fluor-1-(4-methoxybenzyl)-1H-pyrazolo[3,4-b]pyridin-3-yl]-5-hydroxy-1,2,4-triazin-6-yl}-2-methylpropanoat

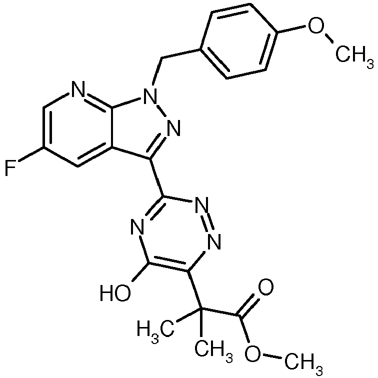

4.89 g (15.557 mmol) der Verbindung aus Beispiel 32A wurden in Analogie zur Vorschrift unter Beispiel 28A mit 4.391 g (23.336 mmol) Dimethyl-2,2-dimethyl-3-oxobutandioat (beschrieben in J. Am. Chem. Soc. 124(14), 3680-3691; 2002) umgesetzt. Nach vollständiger Umsetzung wurde ein Feststoff abfiltriert, welcher mit Ethanol nachgewaschen und anschliessend am Hochvakuum getrocknet wurde. Es wurden 6.04 g (85 % d. Th.) der Titelverbindung erhalten.

LC-MS (Methode 1): Rₜ = 1.05 min; MS (ESIpos): m/z = 453 (M+H)⁺

### Beispiel 34A

### 3-[5-Fluor-1-(4-methoxybenzyl)-1H-pyrazolo[3,4-b]pyridin-3-yl]-7,7-dimethyl-5,7-dihydro-6H-pyrrolo[2,3-e][1,2,4]triazin-6-on

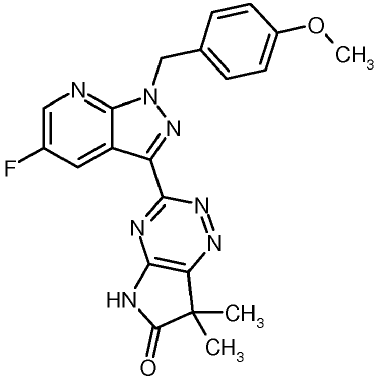

6.04 g (13.350 mmol) der Verbindung aus Beispiel 33A wurden in Analogie zur Vorschrift unter Beispiel 1 umgesetzt. Man erhielt nach Trocknung am Hochvakuum 1.27 g (22 % d. Th.) der Titelverbindung.

LC-MS (Methode 2): Rₜ = 1.02 min; MS (EIpos): m/z = 420 [M+H]⁺.

¹H-NMR (400 MHz, DMSO-d₆): δ [ppm] = 1.45 (s, 6H), 3.70 (s, 3H), 5.75 (s, 2H), 6.88 (d, 2H), 7.29 (d, 2H), 8.53 (dd, 1H), 8.78 (dd, 1H), 12.18 (s br, 1H).

### Beispiel 35A

### 3-[5-Fluor-1-(4-methoxybenzyl)-1H-pyrazolo[3,4-b]pyridin-3-yl]-7,7-dimethyl-5-{[2-(trimethyl-silyl)ethoxy]methyl}-5,7-dihydro-6H-pyrrolo[2,3-e][1,2,4]triazin-6-on

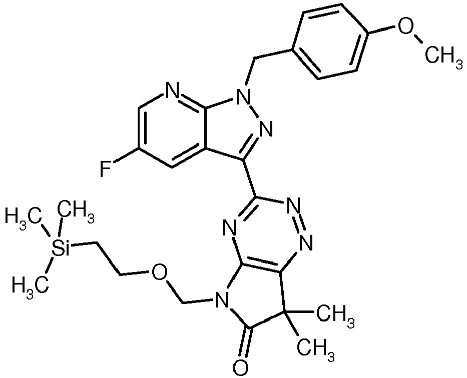

2.45 g (5.768 mmol) der Verbindung aus Beispiel 34A wurden mit 2.067 g (6.345 mmol) Cäsiumcarbonat in DMF (30 ml) versetzt. Danach wurden 1.221 ml (6.922 mmol) 2-(Trimethylsilyl)ethoxymethylchlorid zugegeben und es wurde 1 h bei Raumtemperatur gerührt. Anschliessend wurde von Feststoffen filtriert, mit DMF nachgewaschen, eingeengt und der Rückstand am Hochvakuum getrocknet. Es wurden 4.45 g Rohmaterial erhalten, die ohne weitere Reinigung in der nächsten Stufe eingesetzt wurden.

LC-MS (Methode 2): Rₜ = 1.43 min; MS (EIpos): m/z = 550 [M+H]⁺.

### Beispiel 36A

### 3-(5-Fluor-1H-pyrazolo[3,4-b]pyridin-3-yl)-7,7-dimethyl-5-{[2-(trimethylsilyl)ethoxy]methyl}-5,7-dihydro-6H-pyrrolo[2,3-e][1,2,4]triazin-6-on

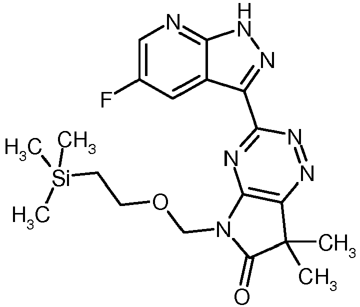

4.148 g (7.546 mmol) der Verbindung aus Beispiel 35A wurden in Acetonitril (110 ml) und Wasser (55 ml) aufgenommen und mit 12.411 g (22.638 mmol) Ammoniumcer(IV)-nitrat versetzt und für 20 min bei Raumtemperatur gerührt. Danach wurde mit viel Wasser versetzt und von einem Niederschlag filtriert. Dieser Feststoff wurde mit Wasser und anschliessend mit wenig Diethylether gewaschen. Es wurden 1.53 g (47 % d. Theorie) der Titelverbindung nach Trocknung am Hochvakuum erhalten.

LC-MS (Methode 2): Rₜ = 1.14 min; MS (EIpos): m/z = 430 [M+H]⁺.

### Beispiel 37A

### 3-[5-Fluor-1-(3,3,4,4-tetrafluorbutyl)-1H-pyrazolo[3,4-b]pyridin-3-yl]-7,7-dimethyl-5-{[2-(trimethylsilyl)ethoxy]methyl}-5,7-dihydro-6H-pyrrolo[2,3-e][1,2,4]triazin-6-on

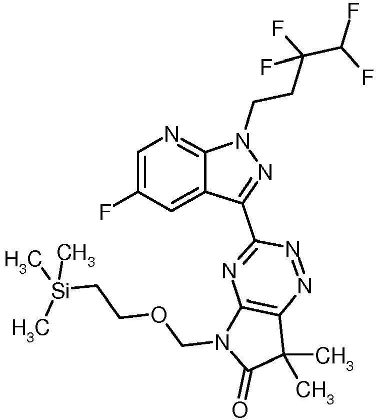

0.150 g (0.349 mmol) der Verbindung aus Beispiel 36A wurden in Analogie zur Vorschrift unter Beispiel 7A mit 1-Brom-3,3,4,4-tetrafluorbutan umgesetzt. In diesem Fall wurde kein Kaliumiodid zugesetzt. Nach Filtration wurde mittels präparativer HPLC gereinigt (Acetonitril:Wasser (+0.05 % Ameisensäure) - Gradient). Es wurden 97 mg der Titelverbindung als Gemisch von Isomeren (N1/N2-alkyliert, Verhältnis 3.2:1) erhalten (50% d. Th.).

LC-MS (Methode 2): Rₜ = 1.36 min (N2) und 1.38 min (N1); MS (EIpos): m/z = 558 [M+H]⁺.

### Beispiel 38A

### 3-{1-[(3,3-Difluorcyclobutyl)methyl]-5-fluor-1H-pyrazolo[3,4-b]pyridin-3-yl}-7,7-dimethyl-5-{[2-(trimethylsilyl)ethoxy]methyl}-5,7-dihydro-6H-pyrrolo[2,3-e][1,2,4]triazin-6-on

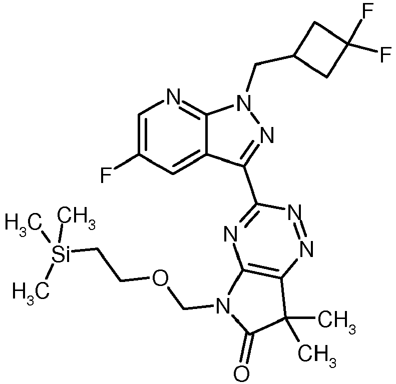

137 mg (0.524 mmol) Triphenylphosphin wurde in einem Kolben in 4 ml Tetrahydrofuran gelöst und auf 0°C gekühlt. Danach wurden 101 µl (0.524 mmol) Diisopropylazodicarboxylat zugegeben und die Lösung wurde 1 h bei 0°C gerührt (Lösung 1). In einem weiteren Kolben wurden 0.150 g (0.349 mmol) der Verbindung aus Beispiel 36A und 64 mg (0.542 mmol) 3,3-Difluorcyclobutyl-methanol in Tetrahydrofuran (6 ml) gelöst und auf 0°C gekühlt (Lösung 2). Danach wurde zu dieser Lösung 2 Lösung 1 hinzugegeben und es wurde über Nacht bei Raumtemperatur gerührt. Anschliessend wurde nochmals Lösung 1 wie oben beschrieben mit 274 mg (1.048 mmol) Triphenylphosphin und 203 µl (1.048 mmol) Diisopropylazodicarboxylat hergestellt und zusammen mit 127 mg (1.048 mmol) 3,3-Difluorcyclobutyl-methanol und Dichlormethan (5 ml) bei 0°C zum Ansatz gegeben. Nach Rühren über Nacht bei Raumtemperatur wurde mittels präparativer HPLC gereinigt (Acetonitril:Wasser (+0.05 % Ameisensäure) - Gradient). Es wurden 102 mg der Titelverbindung als Gemisch von Isomeren (N1/N2-alkyliert, Verhältnis 1.4:1) erhalten (55% d. Th.).

LC-MS (Methode 2): Rₜ = 1.38 min (N2) und 1.41 min (N1); MS (EIpos): m/z = 558 [M+H]⁺.

### Beispiel 39A

### Diethyl-(dicyanmethyl)(methyl)malonat

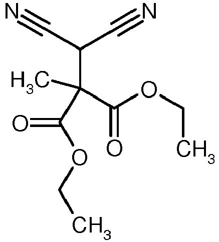

In THF (120 ml) wurden 19.156 g (75.686 mmol) 2-Brom-2-methylmalonsäurediethylester vorgelegt und mit 5 g (75.686 mmol) Malonsäuredinitril und anschliessend mit 8.493 g (75.686 mmol) Kalium-tert.-butylat versetzt. Danach wurde über Nacht zum Rückfluss erhitzt. Anschliessend wurde der Ansatz mit Essigsäureethylester und ges. Ammoniumchlorid-Lösung versetzt und die Phasen getrennt. Die wässrige Phase wurde noch zweimal mit Essigsäureethylester extrahiert. Die vereinigten organischen Phasen wurden mit gesättigter wässriger Natriumchlorid-Lösung gewaschen, über Natriumsulfat getrocknet, filtriert und bis zur Trockene eingeengt. Man erhielt ein Rohprodukt, welches per Chromatographie an Kieselgel (Cyclohexan-Essigsäureethylester 9:1) gereinigt wurde.

Ausbeute: 5.94 g (32% d. Th.)

¹H-NMR (400 MHz, CDCl₃): δ [ppm] = 1.32 (t, 6H), 1.80 (s, 3H), 4.28-4.37 (m, 4H), 4.53 (s, 1H).

### Beispiel 40A

### Ethyl-4-amino-5-methyl-6-oxo-2-[1-(3,3,4,4,4-pentafluorbutyl)-1H-pyrazolo[3,4-b]pyridin-3-yl]-6,7-dihydro-5H-pyrrolo[2,3-d]pyrimidin-5-carboxylat

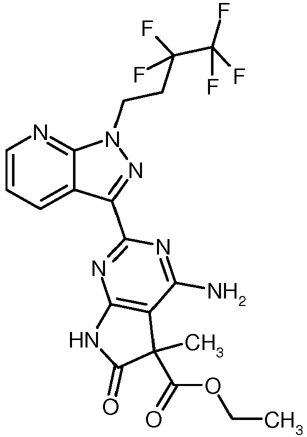

0.5 g (1.361 mmol) Beispiel 13A (70 %ige Reinheit) wurden in tert.-Butanol (10 ml) vorgelegt und mit 272 mg (2.723 mmol) Kaliumhydrogencarbonat versetzt. Anschliessend wurden 372 mg (1.566 mmol) Beispiel 39A zugegeben und die Mischung für 5h zum Rückfluss erhitzt. Nach Abkühlen wurde mit Wasser versetzt, 30min gerührt und danach von einem Niederschlag filtriert. Dieser wurde mit wenig Wasser und Diethylether gewaschen und über Nacht am Hochvakuum getrocknet. So konnten 0.458 g (63 % d. Th.) der Titelverbindung in 94 %iger Reinheit erhalten werden.

LC-MS (Methode 2): Rₜ = 1.00 min; MS (ESIpos): m/z = 500 (M+H)⁺

### Beispiel 41A

### Ethyl-4-brom-5-methyl-6-oxo-2-[1-(3,3,4,4,4-pentafluorbutyl)-1H-pyrazolo[3,4-b]pyridin-3-yl]-6,7-dihydro-5H-pyrrolo[2,3-d]pyrimidin-5-carboxylat

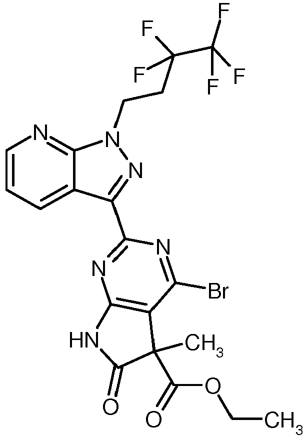

437 mg (0.875 mmol) Beispiel 40A wurden in 1,2-Dichlorethan (14.2 ml) vorgelegt und mit 0.176 ml (1.313 mmol) Isopentylnitrit und 234 mg (1.050 mmol) Kupfer-(II)-bromid versetzt. Anschliessend wurde für 10h auf 65°C erhitzt. Nach Abkühlen wurde mit Wasser und Dichlormethan versetzt und die Phasen getrennt. Die wässrige Phase wurde einmal mit Essigsäureethylester versetzt und die vereinigten organischen Phasen wurden über Natriumsulfat getrocknet, eingeengt und der Rückstand mittels präparativer Chromatographie an Kieselgel (Laufmittel: Dichlormethan/Methanol 66:1) gereinigt. Es wurden 0.393 g (89 % d. Th.) der Titelverbindung in ca. 90 %iger Reinheit erhalten.

LC-MS (Methode 2): Rₜ = 1.23 min; MS (ESIpos): m/z = 563 (⁷⁹Br),565 (⁸¹Br) (M+H)⁺

### Ausführungsbeispiele:

### Beispiel 1

### 7,7-Dimethyl-3-[1-(3,3,4,4,4-pentafluorbutyl)-1H-pyrazolo[3,4-b]pyridin-3-yl]-5,7-dihydro-6H-pyrrolo[2,3-e][1,2,4]triazin-6-on

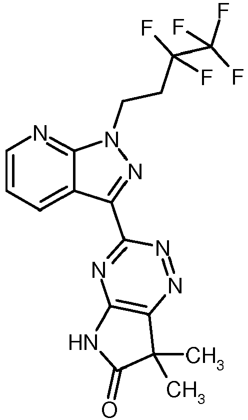

263 mg (0.571 mmol) der Verbindung aus Beispiel 16A wurden mit 4 ml Phosphorylchlorid versetzt und über Nacht bei RT gerührt. Das Reaktionsgemisch wurde in 38 ml Acetonitril gelöst und unter Eiskühlung in 24 ml konzentrierte wässriger Ammoniak-Lösung (33%-ig) eingerührt. Es wurde 2 Tage bei RT gerührt. Anschliessend wurde das Reaktionsgemisch eingeengt. Der Rückstand wurde in Wasser und Ethanol aufgenommen und 1h bei RT gerührt. Der entstandene Niederschlag wurde abgesaugt, und mit Wasser und Ethanol nachgewaschen. Dieser Rückstand wurde mittels präparativer HPLC gereinigt (Acetonitril:Wasser (+0.05 % Ameisensäure) - Gradient). Es wurden 119 mg der Titelverbindung erhalten (49 % d. Th.).

LC-MS (Methode 2): Rₜ = 0.98 min; MS (EIpos): m/z = 428 [M+H]⁺.

¹H-NMR (400 MHz, DMSO-d₆): δ [ppm] = 1.47 (s, 6H), 2.96-3.07 (m, 2H), 4.95 (t, 2H), 7.48 (dd, 1H), 8.71 (dd, 1H), 8.85 (dd, 1H), 12.21 (br s, 1H).

### Beispiel 2

### 3-[5-Fluor-1-(3,3,4,4,4-pentafluorbutyl)-1H-pyrazolo[3,4-b]pyridin-3-yl]-7,7-dimethyl-5,7-dihydro-6H-pyrrolo[2,3-e][1,2,4]triazin-6-on

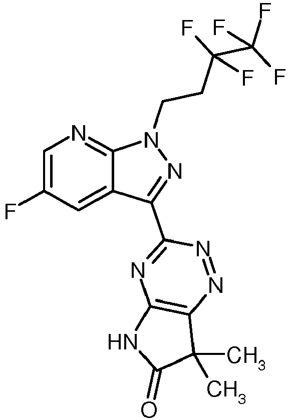

290 mg (0.606 mmol) der Verbindung aus Beispiel 17A wurden mit 4.172 ml Phosphorylchlorid versetzt und über Nacht bei RT gerührt. Das Reaktionsgemisch wurde in 40 ml Acetonitril gelöst und unter Eiskühlung in 27 ml konzentrierte wässriger Ammoniak-Lösung (33%-ig) eingerührt. Es wurde 2 Tage bei Raumtemperatur gerührt. Anschliessend wurde eingeengt. Der Rückstand wurde in Wasser und Ethanol aufgenommen und im Ultraschallbad behandelt. Es bildete sich ein Niederschlag, welcher abgesaugt wurde und mit Wasser, Ethanol und danach mit Diethylether nachgewaschen wurde. Dieser Rückstand wurde mit DMF versetzt und anschliessend wurde Acetonitril und Wasser zugegeben und abermals abgesaugt. Der Niederschlag wurde mit Acetonitril und Wasser gewaschen und anschliessend im Hochvakuum getrocknet. Es wurden 149 mg der Titelverbindung erhalten (52 % d. Th., Reinheit 94 %).

LC-MS (Methode 2): Rₜ = 1.05 min; MS (EIpos): m/z = 446 [M+H]⁺.

¹H-NMR (400 MHz, DMSO-d₆): δ [ppm] = 1.47 (s, 6H), 2.96-3.07 (m, 2H), 4.95 (t, 2H), 8.56 (dd, 1H), 8.80 (br s, 1H), 12.21 (br s, 1H).

### Beispiel 3

### 5,5-Dimethyl-2-[1-(3,3,4,4,4-pentafluorbutyl)-1H-pyrazolo[3,4-b]pyridin-3-yl]-5,7-dihydro-6H-pyrrolo[2,3-d]pyrimidin-6-on

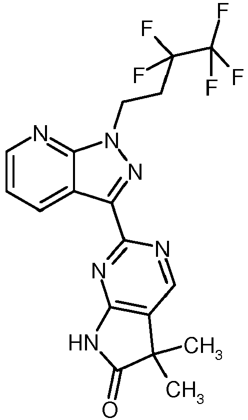

39 mg (0.071 mmol) von Beispiel 20A wurden in DMF (4 ml) gelöst und zu 20 mg Palladium auf Kohle (10 %-ig) in 1 ml DMF gegeben und für 12 h bei Normaldruck hydriert. Anschliessend wurde über Celite filtriert, mit DMF nachgewaschen und bis zur Trockene eingeengt. Der Rückstand wurde mittels präparativer HPLC gereinigt (Acetonitril:Wasser (+0.05 % Ameisensäure) - Gradient). Es wurden 22 mg der Titelverbindung erhalten (74 % d. Th.).

LC-MS (Methode 2): Rₜ = 1.02 min; MS (ESIpos): m/z = 427 (M+H)⁺

¹H-NMR (400 MHz, DMSO-d₆): δ [ppm] = 1.39 (s, 6H), 2.96-3.06 (m, 2H), 4.92 (t, 2H), 7.43 (dd, 1H), 8.66-8.69 (m, 2H), 8.86 (dd, 1H), 11.61 (br s, 1H).

### Beispiel 4

### 2-[5-Fluor-1-(3,3,4,4,4-pentafluorbutyl)-1H-pyrazolo[3,4-b]pyridin-3-yl]-5,5-dimethyl-5,7-dihydro-6H-pyrrolo[2,3-d]pyrimidin-6-on

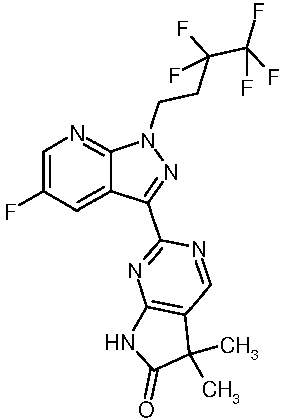

39 mg (0.068 mmol) von Beispiel 21A wurden in DMF (5 ml) gelöst und mit 20 mg Palladium auf Kohle (10 %) versetzt und für 12 h bei Normaldruck hydriert. Anschliessend wurde über Celite filtriert, mit DMF nachgewaschen und bis zur Trockene eingeengt. Der Rückstand wurde mittels präparativer HPLC gereinigt (Acetonitril:Wasser (+0.05 % Ameisensäure) - Gradient). Es wurden 8 mg der Titelverbindung erhalten (74 % d. Th.).

LC-MS (Methode 2): Rₜ = 1.10 min; MS (ESIpos): m/z = 445 (M+H)⁺

¹H-NMR (400 MHz, DMSO-d₆): δ [ppm] = 1.39 (s, 6H), 2.96-3.06 (m, 2H), 4.91 (t, 2H), 8.59 (dd, 1H), 8.65 (m, 1H), 8.76 (dd, 1H), 11.60 (br s, 1H).

### Beispiel 5

### 2-[1-(Cyclopentylmethyl)-5-fluor-1H-pyrazolo[3,4-b]pyridin-3-yl]-5,5-dimethyl-5,7-dihydro-6H-pyrrolo[2,3-d]pyrimidin-6-on

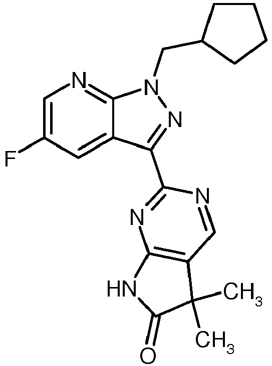

230 mg (0.454 mmol) 2-[1-(Cyclopentylmethyl)-5-fluor-1H-pyrazolo[3,4-b]pyridin-3-yl]-4-iod-5,5-dimethyl-5,7-dihydro-6H-pyrrolo[2,3-d]pyrimidin-6-on wurden in 8 ml absolutem DMF gelöst, mit 150 mg 10% Palladium auf Kohle versetzt und unter Normaldruck mit Wasserstoff für 3 h hydriert. Das Reaktionsgemisch wurde über Celite filtriert und eingeengt. Der Rückstand wurde mit 4 ml Acetonitril verrührt, abgesaugt und im Hochvakuum getrocknet. Es wurden 123 mg der Zielverbindung erhalten (Reinheit 94%, 67% d. Th.).

LC-MS (Methode 1) Rₜ = 1.18 min; MS (ESIpos): m/z = 381 (M+H)⁺

¹H-NMR (400MHz, DMSO-d₆): δ [ppm]= 1.30-1.65 (m, 14H), 2.56-2.63 (m, 1H), 4.50 (d, 2H), 8.57 (dd, 1H), 8.65 (s, 1H), 8.71 (dd, 1H), 11.64 (s br, 1H).

### Beispiel 6

### 3-[1-(Cyclopentylmethyl)-5-fluor-1H-pyrazolo[3,4-b]pyridin-3-yl]-7,7-dimethyl-5,7-dihydro-6H-pyrrolo[2,3-e][1,2,4]triazin-6-on

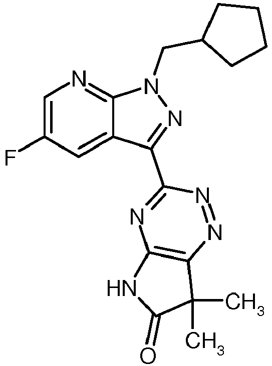

Es wurden 376 mg (0.863 mmol) Methyl-2-{3-[1-(cyclopentylmethyl)-5-fluor-1H-pyrazolo[3,4-b]pyridin-3-yl]-5-hydroxy-1,2,4-triazin-6-yl}-2-methylpropanoat mit 5 ml (53.642 mmol) Phosphorylchlorid versetzt und über Nacht bei RT gerührt. Die Reaktionslösung wurde mit 20 ml trockenem Acetonitril verdünnt, unter Eiskühlung langsam in 83 ml einer 25%-igen wässrigen Ammoniak-Lösung getropft und über Nacht bei RT gerührt. Das Reaktionsgemisch wurde am Rotationsverdampfer eingeengt und der Niederschlag abfiltriert. Der Rückstand wurde mit DMF/Methanol verrührt, abgesaugt und im Hochvakuum getrocknet. Es wurden 84 mg der Zielverbindung erhalten (24% d. Th.).

LC-MS (Methode 1) Rₜ = 1.12 min; MS (ESIpos): m/z = 382 (M+H)⁺

¹H-NMR (400MHz, DMSO-d₆): δ [ppm]= 1.30-1.65 (m, 14H), 2.57-2.63 (m, 1H), 4.53 (d, 2H), 8.54 (dd, 1H), 8.74 (dd, 1H).

### Beispiel 7

### 3-[5-Fluor-1-(3,3,4,4-tetrafluorbutyl)-1H-pyrazolo[3,4-b]pyridin-3-yl]-7,7-dimethyl-5,7-dihydro-6H-pyrrolo[2,3-e][1,2,4]triazin-6-on

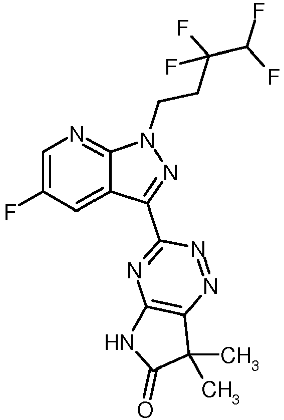

96 mg (0.173 mmol) der Verbindung aus Beispiel 37A wurden in Dichlormethan (4 ml) und Trifluoressigsäure (1 ml) 4.5 h bei Raumtemperatur gerührt. Anschliessend wurde bis zur Trockene eingeengt. Der Rückstand wurde in Ethanol/2N Salzsäure (4:1, 15 ml) 3 h bei 45 °C gerührt. Danach wurde bis zur Trockene eingeengt. Nach Reinigung mittels präparativer HPLC (Methanol:Wasser (+1 % Trifluoressigsäure) - Gradient) wurden 31 mg der Titelverbindung erhalten (42% d. Th.).

LC-MS (Methode 2): Rₜ = 0.95 min; MS (EIpos): m/z = 428 [M+H]⁺.

¹H-NMR (400 MHz, DMSO-d₆): δ [ppm] = 1.47 (s, 6H), 2.76-2.87 (m, 2H), 4.89 (t, 2H), 6.39-6.62 (m, 1H), 8.55 (dd, 1H), 8.78 (dd, 1H), 12.17 (s br, 1H).

### Beispiel 8

### 3-{1-[(3,3-Difluorcyclobutyl)methyl]-5-fluor-1H-pyrazolo[3,4-b]pyridin-3-yl}-7,7-dimethyl-5,7-dihydro-6H-pyrrolo[2,3-e][1,2,4]triazin-6-on

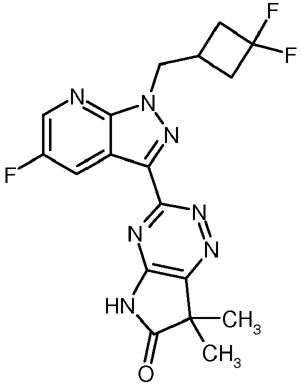

100 mg (0.189 mmol) der Verbindung aus Beispiel 38A wurden in Analogie zur Vorschrift unter Beispiel 7 umgesetzt. Nach Reinigung mittels präparativer HPLC (Acetonitril:Wasser (+1 % Trifluoressigsäure) - Gradient) wurden 15 mg der Titelverbindung erhalten (19% d. Th.).

LC-MS (Methode 1): Rₜ = 1.00 min; MS (EIpos): m/z = 404 [M+H]⁺.

¹H-NMR (400 MHz, DMSO-d₆): δ [ppm] = 1.47 (s, 6H), 2.47-2.54 (m, 2H, von Solvent überlagert), 2.64-2.73 (m, 2H), 2.78-2.86 (m, 1H), 4.75 (d, 2H), 8.54 (dd, 1H), 8.76 (dd, 1H), 12.14 (s br, 1H).

### Beispiel 9

### Ethyl-5-methyl-6-oxo-2-[1-(3,3,4,4,4-pentafluorbutyl)-1H-pyrazolo[3,4-b]pyridin-3-yl]-6,7-dihydro-5H-pyrrolo[2,3-d]pyrimidin-5-carboxylat

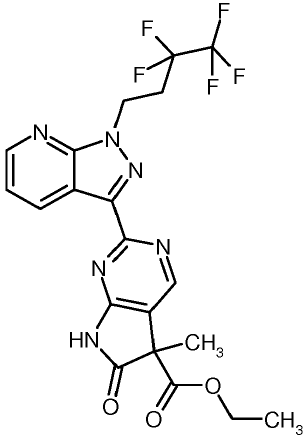

190 mg (0.304 mmol, ca. 90%-ige Reinheit) Beispiel 41A wurden in Analogie zu Beispiel 3 hydriert. Es wurden 36 mg (18 % d. Th.) der Titelverbindung in 77%iger Reinheit erhalten.

LC-MS (Methode 2): Rₜ = 1.08 min; MS (ESIpos): m/z = 485 (M+H)⁺

### Beispiel 10

### N-Cyclopropyl-5-methyl-6-oxo-2-[1-(3,3,4,4,4-pentafluorbutyl)-1H-pyrazolo[3,4-b]pyridin-3-yl]-6,7-dihydro-5H-pyrrolo[2,3-d]pyrimidin-5-carboxamid (Racemat)

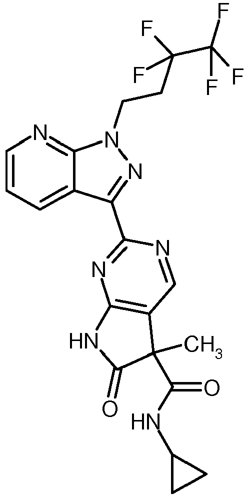

34 mg (0.054 mmol, 77%-ige Reinheit) Beispiel 9 wurden in Methanol (0.5 ml) aufgenommen und mit 30 mg (0.540 mmol) Cyclopropylamin versetzt. Anschliessend wurde 1 Tag bei 80°C in der Mikrowelle behandelt. Nach Reinigung mittels präparativer HPLC (Acetonitril:Wasser (+0.1 % Ameisensäure) - Gradient) wurden 13 mg der Titelverbindung erhalten (50% d. Th.).

LC-MS (Methode 2): Rₜ = 0.99 min; MS (EIpos): m/z = 496 [M+H]⁺.

¹H-NMR (400 MHz, DMSO-d₆): δ [ppm] = 0.41-0.46 (m, 2H), 0.59-0.61 (m, 2H), 1.61 (s, 3H), 2.62-2.66 (m, 1H), 2.95-3.08 (m, 2H), 4.93 (t, 2H), 7.44 (dd, 1H), 7.75 (d, 1H), 8.58 (s, 1H), 8.68 (dd, 1H), 8.87 (dd, 1H), 11.79 (s, 1H).

### B. Bewertung der pharmakologischen Wirksamkeit

Die pharmakologische Wirkung der erfindungsgemäßen Verbindungen kann in folgenden Assays gezeigt werden:

### B-1. Gefäßrelaxierende Wirkung in vitro

Kaninchen werden durch Nackenschlag betäubt und entblutet. Die Aorta wird entnommen, von anhaftendem Gewebe befreit, in 1.5 mm breite Ringe geteilt und einzeln unter einer Vorspannung in 5 ml-Organbäder mit 37°C warmer, Carbogen-begaster Krebs-Henseleit-Lösung folgender Zusammensetzung gebracht (jeweils mM): Natriumchlorid: 119; Kaliumchlorid: 4.8; Calciumchlorid-Dihydrat: 1; Magnesiumsulfat-Heptahydrat: 1.4; Kaliumdihydrogenphosphat: 1.2; Natriumhydrogencarbonat: 25; Glucose: 10. Die Kontraktionskraft wird mit Statham UC2-Zellen erfasst, verstärkt und über A/D-Wandler (DAS-1802 HC, Keithley Instruments München) digitalisiert sowie parallel auf Linienschreiber registriert. Zur Erzeugung einer Kontraktion wird Phenylephrin dem Bad kumulativ in ansteigender Konzentration zugesetzt. Nach mehreren Kontrollzyklen wird die zu untersuchende Substanz in jedem weiteren Durchgang in jeweils steigender Dosierung zugesetzt und die Höhe der Kontraktion mit der Höhe der im letzten Vordurchgang erreichten Kontraktion verglichen. Daraus wird die Konzentration errechnet, die erforderlich ist, um die Höhe des Kontrollwertes um 50% zu reduzieren (IC₅₀-Wert). Das Standardapplikationsvolumen beträgt 5 µl, der DMSO-Anteil in der Badlösung entspricht 0.1%.

Repräsentative IC₅₀-Werte für die erfindungsgemäßen Verbindungen sind in der nachstehenden Tabelle (Tabelle 1) wiedergegeben:

**Tabelle 1:**

| Beispiel Nr. | IC₅₀ [nM] |
|---|---|
| 1 | 263 |
| 2 | 129 |
| 3 | 46 |
| 4 | 101 |
| 5 | 23 |
| 6 | 102 |

### B-2. Wirkung an rekombinanter Guanylatcyclase-Reporterzelllinie

Die zelluläre Wirkung der erfindungsgemäßen Verbindungen wird an einer rekombinanten Guanylatcyclase-Reporterzelllinie, wie in F. Wunder et al., Anal. Biochem. 339, 104-112 (2005) beschrieben, bestimmt.

Repräsentative Werte (MEC = minimal effektive Konzentration) für die erfindungsgemäßen Verbindungen sind in der nachstehenden Tabelle (Tabelle 2) wiedergegeben:

**Tabelle 2:**

| Beispiel Nr. | MEC [µM] |
|---|---|
| 1 | 0.3 |
| 2 | 0.1 |
| 3 | 0.03 |
| 4 | 0.03 |
| 5 | 0.1 |
| 6 | 0.1 |
| 7 | 0.3 |
| 8 | 3.0 |

### B-3. Radiotelemetrische Blutdruckmessung an wachen, spontan hypertensiven Ratten

Für die im Folgenden beschriebene Blutdruckmessung an wachen Ratten wird ein im Handel erhältliches Telemetriesystem der Firma DATA SCIENCES INTERNATIONAL DSI, USA eingesetzt.

Das System besteht aus 3 Hauptkomponenten:
Implantierbare Sender (Physiotel® Telemetrietransmitter)
Empfänger (Physiotel® Receiver), die über einen Multiplexer (DSI Data Exchange Matrix ) mit einem
Datenakquisitionscomputer
verbunden sind.

Die Telemetrieanlage ermöglicht eine kontinuierliche Erfassung von Blutdruck Herzfrequenz und Körperbewegung an wachen Tieren in ihrem gewohnten Lebensraum.

### Tiermaterial

Die Untersuchungen werden an ausgewachsenen weiblichen spontan hypertensiven Ratten (SHR Okamoto) mit einem Körpergewicht von >200 g durchgeführt. SHR/NCrl von Okamoto Kyoto School of Medicine, 1963 wurden aus männlichen Wistar Kyoto Ratten mit stark erhöhtem Blutdruck und weiblichen mit leicht erhöhtem Blutdruck gekreuzt und in der F13 an die U.S. National Institutes of Health abgegeben.

Die Versuchstiere werden nach Senderimplantation einzeln in Makroion - Käfigen Typ 3 gehalten. Sie haben freien Zugang zu Standardfutter und Wasser.

Der Tag - Nacht - Rhythmus im Versuchslabor wird per Raumbeleuchtung um 6:00 Uhr morgens und um 19:00 Uhr abends gewechselt.

### Senderimplantation

Die eingesetzten Telemetriesender TA11 PA - C40 werden den Versuchstieren mindestens 14 Tage vor dem ersten Versuchseinsatz unter aseptischen Bedingungen chirurgisch implantiert. Die so instrumentierten Tiere sind nach Abheilen der Wunde und Einwachsen des Implantats wiederholt einsetzbar.

Zur Implantation werden die nüchternen Tiere mit Pentobabital (Nembutal, Sanofi: 50mg/kg i.p. ) narkotisiert und an der Bauchseite weiträumig rasiert und desinfiziert. Nach Eröffnung des Bauchraumes entlang der Linea alba wird der flüssigkeitsgefüllte Meßkatheter des Systems oberhalb der Bifurcation nach cranial in die Aorta descendens eingesetzt und mit Gewebekleber (VetBonD TM, 3M) befestigt. Das Sendergehäuse wird intraperitoneal an der Bauchwandmuskulatur fixiert und die Wunde wird schichtweise verschlossen.

Postoperativ wird zur Infektionsprophylaxe ein Antibiotikum verabreicht (Tardomyocel COMP Bayer 1ml/kg s.c.)

### Substanzen und Lösungen

Wenn nicht anders beschrieben werden die zu untersuchenden Substanzen jeweils einer Gruppe von Tieren (n = 6 ) per Schlundsonde oral verabreicht. Entsprechend einem Applikationsvolumen von 5 ml/kg Körpergewicht werden die Testsubstanzen in geeigneten Lösungsmittelgemischen gelöst oder in 0.5%-iger Tylose suspendiert.

Eine Lösungsmittel- behandelte Gruppe von Tieren wird als Kontrolle eingesetzt.

### Versuchsablauf

Die vorhandene Telemetrie - Meßeinrichtung ist für 24 Tiere konfiguriert. Jeder Versuch wird unter einer Versuchsnummer registiert (VJahr Monat Tag).

Den in der Anlage lebenden instrumentierten Ratten ist jeweils eine eigene Empfangsantenne zugeordnet (1010 Receiver, DSI).

Die implantierten Sender sind über einen eingebauten Magnetschalter von außen aktivierbar. Sie werden bei Versuchsvorlauf auf Sendung geschaltet. Die ausgestrahlten Signale können durch ein Datenakquisitionssystem (Dataquest TM A.R.T. for WINDOWS, DSI ) online erfasst und entsprechend aufgearbeitet werden. Die Ablage der Daten erfolgt jeweils in einem hierfür eröffneten Ordner der die Versuchsnummer trägt.

Im Standardablauf werden über je 10 Sekunden Dauer gemessen:
Systolischer Blutdruck (SBP)
Diastolischer Blutdruck (DBP)
Arterieller Mitteldruck (MAP)
Herzfrequenz (HR)
Aktivität (ACT).

Die Messwerterfassung wird rechnergesteuert in 5 Minuten Abständen wiederholt. Die als Absolutwert erhobenen Quelldaten werden im Diagramm mit dem aktuell gemessenen Barometerdruck (Ambient Pressure Reference Monitor; APR-1) korrigiert und in Einzeldaten abgelegt. Weitere technische Details sind der umfangreichen Dokumentation der Herstellerfirma (DSI) zu entnehmen.

Wenn nicht anders beschrieben erfolgt die Verabreichung der Prüfsubstanzen am Versuchstag um 9.00 Uhr. Im Anschluss an die Applikation werden die oben beschriebenen Parameter 24 Stunden gemessen.

### Auswertung

Nach Versuchsende werden die erhobenen Einzeldaten mit der Analysis-Software (DATAQUEST TM A. R.T. TM ANALYSIS) sortiert. Als Leerwert werden hier 2 Stunden vor Applikation angenommen, so dass der selektierte Datensatz den Zeitraum von 7:00 Uhr am Versuchstag bis 9:00 Uhr am Folgetag umfasst.

Die Daten werden über eine voreinstellbare Zeit durch Mittelwertbestimmung geglättet (15 Minuten Average) und als Textdatei auf einen Datenträger übertragen. Die so vorsortierten und komprimierten Messwerte werden in Excel-Vorlagen übertragen und tabellarisch dargestellt. Die Ablage der erhobenen Daten erfolgt pro Versuchstag in einem eigenen Ordner, der die Versuchsnummer trägt. Ergebnisse und Versuchsprotokolle werden in Papierform nach Nummern sortiert in Ordnern abgelegt.

### Literatur

Klaus Witte, Kai Hu, Johanna Swiatek, Claudia Müssig, Georg Ertl and Björn Lemmer: Experimental heart failure in rats: effects on cardiovascular circadian rhythms and on myocardial β-adrenergic signaling. Cardiovasc Res 47 (2): 203-405, 2000; Kozo Okamoto: Spontaneous hypertension in rats. Int Rev Exp Pathol 7: 227- 270, 1969; Maarten van den Buuse: Circadian Rhythms of Blood Pressure, Heart Rate, and Locomotor Activity in Spontaneously Hypertensive Rats as Measured With Radio-Telemetry. Physiology & Behavior 55(4): 783-787, 1994

### B-4. Bestimmung pharmakokinetischer Kenngrößen nach intravenöser und oraler Gabe

Die pharmakokinetischen Parameter der erfindungsgemäßen Verbindungen der Formel (I) werden in männlichen CD-1-Mäusen, männlichen Wister-Ratten und weiblichen Beagle-Hunden bestimmt. Die intravenöse Gabe erfolgt bei Mäusen und Ratten mittels einer speziesspezifischen Plasma/DMSO-Formulierung und bei Hunden mittels einer Wasser/PEG400/Ethanol-Formulierung. Die orale Gabe der gelösten Substanz mittels Schlundsonde wird in allen Spezies basierend auf einer Wasser/PEG400/Ethanol-Formulierung durchgeführt. Den Ratten wird zur vereinfachten Blutabnahme vor der Substanzgabe ein Silikonkatheter in die rechte *Vena jugularis externa* gelegt. Die Operation erfolgt mindestens einen Tag vor dem Versuch unter Isofluran-Narkose und unter Gabe eines Analgetikums (Atropin/Rimadyl (3/1) 0.1 mL s.c.). Die Blutabnahme (in der Regel mehr als 10 Zeitpunkte) erfolgt in einem Zeitfenster, welches terminale Zeitpunkte von mindestens 24 bis maximal 72 Stunden nach Substanzgabe beinhaltet. Das Blut wird bei der Entnahme in heparinisierte Röhrchen geleitet. So dann wird mittels Zentrifugation das Blutplasma gewonnen und gegebenenfalls bis zur weiteren Bearbeitung bei -20°C gelagert.

Den Proben der erfindungsgemäßen Verbindungen der Formel (I), Kalibrierproben und Qualifier wird ein interner Standard zugesetzt (dies kann auch eine chemisch nicht verwandte Substanz sein) und es folgt eine Proteinfällung mittels Acetonitril im Überschuss. Nach Zugabe einer PufferLösung, die an die LC-Bedingungen angepasst ist, und folgendem Vortexen wird bei 1000 g zentrifugiert. Der Überstand wird mittels LC-MS/MS unter Verwendung von C18-reversed-phase-Säulen und variablen Eluenten-Gemischen vermessen. Die Quantifizierung der Substanzen erfolgt anhand der Peakhöhen oder -flächen aus extrahierten Ionenchromatogrammen spezifischer selected ion monitoring-Experimente.

Aus den ermittelten Plasmakonzentration-Zeit-Verläufen werden die pharmakokinetischen Kenngrößen wie AUC, Cₘₐₓ, t_{1/2} (terminale Halbwertszeit), MRT (Mean Residence Time) und CL (Clearance) mittels eines validierten pharmakokinetischen Rechenprogramms berechnet.

Da die Substanzquantifizierung in Plasma durchgeführt wird, muss die Blut/Plasma-Verteilung der Substanz bestimmt werden, um die pharmakokinetischen Parameter entsprechend anpassen zu können. Dazu wird eine definierte Menge Substanz in heparinisiertem Vollblut der entsprechenden Spezies für 20 min im Taumelrollenmischer inkubiert. Nach Zentrifugation bei 1000g wird die Konzentration im Plasma gemessen (mittels LC-MS/MS; s.o.) und durch Quotientenbildung der C_{Blut}/Cₚₗₐₛₘₐ-Wert ermittelt.

### B-5. Metabolismus-Untersuchung

Zur Bestimmung des Metabolismus-Profils der erfindungsgemäßen Verbindungen werden diese mit rekombinanten humanen Cytochrom P450 (CYP) Enzymen, Lebermikrosomen oder mit primären frischen Hepatozyten verschiedener Tierspezies (z.B. Ratte, Hund) als auch humanen Ursprungs inkubiert, um Informationen über einen möglichst kompletten hepatischen Phase I- und Phase II-Metabolismus sowie über die am Metabolismus beteiligten Enzyme zu erhalten und zu vergleichen.

Die erfindungsgemäßen Verbindungen wurden mit einer Konzentration von etwa 0.1-10 µM inkubiert. Dazu wurden Stammlösungen der erfindungsgemäßen Verbindungen mit einer Konzentration von 0.01-1 mM in Acetonitril hergestellt, und dann mit einer 1:100 Verdünnung in den Inlcubationsansatz pipettiert. Die Lebermikrosomen und rekombinanten Enzyme wurden in 50 mM Kaliumphosphatpuffer pH 7.4 mit und ohne NADPH-generierendem System, bestehend aus 1 mM NADP⁺, 10 mM Glucose-6-phosphat und 1 Unit Glucose-6-phosphat Dehydrogenase, bei 37°C inkubiert. Primäre Hepatozyten wurden in Suspension in Williams E Medium ebenfalls bei 37°C inkubiert. Nach einer Inkubationszeit von 0 - 4h wurden die Inkubationsansätze mit Acetonitril abgestoppt (Endkonzentration ca. 30%) und das Protein bei ca. 15000 x g abzentrifugiert. Die so abgestoppten Proben wurden entweder direkt analysiert oder bis zur Analyse bei -20°C gelagert.

Die Analyse erfolgt mittels Hochleistungsflüssigkeits-Chromatographie mit Ultraviolett- und massenspektrometrischer Detektion (HPLC-UV-MS/MS). Dazu werden die Überstände der Inkubationsproben mit geeigneten C18-reversed-phase-Säulen und variablen Eluenten-Gemischen aus Acetonitril und 10 mM wässriger Ammoniumformiat-Lösung oder 0.05 % Ameisensäure chromatographiert. Die UV-Chromatogramme in Verbindung mit massenspektrometrischen Daten dienen zur Identifizierung, Strukturaufklärung und quantitativen Abschätzung der Metabolite, und der quantitativen metabolischen Abnahme der erfindungsgemäßen Verbindung in den Inkubationsansätzen.

### C. Ausführungsbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der erfindungsgemäßen Verbindung, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat 5 Minuten gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der erfindungsgemäßen Verbindung, 1000 mg Ethanol (96%), 400 mg Rhodigel^{®} (Xanthan gum der Firma FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäße Verbindung wird der Suspension zugefügt. Unter Rühren erfolgt die Zugabe des Wassers. Bis zum Abschluß der Quellung des Rhodigels wird ca. 6 h gerührt.

### Oral applizierbare Lösung:

### Zusammensetzung:

500 mg der erfindungsgemäßen Verbindung, 2.5 g Polysorbat und 97 g Polyethylenglycol 400. Einer Einzeldosis von 100 mg der erfindungsgemäßen Verbindung entsprechen 20 g orale Lösung.

### Herstellung:

Die erfindungsgemäße Verbindung wird in der Mischung aus Polyethylenglycol und Polysorbat unter Rühren suspendiert. Der Rührvorgang wird bis zur vollständigen Auflösung der erfindungsgemäßen Verbindung fortgesetzt.

### i.v.-Lösung:

Die erfindungsgemäße Verbindung wird in einer Konzentration unterhalb der Sättigungslöslichkeit in einem physiologisch verträglichen Lösungsmittel (z.B. isotonische Kochsalzlösung, Glucoselösung 5% und/oder PEG 400-Lösung 30%) gelöst. Die Lösung wird steril filtriert und in sterile und pyrogenfreie Injektionsbehältnisse abgefüllt.

## Patentansprüche

1. Verbindung der Formel (I) 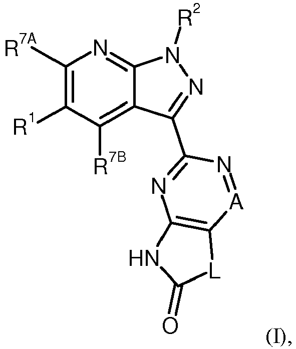 in welcher
A für Stickstoff oder CR³ steht,
wobei
R³ für Wasserstoff steht,
L für eine Gruppe #-CR^{4A}R^{4B}-(CR^{5A}R^{5B})ₚ-## steht,
wobei
# für die Anknüpfstelle an die Carbonylgruppe steht,
## für die Anknüpfstelle an den Pyrimidinring bzw Triazinring steht,
p für eine Zahl 0 steht,
R^{4A} für Wasserstoff, Fluor, Methyl oder Hydroxy steht,
R^{4B} für Wasserstoff, Fluor, Trifluormethyl, 2,2,2-Trifluorethyl, Methyl oder eine Gruppe der Formel -M-R⁸ steht,
worin
M für eine Bindung steht,
R⁸ für -(C=O)ᵣ-NR⁹R¹⁰ steht,
worin
r die Zahl 1 bedeutet,
R⁹ und R¹⁰ unabhängig voneinander jeweils für Wasserstoff, oder Cyclopropyl stehen,
R¹ für Wasserstoff oder Fluor steht,
R² für 4,4,4-Trifluorbut-1yl, 3,3,4,4-Tetrafluorbut-1yl, 3,3,4,4,4-Pentafluorbut-1yl, Cyclobutylmethyl oder Cyclopentylmethyl steht,
wobei Cyclobutylmethyl und Cyclopentylmethyl mit 1 oder 2 Substituenten Fluor substituiert sein können,
R^{7A} für Wasserstoff oder Methyl steht,
R^{7B} für Wasserstoff steht,
sowie ihre Salze, Solvate und Solvate der Salze.

2. Verfahren zur Herstellung von Verbindungen der Formel (I), wie in Anspruch 1 definiert, **dadurch gekennzeichnet, dass** man die Verbindung der Formel (II) 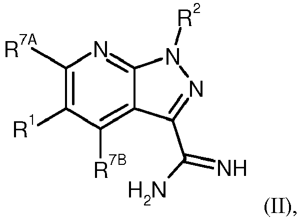 in welcher R¹, R², R^{7A} und R^{7B} jeweils die oben genannten Bedeutungen haben,
[A] in einem inerten Lösungsmittel in Gegenwart einer geeigneten Base mit einer Verbindung der Formel (III) 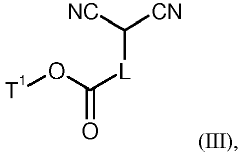 in welcher L die in Anspruch 1 genannte Bedeutung hat und
T¹ für (C₁-C₄)-Alkyl steht,
zu einer Verbindung der Formel (IV) 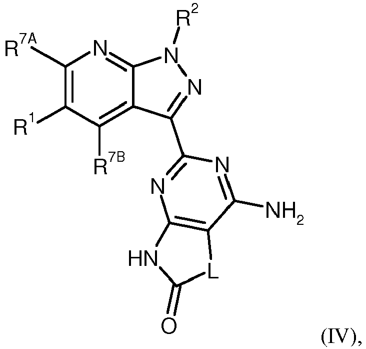 in welcher L, R¹, R², R^{7A} und R^{7B} jeweils die in Anspruch 1 angegebenen Bedeutungen haben,
umsetzt, diese dann mit iso-Pentylnitrit und einem Halogen-Äquivalent in eine Verbindung der Formel (V) 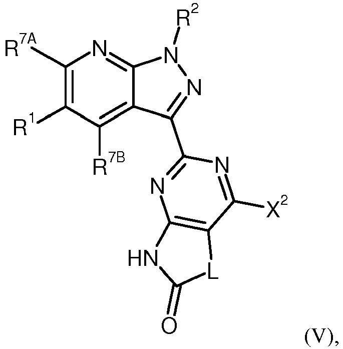 in welcher L, R¹, R², R^{7A} und R^{7B} jeweils die in Anspruch 1 angegebenen Bedeutungen haben und
X² für Brom oder Iod steht,
überführt, und diese im Anschluss in einen inerten Lösungsmittel in Gegenwart eines geeigneten Übergangsmetallkatalysators zu einer Verbindung der Formel (I-A) 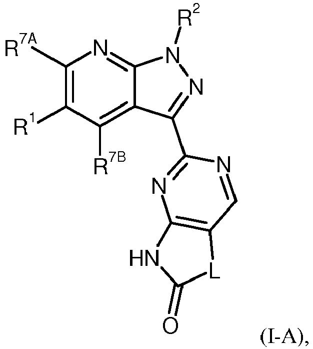 in welcher L, R¹, R², R^{7A} und R^{7B} jeweils die in Anspruch 1 angegebenen Bedeutungen haben,
umsetzt,
oder
[B] in einem inerten Lösungsmittel in Gegenwart einer geeigneten Base mit Hydrazinhydrat zu einer Verbindung der Formel (VI) 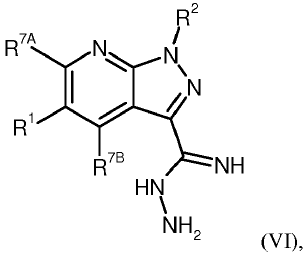 in welcher R¹, R², R^{7A} und R^{7B} jeweils die in Anspruch 1 genannten Bedeutungen haben,
umsetzt, diese dann in einem inerten Lösungsmittel mit einer Verbindung der Formel (VII) 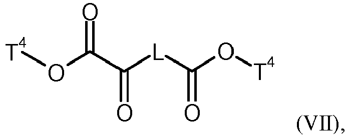 in welcher L die in Anspruch 1 angegebene Bedeutung hat und
T⁴ für (C₁-C₄)-Alkyl steht,
zu einer Verbindung der Formel (VIII) 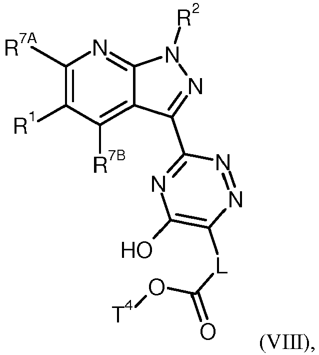 in welcher L, R¹, R², R^{7A}, R^{7B} und T⁴ jeweils die in Anspruch 1 angegebenen Bedeutungen haben,
reagiert, diese anschliessend mit Phosphorylchlorid in eine Verbindung der Formel (IX) 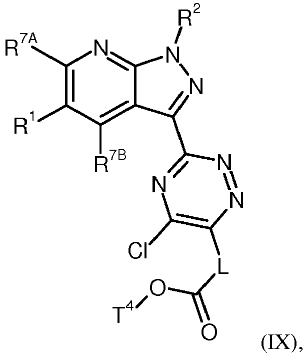 in welcher L, R¹, R², R^{7A}, R^{7B} und T⁴ jeweils die in Anspruch 1 angegebenen Bedeutungen haben,
überführt, und diese direkt mit Ammoniak zu einer Verbindung der Formel (X) 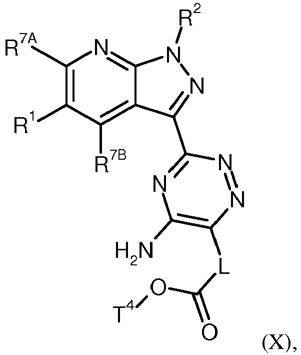 in welcher L, R¹, R², R^{7A}, R^{7B} und T⁴ jeweils die in Anspruch 1 angegebenen Bedeutungen haben,
umsetzt, und schliesslich in einem inerten Lösungsmittel gegebenenfalls in Gegenwart einer geeigneten Base zu einer Verbindung der Formel (I-B) 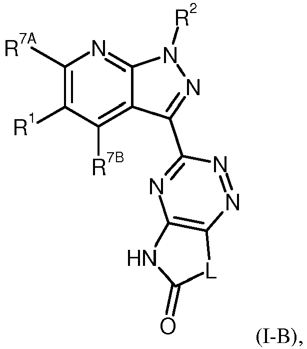 in welcher L, R¹, R², R^{7A} und R^{7B} jeweils die in Anspruch 1 angegebenen Bedeutungen haben,
cyclisiert, und gegebenenfalls die resultierenden Verbindungen der Formeln (I-A) und (I-B) gegebenenfalls mit den entsprechenden (i) Lösungsmitteln und/oder (ii) Säuren oder Basen in ihre Solvate, Salze und/oder Solvate der Salze überführt.

3. Verbindung der Formel (I), wie in Anspruch 1 definiert, zur Behandlung und/oder Prophylaxe von Krankheiten.

4. Verwendung einer Verbindung der Formel (I), wie in Anspruch 1 definiert, zur Herstellung eines Arzneimittels zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

5. Verbindung der Formel (I), wie in Anspruch 1 definiert, zur Verwendung in einem Verfahren zur Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

6. Arzneimittel enthaltend eine Verbindung der Formel (I), wie in Anspruch 1 definiert, in Kombination mit einem inerten, nicht-toxischen, pharmazeutisch geeigneten Hilfsstoff.

7. Arzneimittel enthaltend eine Verbindung der Formel (I), wie in Anspruch 1 definiert, in Kombination mit einem weiteren Wirkstoff ausgewählt aus der Gruppe bestehend aus organischen Nitraten, NO-Donatoren, cGMP-PDE-Inhibitoren, antithrombotisch wirkenden Mitteln, den Blutdruck senkenden Mitteln sowie den Fettstoffwechsel verändernden Mitteln.

8. Arzneimittel nach Anspruch 6 oder zur Verwendung in der Behandlung und/oder Prophylaxe von Herzinsuffizienz, Angina pectoris, Hypertonie, pulmonaler Hypertonie, Ischämien, Gefäßerkrankungen, Niereninsuffizienz, thromboembolischen Erkrankungen, fibrotischen Erkrankungen und Arteriosklerose.

## Claims

1. Compound of the formula (I) 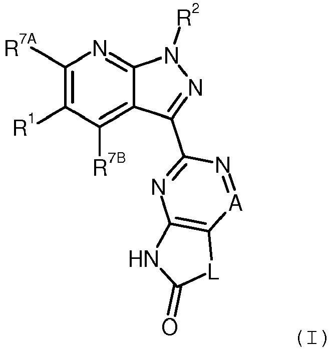 in which
A is nitrogen or CR³,
where
R³ represents hydrogen,
L is a #-CR^{4A}R^{4B}-(CR^{5A}R^{5B})ₚ-## group
where
# is the point of attachment to the carbonyl group,
## is the point of attachment to the pyrimidine ring or triazine ring,
p is a number 0,
R^{4A} is hydrogen, fluorine, methyl or hydroxy,
R^{4B} is hydrogen, fluorine, trifluoromethyl, 2,2,2-trifluoroethyl, methyl or a group of the formula -M-R⁸,
in which
M is a bond,
R⁸ is - (C=O) ᵣ-NR⁹R¹⁰,
in which
r is the number 1,
R⁹ and R¹⁰ independently of one another are each hydrogen or cyclopropyl,
R¹ is hydrogen or fluorine,
R² is 4,4,4-trifluorobut-1-yl, 3,3,4,4-tetrafluorobut-1-yl, 3,3,4,4,4-pentafluorobut-1-yl, cyclobutylmethyl or cyclopentylmethyl,
where cyclobutylmethyl and cyclopentylmethyl may be substituted by 1 or 2 fluorine substituents,
R^{7A} is hydrogen or methyl,
R^{7B} is hydrogen,
and the salts, solvates and solvates of the salts thereof.

2. Process for preparing compounds of the formula (I) as defined in Claim 1, **characterized in that** the compound of the formula (II) 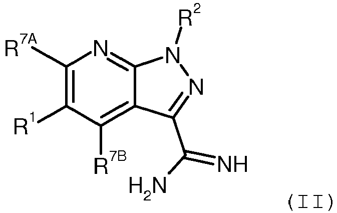 in which R¹, R², R^{7A} and R^{7B} each have the meanings given above,
[A] is reacted in an inert solvent in the presence of a suitable base with a compound of the formula (III) 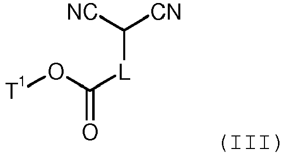 in which L has the meaning given in Claim 1 and
T¹ is (C₁-C₄)-alkyl,
to give a compound of the formula (IV) 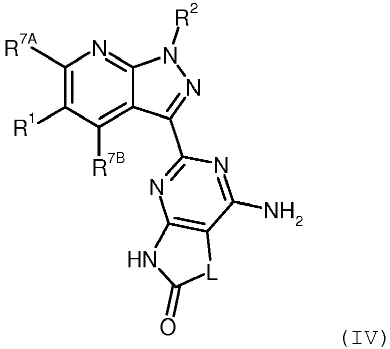 in which L, R¹, R², R^{7A} and R^{7B} each have the meanings given in Claim 1,
this is then converted with isopentyl nitrite and a halogen equivalent into a compound of the formula (V) 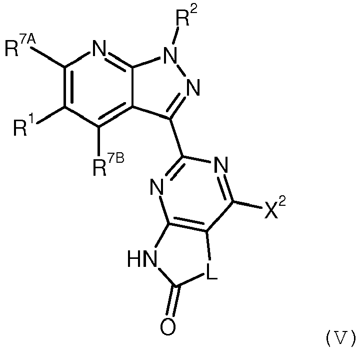 in which L, R¹, R², R^{7A} and R^{7B} each have the meanings given in Claim 1 and
X² is bromine or iodine,
and this is then reacted in an inert solvent, in the presence of a suitable transition metal catalyst, to give a compound of the formula (I-A) 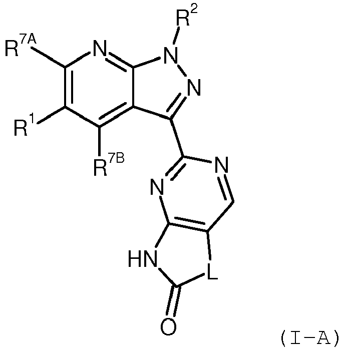 in which L, R¹, R², R^{7A} and R^{7B} each have the meanings given in Claim 1,
or
[B] is reacted in an inert solvent in the presence of a suitable base with hydrazine hydrate to give a compound of the formula (VI) 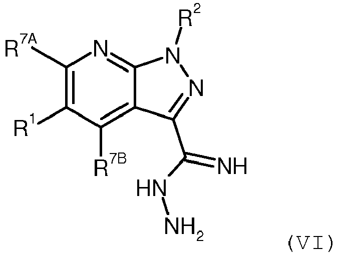 in which R¹, R², R^{7A} and R^{7B} each have the meanings given in Claim 1,
this is then reacted in an inert solvent with a compound of the formula (VII) 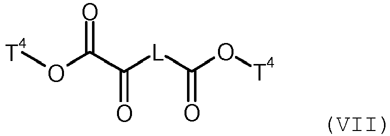 in which L has the meaning given in Claim 1 and
T⁴ is (C₁-C₄)-alkyl
to give a compound of the formula (VIII) 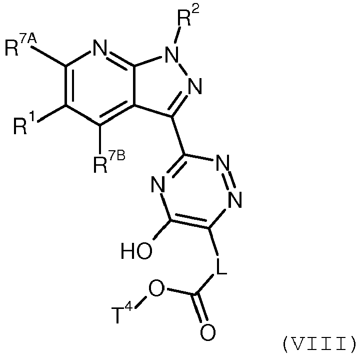 in which L, R¹, R², R^{7A}, R^{7B} and T⁴ each have the meanings given in Claim 1,
this is then converted with phosphoryl chloride into a compound of the formula (IX) 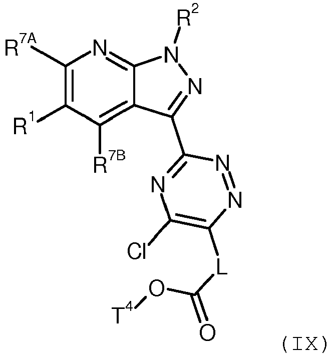 in which L, R¹, R², R^{7A}, R^{7B} and T⁴ each have the meanings given in Claim 1,
and this is reacted directly with ammonia to give a compound of the formula (X) 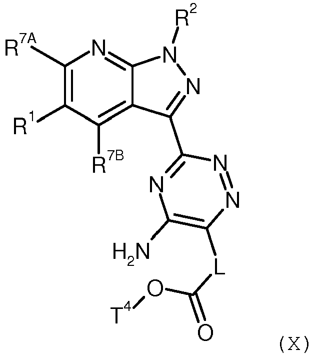 in which L, R¹, R², R^{7A}, R^{7B} and T⁴ each have the meanings given in Claim 1,
and finally cyclized in an inert solvent, optionally in the presence of a suitable base, to give a compound of the formula (I-B) 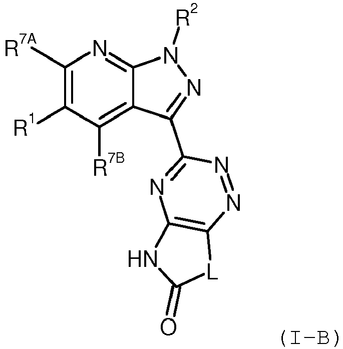 in which L, R¹, R², R^{7A}, R^{7B} and T⁴ each have the meanings given in Claim 1,
and the resulting compounds of the formulae (I-A) and (I-B) are, where appropriate, converted with the appropriate (i) solvents and/or (ii) acids or bases into their solvates, salts and/or solvates of the salts.

3. Compound of the formula (I) as defined in Claim 1 for the treatment and/or prophylaxis of diseases.

4. Use of a compound of the formula (I) as defined in Claim 1 for production of a medicament for the treatment and/or prophylaxis of heart failure, angina pectoris, hypertension, pulmonary hypertension, ischemias, vascular disorders, renal insufficiency, thromboembolic disorders, fibrotic disorders and arteriosclerosis.

5. Compound of the formula (I) as defined in Claim 1 for use in a method for the treatment and/or prophylaxis of heart failure, angina pectoris, hypertension, pulmonary hypertension, ischemias, vascular disorders, renal insufficiency, thromboembolic disorders, fibrotic disorders and arteriosclerosis.

6. Medicament comprising a compound of the formula (I) as defined in Claim 1 in combination with an inert, nontoxic, pharmaceutically suitable excipient.

7. Medicament comprising a compound of the formula (I) as defined in Claim 1 in combination with a further active compound selected from the group consisting of organic nitrates, NO donors, cGMP-PDE inhibitors, antithrombotic agents, hypotensive agents and lipid metabolism modifiers.

8. Medicament according to Claim 6 or 7 for use in the treatment and/or prophylaxis of heart failure, angina pectoris, hypertension, pulmonary hypertension, ischemias, vascular disorders, renal insufficiency, thromboembolic disorders, fibrotic disorders and arteriosclerosis.

## Revendications

1. Composé de formule (I) 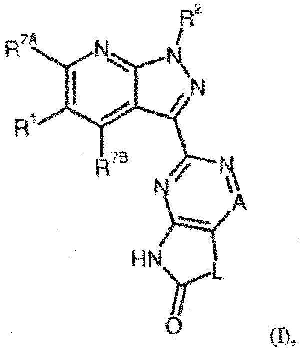 dans laquelle
A représente azote ou CR³,
R³ représentant hydrogène,
L représente un groupe #-CR^{4A}R^{4B}- (CR^{5A}R^{5B}) ₚ-##,
# représentant l'emplacement de liaison au groupe carbonyle,
## représentant l'emplacement de liaison au cycle pyrimidine ou au cycle triazine
p représentant le nombre 0,
R^{4A} représentant hydrogène, fluor, méthyle ou hydroxy, R^{4B} représentant hydrogène, fluor, trifluorométhyle, 2,2,2-trifluoroéthyle, méthyle ou un groupe de formule -M-R⁸,
M représentant une liaison,
R⁸ représentant - (C=O) ᵣ-NR⁹R¹⁰,
r signifiant le nombre 1,
R⁹ et R¹⁰ représentant indépendamment l'un de l'autre hydrogène ou cyclopropyle,
R¹ représente hydrogène ou fluor,
R² représente 4,4,4-trifluorobut-1-yle, 3,3,4,4-tétrafluorobut-1-yle, 3,3,4,4,4-pentafluorobut-1-yle, cyclobutylméthyle ou cyclopentylméthyle, cyclobutylméthyle et cyclopentylméthyle pouvant être substitués avec 1 ou 2 substituants fluor,
R^{7A} représente hydrogène ou méthyle,
R^{7B} représente hydrogène,
ainsi que leurs sels, solvates et solvates des sels.

2. Procédé de fabrication de composés de formule (I), tels que définis dans la revendication 1, **caractérisé en ce que** le composé de formule (II) 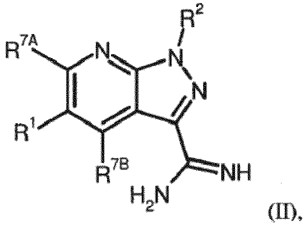 dans laquelle R¹, R², R^{7A} et R^{7B} ont chacun les significations indiquées précédemment,
[A] est mis en réaction dans un solvant inerte en présence d'une base appropriée avec un composé de formule (III) 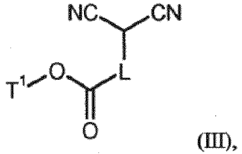 dans laquelle L a la signification indiquée dans la revendication 1, et
T¹ représente alkyle en (C₁-C₄),
pour former un composé de formule (IV) 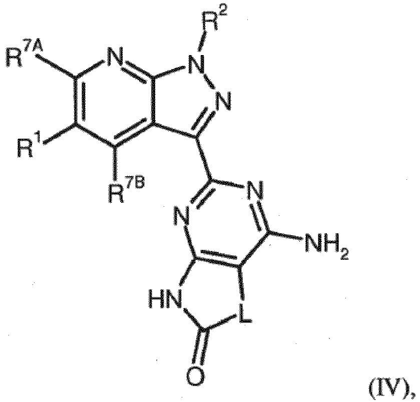 dans laquelle L, R¹, R², R^{7A} et R^{7B} ont chacun les significations indiquées dans la revendication 1,
puis celui-ci est transformé avec de l'isopentylnitrite et un équivalent d'halogène en un composé de formule (V) 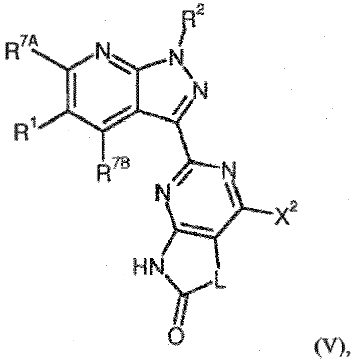 dans laquelle L, R¹, R², R^{7A} et R^{7B} ont chacun les significations indiquées dans la revendication 1, et
X² représente brome ou iode,
puis celui-ci est mis en réaction dans un solvant inerte en présence d'un catalyseur de métal de transition approprié pour former un composé de formule
(I-A) 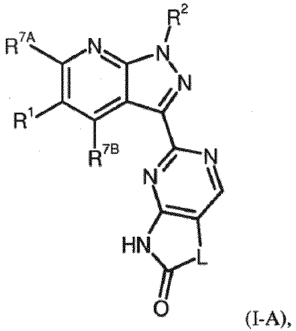
dans laquelle L, R¹, R², R^{7A} et R^{7B} ont chacun les significations indiquées dans la revendication 1,
ou
[B] est mis en réaction dans un solvant inerte en présence d'une base appropriée avec un hydrate d'hydrazine pour former un composé de formule (VI) 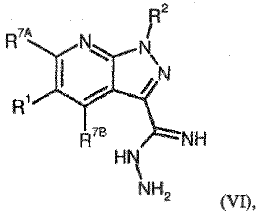 dans laquelle R¹, R², R^{7A} et R^{7B} ont chacun les significations indiquées dans la revendication 1,
puis celui-ci est mis en réaction dans un solvant inerte avec un composé de formule (VII) 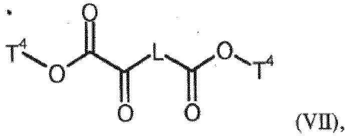 dans laquelle L a la signification indiquée dans la revendication 1, et
T⁴ représente alkyle en (C₁-C₄),
pour former un composé de formule (VIII) 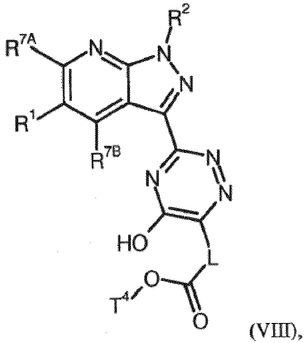 dans laquelle L, R¹, R², R^{7A}, R^{7B} et T⁴ ont chacun les significations indiquées dans la revendication 1,
puis celui-ci est transformé avec du chlorure de phosphoryle en un composé de formule (IX) 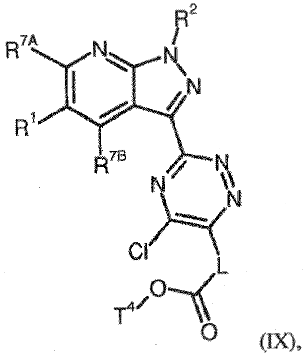 dans laquelle L, R¹, R², R^{7A}, R^{7B} et T⁴ ont chacun les significations indiquées dans la revendication 1,
et celui-ci est mis en réaction directement avec de l'ammoniac pour former un composé de formule (X) 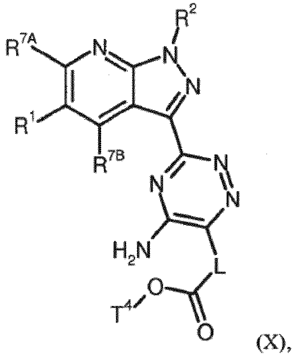 dans laquelle L, R¹, R², R^{7A}, R^{7B} et T⁴ ont chacun les significations indiquées dans la revendication 1,
et enfin cyclisé dans un solvant inerte, éventuellement en présence d'une base appropriée, en un composé de formule (I-B) 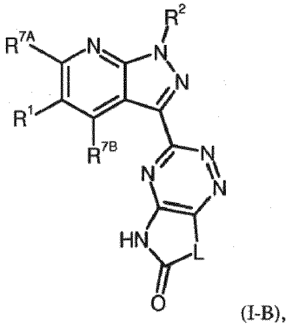 dans laquelle L, R¹, R², R^{7A} et R^{7B} ont chacun les significations indiquées dans la revendication 1,
et les composés de formule (I-A) et (I-B) résultants sont éventuellement transformés avec (i) les solvants et/ou (ii) les acides ou bases appropriés en leurs solvates, sels et/ou solvates des sels.

3. Composé de formule (I), tel que défini dans la revendication 1, pour le traitement et/ou la prophylaxie de maladies.

4. Utilisation d'un composé de formule (I), tel que défini dans la revendication 1, pour la fabrication d'un médicament pour le traitement et/ou la prophylaxie de l'insuffisance cardiaque, l'angine de poitrine, l'hypertension, l'hypertension pulmonaire, les ischémies, les maladies vasculaires, l'insuffisance rénale, les maladies thromboemboliques, les maladies fibrotiques et l'artériosclérose.

5. Composé de formule (I), tel que défini dans la revendication 1, destiné à une utilisation dans un procédé de traitement et/ou de prophylaxie de l'insuffisance cardiaque, l'angine de poitrine, l'hypertension, l'hypertension pulmonaire, les ischémies, les maladies vasculaires, l'insuffisance rénale, les maladies thromboemboliques, les maladies fibrotiques et l'artériosclérose.

6. Médicament contenant un composé de formule (I), tel que défini dans la revendication 1, en combinaison avec un adjuvant inerte, non toxique, pharmaceutiquement approprié.

7. Médicament contenant un composé de formule (I), tel que défini dans la revendication 1, en combinaison avec un autre agent actif choisi dans le groupe constitué par les nitrates organiques, les donneurs de NO, les inhibiteurs de cGMP-PDE, les agents antithrombotiques, les agents abaissant la tension artérielle et les agents modifiant le métabolisme des lipides.

8. Médicament selon la revendication 6 ou 7, destiné à une utilisation pour le traitement et/ou la prophylaxie de l'insuffisance cardiaque, l'angine de poitrine, l'hypertension, l'hypertension pulmonaire, les ischémies, les maladies vasculaires, l'insuffisance rénale, les maladies thromboemboliques, les maladies fibrotiques et l'artériosclérose.
